(19) Europäisches Patentamt
European Patent Office
Office européen des brevets

(11) EP 3 233 807 B1

(12) **EUROPÄISCHE PATENTSCHRIFT**

(45) Veröffentlichungstag und Bekanntmachung des Hinweises auf die Patenterteilung:
**04.09.2019 Patentblatt 2019/36**

(21) Anmeldenummer: **15826016.6**

(22) Anmeldetag: **18.12.2015**

(51) Int Cl.:
***C07D 237/32*** *(2006.01)* ***A61K 31/502*** *(2006.01)*
***A61P 37/02*** *(2006.01)* ***A61P 9/10*** *(2006.01)*

(86) Internationale Anmeldenummer:
**PCT/EP2015/002555**

(87) Internationale Veröffentlichungsnummer:
**WO 2016/096143 (23.06.2016 Gazette 2016/25)**

(54) **KRISTALLINE FORM VON 5-AMINO-2,3-DIHYDROPHTHALAZIN-1,4-DION NATRIUMSALZ, DIESE ENTHALTENDE PHARMAZEUTISCHE ZUBEREITUNGEN UND VERFAHREN ZU IHRER HERSTELLUNG**

CRYSTALLINE FORM OF 5-AMINO-2,3-DIHYDROPHTHALAZINE-1,4-DION SODIUM SALT, PHARMACEUTICAL PREPARATIONS CONTAINING THE SAME AND METHOD FOR THE PRODUCTION OF SAID FORM

FORME CRISTALLINE DU SEL DE SODIUM DE 5-AMINO-2,3-DIHYDROPHTALAZINE-1,4-DIONE, PRÉPARATIONS PHARMACEUTIQUES CONTENANT CELLES-CI ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2014 EP 14004274**

(43) Veröffentlichungstag der Anmeldung:
**25.10.2017 Patentblatt 2017/43**

(73) Patentinhaber: **MetrioPharm AG**
**8002 Zürich (CH)**

(72) Erfinder:
- **MARTIN, Thomas**
**95100 Selb (DE)**
- **BREU, Josef**
**95448 Bayreuth (DE)**
- **BRYSCH, Wolfgang**
**13505 Berlin (DE)**
- **KOSEL, David**
**04105 Leipzig (DE)**
- **LUDESCHER, Beate**
**13439 Berlin (DE)**
- **NIEDERMAIER, Michael**
**12359 Berlin (DE)**
- **VON WEGERER, Jörg**
**13597 Berlin (DE)**

(74) Vertreter: **Gruber, Daniel**
**Ruschke, Madgwick, Seide & Kollegen**
**Postfach 86 06 29**
**81633 München (DE)**

(56) Entgegenhaltungen:
**EP-A1- 1 203 587 WO-A1-2011/107295**

- **V. B. RYBAKOV ET AL: "On the structure of luminol sodium salts", CRYSTALLOGRAPHY REPORTS, Bd. 59, Nr. 3, 1. Mai 2014 (2014-05-01), Seiten 383-393, XP055256453, RU ISSN: 1063-7745, DOI: 10.1134/S1063774514020187 in der Anmeldung erwähnt**
- **HALEBLIAN J ET AL: "PHARMACEUTICAL APPLICATIONS OF POLYMORPHISM", JOURNAL OF PHARMACEUTICAL SCIENCES, AMERICAN PHARMACEUTICAL ASSOCIATION, WASHINGTON, US, Bd. 58, Nr. 8, 1. August 1969 (1969-08-01) , Seiten 911-929, XP009058367, ISSN: 0022-3549, DOI: 10.1002/JPS.2600580802 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

TECHNISCHES GEBIET DER ERFINDUNG:

Die vorliegende Erfindung betrifft die Bereitstellung einer neuen kristallinen Form von 5-Amino-2,3-dihydrophthalazin-1,4-dion Natriumsalz mit vorteilhaften Eigenschaften, diese enthaltende pharmazeutische Zubereitungen, sowie Verfahren zu deren Herstellung.

Die Erfindung betrifft insbesondere die Bereitstellung einer neuen kristallinen Form von 5-Amino-2,3-dihydrophthalazin-1,4-dion zu medizinischen Zwecken.

HINTERGRUND DER ERFINDUNG:

Seit längerem sind aus dem Stand der Technik chemische Verbindungen mit immunmodulatorischer Wirkung bekannt. Zu diesen Verbindungen zählt u.a. 5-Amino-2,3-dihydrophthalazin-1,4-dion Natriumsalz. Diese Verbindung ist aus der EP1203587A1 bekannt und weist die folgende Grundstruktur auf ($Na^+$ nicht gezeigt):

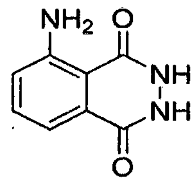

Diese oben gezeigte Grundstruktur wird auch als Luminol bezeichnet. Weitere gebräuchliche Synonyme sind 3-Aminophthalhydrazid, 3-Aminophthalsäurehydrazid, o-Aminophthalhydrazid und o-Aminophthalsäurehydrazid. Aus dem Stand der Technik ist bekannt, dass 5-Amino-2,3-dihydrophthalazin-1,4-dion Alkalisalze als Feststoffe in verschiedenen Hydratformen kristallisieren. Im Stand der Technik sind insbesondere das Dihydrat des Natriumsalzes (RU2113222C1) und ein Trihydrat des Kaliumsalzes beschrieben sowie Mischformen hierzu (RU2211036C2). Aus wissenschaftlichen Publikationen sind zudem Kristallstrukturen von Alkalisalzen des 5-Amino-2,3-dihydrophthalazin-1,4-dions, teils in unterschiedlichen Hydratstufen bekannt. Strukturell charakterisiert sind hier ein Anhydrat des Kaliumsalzes, ein Monohydrat des Lithiumsalzes, ein Dihydrat des Rubidiumsalzes, ein Trihydrat des Caesiumsalzes und ein Hexahydrat des Natriumsalzes. (vgl. Guzei et al. (2013): Journal of Coordination Chemistry, 66:21, 3722-3739) Ebenso strukturell charakterisiert wurde die Dihydratform des Natriumsalzes, sowie zwei polymorphe Anhydratformen. Die Charakterisierung erfolgte aus jeweiligen Mischungen beider Anhydratformen oder aller drei Formen, (vgl. Rybakov et al. (2014): Crystallography Reports, 59, 383-393).

Reine kristalline Anhydratformen von 5-Amino-2,3-dihydrophthalazin-1,4-dion Natriumsalz wurden erstmals in der WO2011/107295A1 als Form I und Form II beschrieben. Die kristallinen Formen aus WO2011/107295A1 wurden dargestellt durch ein Röntgenpulverbeugungsdiagramm, das in D - oder 2-Theta-Werten ausgedrückt wurde, wobei mit "D" die Interplanarabstände und mit "2-Theta" die 2-Theta-Winkel in Grad bezeichnet waren. Der Interplanarabstand D (auch d) beschreibt die senkrechte Distanz zwischen zwei aufeinanderfolgenden parallelen Gitterebenen in einem Kristall. Der Bragg-Winkel Theta ($\theta$) bezeichnet den charakteristischen Winkel, unter dem ein einfallender Röntgenstrahl an einer Gitterebene eines Kristalls reflektiert wird und somit ein Röntgenbeugungsmuster erzeugt. Beide Größen sind über die Bragg-Formel

$$n\lambda = 2d\sin(\theta)$$

miteinander verknüpft. Durch die charakteristischen Werte von d und $\theta$ wird ein Kristall notwendig und hinreichend beschrieben.

Die D-Werte wurden in der WO2011/107295A1 angegeben mit
13,5; 6,9; 5,2; 4,6; 3,9; 3,5; 3,4; 3,3; 3,1 und 3,0 für Form I; und mit
12,9; 7,9; 7,1; 6,5; 5,3; 4,0; 3,7; 3,6; 3,3 und 3,2 für Form II.

Die 2-Theta-Winkel wurden angegeben mit
6,5; 12,7; 16,9; 19,3; 22,8; 25,8; 26,6; 27,2; 28,7 und 30,3 für Form I; und mit
6,8; 11,2; 12,5; 13,7; 16,7; 22,4; 24,3; 24,9; 27,2 und 27,8 für Form II.

Zusätzlich wurden die relativen Intensitäten der Reflexe wiedergegeben.

Die WO2011/107295A1 führt auch eine Reihe von relativ komplexen Herstellungsverfahren sowohl für Form I als auch für Form II auf.

Es ist bekannt, dass kristalline Formen einer Substanz in ihren physikalischen Eigenschaften wie z.B. Löslichkeit, Auflöserate und Stabilität divergieren können (vgl. Haleblian und McCrone (1969): Journal of Pharmaceutical Sciences, 58:911-929). Solche Eigenschaften können die pharmazeutische Verarbeitung eines Wirkstoffs ebenso beeinflussen wie seine biologische Verfügbarkeit und Pharmakokinetik und somit seine biologische Wirksamkeit (vgl. Griesser (2006) in: Polymorphisms in the Pharmaceutical Industry. Hilfiker (Ed.) 211-234). Für die Arzneimittelherstellung ist es wichtig, dass der Ausgangsstoff stabil ist, nicht wasserziehend und in seinem Feststoffverhalten über den gesamten Herstellungsprozess kontrollierbar. Zudem sind die chemische Stabilität und Festphasenstabilität (Phasenreinheit) mit langer Lagerfähigkeit eines Wirkstoffs von herausragender Bedeutung (vgl. Miller et al. (2006) in: Polymorphisms in the Pharmaceutical Industry. Hilfiker (Ed.) 385-403). Dabei ist es wünschenswert, dass auch über eine möglichst lange Lagerperiode die physikalischen Eigenschaften des Wirkstoffs erhalten bleiben. Dies betrifft z.B. die Hygroskopizität, Löslichkeit oder initiale Auflösungsrate des Wirkstoffs, aber auch die Phasenreinheit.

Von großer Bedeutung für die pharmazeutische Verarbeitung und die medizinische Verwendung sind Herstellungsverfahren, welche die Herstellung der gewünschten kristallinen Formen verlässlich und reproduzierbar ermöglichen. Bei der Herstellung kristalliner Formen ist zu bedenken, dass bereits geringe Abweichungen in den Prozessparametern Änderungen in der Kristallstruktur des Produkts verursachen und damit im Ergebnis zu anderen kristallinen Formen bzw. Mischformen führen können. Dadurch veränderte Eigenschaften - zum Beispiel eine veränderte biologische Wirksamkeit durch eine andere Löslichkeit - können zum Ausfall ganzer Chargen führen. Mitunter ist es gar nicht mehr möglich, die gewünschte Form herzustellen (vgl. Ulrich und Jones (2005): Nachrichten aus der Chemie 53:19-23). Neben der Phasenreinheit des Wirkstoffs und den sich daraus ergebenden möglichen Änderungen der Wirksamkeit können so auch weitere wichtige Eigenschaften für die pharmazeutische Verarbeitung nachteilig beeinflusst werden, z.B. die Eignung zur Tablettenverpressung durch eine Beeinträchtigung der Rieselfähigkeit oder Fließgeschwindigkeit der kristallinen Form.

5-Amino-2,3-dihydrophthalazin-1,4-dion Alkalisalze gehören zur Gruppe der Aminophthalhydrazide und werden im Stand der Technik als Immunmodulatoren mit besonderen anti-inflammatorischen, anti-oxidativen und anti-toxischen Eigenschaften (vgl. WO2011/107295A1, US6489326B1; EP0617024B1, US5512573A, US5543410A, US7326690B2) beschrieben.

Immunmodulatorische Substanzen werden üblicherweise ihrer Wirkung entsprechend in Immunsuppressiva und Immunstimulanzien unterteilt (vgl. Rote Liste Service GmbH (2014): www.rote-liste.de, Zugriff am 02.09.2014). Die entsprechenden Präparate mit ausschließlich immunsupprimierender oder ausschließlich immunstimulierender Wirkung, wie zum Beispiel immunsupprimierende TNF-alpha-Blocker oder immunstimulierende Interferon-beta-Präparate, rufen häufig gerade aufgrund ihres sehr spezifischen Wirkmechanismus erhebliche unerwünschte Nebenwirkungen im Organismus hervor. Einige bekannte immunsupprimierende Substanzen, wie beispielsweise der TNF-alpha-Blocker Adalimumab, hemmen gezielt bestimmte Entzündungsmediatoren. Derartige Therapien sind bekanntermaßen mit gravierenden Nebenwirkungen verbunden (vgl. Descotes (2008): Expert Opin. Drug Metab. Toxicol., 4:12:1537-1549), da die Blockade einzelner Entzündungsmediatoren einen schweren Eingriff in das komplexe Immunsystem darstellt. So treten bei Adalimumab häufig opportunistische Infektionen wie beispielsweise Sepsis oder gegebenenfalls auch maligne Lymphome auf. Infolge dessen ist der Organismus nicht mehr in der Lage seine Funktion zu erfüllen, also selbständig und physiologisch angemessen auf endogene oder exogene Entzündungsreize, wie z.B. bakterielle Infektionen, zu reagieren. So ist zum Beispiel der Einsatz von TNF-alpha-Blockern im Falle schwerwiegender Infektionen kontraindiziert, dies gilt insbesondere für Sepsis und Tuberkulose. Vor der Gabe einer entsprechenden Medikation, wie z.B. bei der Behandlung Rheumatoider Arthritis, wird ein TBC-Screening dringend empfohlen. Außerdem konnte durch Hoffmann (2005: Intensivmed 42:371-377) anschaulich gezeigt werden, dass sich TNF-alpha-Blocker nicht für die klinische Anwendung bei septischen Zuständen eignen, sondern im Gegenteil sogar zu einer Erhöhung der Sterblichkeit führen können.

Die besonderen pharmakologischen Eigenschaften der 5-Amino-2,3-dihydrophthalazin-1,4-dion Alkalisalze dagegen erweisen sich als ausgesprochen nützlich u.a. bei der Bekämpfung von so genannten Zytokinstürmen infolge überschießender Immunreaktionen. Denn im Unterschied zu den besagten Zytokinblockern sind diese Salze weitestgehend nebenwirkungsfrei, da es nicht zu einer Hemmung einzelner Zytokine kommt, sondern diese auf ein physiologisches Niveau reguliert werden und somit weiterhin eine adäquate Reaktion des Organismus auf infektiöse Keime gewährleistet ist. Korrekterweise sollte hier also nicht vom Immunmodulatoren, sondern von Immunregulatoren gesprochen werden.

## ZUSAMMENFASSUNG DER ERFINDUNG

Bei der Herstellung von Arzneimitteln, insbesondere bei der Dosierung von Wirkstoffen in pharmazeutischen Zubereitungen spielt die Schüttdichte als Stoffeigenschaft eine große Rolle. Insbesondere zu niedere Schüttdichten können Probleme im Produktionsprozess bereiten, die von schlechten Fließeigenschaften bis zu Schwierigkeiten bei der exakten Dosierung reichen. Ein weiteres häufiges Problem bei Arzneimitteln, speziell bei polymorphen Wirkstoffen,

ist der Erhalt der Phasenreinheit des Wirkstoffes über einen längeren Zeitraum (Lagerbeständigkeit). Insbesondere in pharmazeutischen Zubereitungen, die die Gefahr einer Benetzung des Wirkstoffes mit Lösungsmitteln beinhalten, wie dies z.B. bei Tabletten, Cremes, Lotionen oder Emulsionen der Fall sein kann, ist das Risiko einer Festphasenumwandlung, insbesondere auch innerhalb der aus wirtschaftlichen Gründen möglichst lange angedachten Haltbarkeitsdauer, besonders groß.

Aufgabe der vorliegenden Erfindung war es, eine neue Anhydratform zu 5-Amino-2,3-dihydrophthalazin-1,4-dion Natriumsalz mit vorteilhaften Eigenschaften, insbesondere einer besseren Lagerbeständigkeit und/oder einer verbesserten Schüttdichte, bereitzustellen, die solcherart gezielt für medizinische, insbesondere anti-inflammatorische und immunregulierende Zwecke eingesetzt werden kann. Darüber hinaus soll die bereitgestellte Form physikalischchemische Eigenschaften aufweisen, welche für daraus einzeln oder in Kombination hergestellte, gelagerte und/oder angewendete Arzneimittel vorteilhaft sind.

Die Aufgabe wurde durch die Bereitstellung eines neuen Anhydrats von 5-Amino-2,3-dihydrophthalazin-1,4-dion Natriumsalzes (Form III) gelöst, welche sich überraschenderweise anhand experimenteller Daten in physikochemischen und biologischen Eigenschaften vom Stand der Technik nachweislich abhebt, insbesondere auch von den bereits in der WO2011/107295A1 beschriebenen Anhydratformen (Form I und Form II) von 5-Amino-2,3-dihydrophthalazin-1,4-dion Natriumsalz.

Die kristalline Anhydratform III (Form III) wird definiert durch je 15 charakteristische Werte zu Interplanarabständen und 2-Theta-Winkelwerten (vgl. Tab. 1), ausgedrückt in einem Röntgenpulverdiffraktogramm (Abb. 1). Form III wird weiterhin durch eine Strukturlösung und Rietveld-Verfeinerung aus XRPD-Daten mittels der Software TOPAS Academic (Tab. 2) resultierend in der Kristallstruktur mit entsprechendem Packungsmotiven (Abb. 2), sowie einem Festkörper-FT-IR-Spektrum (Abb. 3) und einem Raman-Spektrum (Abb. 4) definiert.

Unterschiede in physikalischen Eigenschaften bestehen zwischen der erfindungsgemäßen Form und den im Stand der Technik bereits beschriebenen 5-Amino-2,3-dihydrophthalazin-1,4-dion Natriumsalz Anhydrat-Reinformen beispielsweise in der Schüttdichte und in der Lagerbeständigkeit. Gegenüber den Formen I und II zeichnet sich die erfindungsgemäße Form III sowohl durch eine höhere Schüttdichte als auch eine bessere Lagerbeständigkeit aus; wobei die Schüttdichte einzelner Herstellungschargen der erfindungsgemäßen Form III über 150 kg/m$^3$, bevorzugt über 175 kg/m$^3$ und besonders bevorzugt über 200 kg/m$^3$ liegt und wobei die Lagerbeständigkeit sich insbesondere auf den Erhalt der Phasenreinheit bezieht, besonders bevorzugt in Situationen, in denen es gewollt oder ungewollt zur Benetzung mit Lösungsmitteln kommen kann, wobei bereits geringe Mengen an Lösungsmittel eine partielle Phasenumwandlung bewirken können.

Die Erfinder haben es sich ferner zur Aufgabe gemacht, ein oder mehrere Verfahren bereitzustellen, welche die Herstellung der erfindungsgemäßen neuen Anhydratform zu 5-Amino-2,3-dihydrophthalazin-1,4-dion Natriumsalz praktikabel, wirtschaftlich und reproduzierbar ermöglichen. Die aufgezeigten Verfahren sollen bevorzugt ohne den Einsatz von Schwermetallkatalysatoren auskommen und die Herstellung der neuen kristallinen Form III auch für beliebige Ansatzgrößen reproduzierbar ermöglichen.

Diese Aufgabe wurde durch die erfindungsgemäßen Verfahren zur Herstellung kristalliner Form III gelöst. In einem erfindungsgemäßen Verfahren wird zunächst eine beliebige Form von 5-Amino-2,3-dihydrophthalazin-1,4-dion Natriumsalz Anhydrat hergestellt und danach unter Rühren in DMSO (Dimethylsulfoxid) vollständig gelöst. Das DMSO wird bis zum Entstehen einer Suspension abgedampft, die Suspension anschließend eingetrocknet. Dieses erfindungsgemäße Verfahren lässt sich für beliebige Ansatzmengen verwenden. Das als Ausgangsprodukt zur Herstellung verwendete 5-Amino-2,3-dihydrophthalazin-1,4-dion Natriumsalz sollte möglichst rein sein. Dieses kann beispielsweise durch Reduktion von 3-Nitrophthalsäure in alkalischem Medium mit einem geeigneten Reduktionsmittel über 3-Nitrophthalanhydrid hergestellt werden. Optionale Aufreinigungsschritte durch Rekristallisation können sich anschließen. Weiterhin wird ein besonders vorteilhaftes Verfahren zur Herstellung der Anhydratform II des 5-Amino-2,3-dihydrophthalazin-1,4-dion Natriumsalzes (Form II) vorgestellt.

Die vorliegende Erfindung umfasst schließlich die Verwendung von Form III zu medizinischen Zwecken als Einzelstoff oder in Kombination mit einem oder mehreren weiteren Wirkstoffen, sowie pharmazeutische Zubereitungen enthaltend Form III, alleine oder in Kombination mit einem oder mehreren weiteren Wirkstoffen.

## DETAILLIERTE BESCHREIBUNG

Wenn nicht anders dargestellt, kommt den in der vorliegenden Erfindung verwendeten technischen und wissenschaftlichen Begriffen die Bedeutung zu, die ihnen der Fachmann im relevanten technischen Gebiet beimisst.

### Definitionen:

"*Form I*" und "*Form II*" sind aus der WO2011/107295A1 bekannte kristalline Formen zu 5-Amino-2,3-dihydrophthalazin-1,4-dion Natriumsalz. Im Dienste einer besseren Lesbarkeit werden diese Bezeichnungen beibehalten.

"*Form III*" ist die erfindungsgemäße kristalline Form zu 5-Amino-2,3-dihydrophthalazin-1,4-dion Natriumsalz.

Der Begriff "*Medizin*" bzw. "*Verwendung in der Medizin*" bezieht sich sowohl auf die Humanmedizin als auch die Veterinärmedizin.

Der Begriff "*Wirkstoff*" umfasst im weiteren Sinne jeden pharmakologisch wirksamen Bestandteil eines Arzneimittels. Ein Wirkstoff kann alleine - als Einzelstoff - oder in einer pharmazeutischen Zubereitung zur Anwendung kommen.

Ein "*Organismus*" im Sinne der Anmeldung ist ein tierisches Lebewesen, insbesondere Mensch, Heim- oder Nutztier.

Der Begriff "*pharmazeutische Zubereitung*" oder "*pharmazeutische Zusammensetzung*" umfasst den erfindungsgemäßen Wirkstoff in jeder pharmakologisch geeigneten, definierten Dosierung und Darreichungsform mit mindestens einem pharmazeutisch geeigneten Hilfsstoff. Der Begriff kann ferner weitere, andere aktive Wirkstoffe allein oder in Kombination umfassen.

Der Ausdruck "*Hilfsstoff*" wird hierin verwendet, um jeden Bestandteil einer pharmazeutischen Zubereitung neben dem Wirkstoff selbst zu beschreiben. Die Wahl eines geeigneten Hilfsstoff hängt von Faktoren wie Applikationsart und Dosierung ab, sowie von der Beeinflussung der Löslichkeit und Stabilität der Zubereitung durch den Hilfsstoff selbst. Pharmazeutische "Hilfsstoffe" sind Stoffe, die dem Fachmann bekannt oder Standardwerken der Pharmazeutik oder offiziellen Arzneibüchern (z.B. Europäische Pharmakopöe) zu entnehmen sind. Als Beispiele für Hilfsstoffe seien beispielhaft genannt: Trägerstoffe, Adjuvanzien, Additive und Zusatzstoffe allgemein, sowie im Speziellen Füllstoffe (Grundlage), Umhüllungsmittel, Schmiermittel, Gleitmittel, Formentrennmittel, Fließregulierungsmittel, Befeuchtungsmittel, Zerfallsbeschleuniger, Sprengmittel, Süßungsmittel, Aromen, Geschmackskorrigentien, Geschmacksstoffe, Konservierungsmittel, Dispersionsmittel, Farbstoffe, Lösungsmittel, Lösungsvermittler (Netzmittel) und Lösungsverlangsamer, Resorptionsbeschleuniger und -verlangsamer (Retard-Präparate), Eindringungsverstärker oder Penetrationsbeschleuniger ("penetration enhancers"), Verdünnungsmittel, Gelbildner, Verdickungsmittel, Bindemittel, Absorptionsmittel, Geschmacksstoffe, Aromastoffe, Antioxidantien, oberflächenaktive Substanzen, Emulgatoren, Triglyceride, pH-regulierende Mittel (Puffer), Fettungsmittel, Konsistenzgeber, Hydrotope und Substanzen, die durch eine chemische Reaktion mit Wasser Gas erzeugen.

"*Adjuvanzien*" sind Hilfsstoffe, die die Wirkung des Wirkstoffes verstärken (können), wie z.B. Lösungsvermittler, oberflächenaktive Substanzen, Resorptions- und Penetrationsbeschleuniger.

Die Begriffe "*Puffer*", "*Puffersystem*" und "*Pufferlösung*" beziehen sich auf die Fähigkeit eines Systems, insbesondere einer wässrigen Lösung, einer pH-Veränderung durch Zugabe von Säure oder Base oder durch Verdünnung mit einem Lösungsmittel in dem Maße zu widerstehen, das durch die jeweilige Pufferkapazität vorgegeben ist.

Der Begriff "*Sprengmittel*" bezieht sich auf Materialien, die einer Zusammensetzung zugegeben werden, damit diese leichter auseinandergebrochen werden kann (z.B. bei Tabletten) oder leichter quillt.

Der Begriff "*Bindemittel*" bezieht sich auf Materialien, die einer Zusammensetzung zugegeben werden, damit die einzelnen in der Zusammensetzung enthaltenen Stoffe, insbesondere Feststoffe mit einem feinen Verteilungsgrad, sich fest miteinander, oder mit einer anderen Zusammensetzung oder mit einer Unterlage verbinden können.

Der Begriff "*Schmiermittel*" bezieht sich auf Substanzen, die der Dosierungsform beigefügt werden, um Tabletten, Granulate etc. nach ihrer Verpressung leichter z.B. aus einer Pressform oder Austrittsdüse lösen zu können, indem sie die Reibung oder den Abrieb verringern.

"*Gleitmittel*" sind Materialien, die ein Zusammenbacken unterbinden und die Fließeigenschaften der einzelnen Bestandteile der Zusammensetzung verbessern, so dass der Fluss glatt und gleichmäßig erfolgt.

"*Färbemittel*" oder auch "*Farbstoffe*" sind Hilfsmittel, die der Zusammensetzung bzw. der Darreichungsform eine Färbung verleihen.

Der Begriff "*phasenrein*" beziehungsweise "*Phasenreinheit*" meint die Festphasenreinheit eines polymorphen Wirkstoffs von mindestens 90%, bevorzugt mindestens 95%, besonders bevorzugt mindestens 98%, höchst bevorzugt mindestens 99%.

Der Begriff "*Wirkung*" beschreibt jede intrinsische Wirkweise eines Wirkstoffs.

Der Begriff "*Lagerstabilität*" oder "*Lagerbeständigkeit*" beschreibt die Stabilität, insbesondere den Erhalt der Phasenreinheit, eines Wirkstoffes als Einzelstoff und/oder in einer pharmazeutischen Zubereitung.

## Neue kristalline Form III zu 5-Amino-2,3-dihydrophthalazin-1,4-dion Natriumsalz

Die vorliegende Erfindung beinhaltet eine neue kristalline Anhydratform (Form III) von 5-Amino-2,3-dihydrophthalazin-1,4-dion Natriumsalz, die durch ein Röntgenpulverbeugungsdiagramm gekennzeichnet ist, welches mit einem Bragg-Brentano-Diffraktometer (STOE STADI P), ausgestattet mit einem DECTRIS Mythen1K Detektor unter Verwendung einer monochromatisierten (Ge111-Monochromator) Kupferstrahlung Cu K($\alpha$1) (Wellenlänge $\lambda$ = 1,54187 Å) erstellt wurde und in D - oder 2-Theta-Werten ausgedrückt ist (Tabelle 1), wobei mit "D" die Interplanarabstände und mit "2-Theta" die 2-Theta-Winkel in Grad bezeichnet sind. $I/I_o$ gibt die relativen Intensitäten der Reflexe wieder, in Tabelle 1 sowohl in Prozent (%) sowie in einer ordinalen Einteilung (rel) dargestellt.

Tabelle 1: D-Werte, 2-Theta-Werte und relative Intensitäten $I/I_o$ für Form III

| D | 2-Theta | $I/I_o$ (%) | $I/I_o$ (rel) | |
|---|---|---|---|---|
| 13,131 | 6,73 | 100,00 | vst | |
| 7,987 | 11,07 | 3,84 | vw | |
| 7,186 | 12,31 | 10,06 | w | |
| 6,566 | 13,48 | 5,07 | w | |
| 6,512 | 13,59 | 25,59 | m | |
| 5,372 | 16,49 | 11,21 | w | |
| 3,994 | 22,24 | 2,43 | vw | |
| 3,662 | 24,29 | 22,51 | m | wobei gilt: |
| 3,406 | 26,14 | 15,19 | m | |
| 3,288 | 27,10 | 41,04 | w | vw = very weak (0 % < $I/I_0 \leq$ 5 %) |
| 3,283 | 27,14 | 10,62 | w | w = weak (5 % < $I/I_0 \leq$ 15 %) |
| 3,222 | 27,67 | 44,69 | st | m = medium (15 % < $I/I_0 \leq$ 35 %) |
| 3,215 | 27,72 | 19,65 | m | st = strong (35 % < $I/I_0 \leq$ 75 %) |
| 3,127 | 28,52 | 3,20 | vw | vst = very strong (75 % < $I/I_0 \leq$ 100 %) |
| 2,889 | 30,93 | 4,80 | w | |

Anhydratform III ist weiterhin gekennzeichnet durch kristallographische Daten (Zellparameter der Elementarzelle, Kristallsystem, Raumgruppe) (Tabelle 2) sowie die Kristallstruktur und daraus resultierende Packungsmotive, dargestellt als Kugel-Stabmodelle und als Kalottenmodelle (Abb. 2). Während die Kalottenmodelle durch die dargestellte Raumerfüllung der Atome eine plastische Darstellung der Moleküle erzeugen und somit deren Orientierung entlang der jeweils betrachteten Raumrichtung (Elementarzellachsen) in der Kristallstruktur deutlich wird, lassen die Kugel-Stab-Modelle durch den Verzicht auf die Darstellung der Raumerfüllung einen besseren Tiefenblick ins Strukturinnere zu. Die Strukturlösung und Rietveld-Verfeinerung der Kristallstruktur wurde aus XRPD-Daten, aufgenommen an einem Bragg-Brentano-Diffraktometer (STOE STADI P), ausgestattet mit einem DECTRIS Mythen1K Detektor unter Verwendung einer monochromatisierten (Ge111-Monochromator) Kupferstrahlung Cu K($\alpha$1) (Wellenlänge $\lambda$ = 1,54187 Å) mittels der Software TOPAS Academic durchgeführt.

Tabelle 2: Daten zur Strukturlösung und Rietveld-Verfeinerung von Form III aus XRPD-Daten (Zahlen in Klammern repräsentieren die Standardabweichungen des jeweiligen Wertes):

| | | |
|---|---|---|
| Chemische Formel | | $C_8H_6N_3NaO_2$ |
| Molare Masse [g/mol] | | 199.15 |
| Kristallsystem | | Monoklin |
| Raumgruppe | | $P\ 2_1/c$ |
| Achsenlängen der Elementarzelle | *a* [Å] | 8.0364(2) |
| | *b* [Å] | 3.69765(5) |
| | *c* [Å] | 26.4246(7) |
| Winkel zwischen den Achsen der Elementarzelle | $\alpha$ [°] | 90 |
| | $\beta$ [°] | 96.346(2) |
| | $\gamma$ [°] | 90 |
| Volumen der Elementarzelle | $V$ [Å$^3$] | 780.416(33) |
| Zahl der Formeleinheiten pro Elementarzelle / Zahl der Formeleinheiten pro asymmetrischer Einheit | *Z*/*Z'* | 4/1 |

(fortgesetzt)

| | | |
|---|---|---|
| Packungsdichte (Volumen aller Atome in der Zelle / Volumen Elementarzelle) | $\rho$ [g cm$^{-3}$] | 1.69491(7) |
| Strukturfaktor der nullten Ordnung (für h=k=l = 0). | F(000) | 408 |
| Messtemperatur [°C] | | 295(2) |
| Gemessener Bereich [°2θ] | | 3 - 80.085 |
| Verwendete Reflexe | | 481 |
| Verfeinerte Parameter | | 66 |
| Gütefaktoren (Residual-Werte, R-Faktoren) der Strukturverfeinerung: | $R_p$ | 0.0214 |
| | $R_{wp}$ | 0.0296 |
| | $R_{exp}$ | 0.0104 |
| | $R_{Bragg}$ | 0.0148 |

Im Fall von Röntgenexperimenten gibt der nicht-dispersive Strukturfaktor F(000) die Anzahl der Elektronen pro Elementarzelle an. Die Gütefaktoren $R_p$ und $R_{wp}$ beziehen sich auf die Güte der Anpassung des berechneten an das gemessene Profil eines XRPDs. $R_{exp}$ repräsentiert den niedrigstmöglichen $R_{wp}$-Wert, welcher unter Anwendung des theoretischen Strukturmodells und der Anzahl der verfeinerten Parameter erreicht werden kann. $R_{Bragg}$ bezieht sich auf die Richtigkeit des verfeinerten Strukturmodells in Bezug auf die extrahierten Reflexintensitäten.

Die erfindungsgemäße Form III ist zudem gekennzeichnet durch ein Festkörper-FT-IR-Spektrum, welches mit einem JASCO FT/IR 6100 FT-IR-Spektrometer, ausgestattet mit einem DLATGS Detektor erstellt wurde. Das Spektrum wird durch eine graphische Auftragung der gemessenen Transmission in % gegen die entsprechende Wellenzahl cm$^{-1}$ ausgedrückt (Abb. 3).

Die erfindungsgemäße Form III ist weiterhin gekennzeichnet durch ein Raman-Spektrum, welches mit einem LabRam-Spektrometer, mit einer Auflösung von 2 cm$^{-1}$, ausgestattet mit einem Helium-Neon-Laser (Wellenlänge 628,8 nm) und einem 100x Objektiv, erstellt wurde. Das Spektrum wird durch eine graphische Auftragung der gemessenen relativen Intensität in % gegen die entsprechende Wellenzahl cm$^{-1}$ ausgedrückt (Abb. 4).

Die erfindungsgemäße Anhydratform (Form III) weist bevorzugt einen Kristallwassergehalt von $\leq$ 0,4% auf.

Vorteilhafte physikalische Eigenschaften:

Die Erfinder haben überraschend gefunden, dass Form III positive physikalische Eigenschaften für die pharmazeutische Verarbeitung und Anwendung besitzt, einschließlich Thermostabilität, Lagerbeständigkeit, Löslichkeit, Schüttdichte, kristalliner Form und Phasenreinheit. Diese sind vorteilhaft für die pharmazeutische Herstellung und Weiterverarbeitung im Vergleich zu beispielsweise Hydraten (Di-, Tri- und Hexahydrat), bei denen es zu Veränderungen im Wassergehalt und dadurch bedingt zu Formulierungsproblemen kommen kann, z.B. durch Gewichtsveränderungen des aktiven Wirkstoffs während der Tablettenpressung, Verkapselung oder Sterilisation.

Für die Anwendung in der Medizin ist Form III vorteilhaft gegenüber den von Rybakov et al. (2014: Crystallography Reports, 59, 383-393) beschriebenen Mischformen, da in der pharmazeutischen Anwendung Reinformen aufgrund ihrer besseren Charakterisierbarkeit und Standardisierbarkeit immer gegenüber Mischformen vorzuziehen sind. Dies ist insbesondere von Bedeutung, da verschiedene Polymorphe unterschiedliche pharmakokinetische Eigenschaften haben können und daher bei Mischformen keine gleichbleibende Bioverfügbarkeit gewährleistet werden kann.

Die erfindungsgemäße Form lässt sich rasterelektronenmikroskopisch von den bekannten Formen I und II unterscheiden: Form II weist vor allem nadelähnliche Kristallite oktaedrischer Struktur von mehreren Mikrometern Länge auf, die schichtweise aufgebaut sind; im REM der Form I zeigen sich größtenteils morphologisch uneinheitliche Kristallite mit abgerundeten Kanten, die pulverförmig agglomerieren. Die erfindungsgemäße Form III zeigt ebenfalls eher nadelförmige Kristallite, im Gegensatz zu Form II verfügen diese Kristallite jedoch an ihren Spitzen über abgerundete Kanten (Abb. 5).

Aus ihrer Kristallform ergeben sich für die erfindungsgemäße Form vorteilhafte Eigenschaften für die pharmazeutische Verarbeitung: So eignen sich Form II und Form III aufgrund ihrer nadelförmigen Kristallpartikelform zum Beispiel besser zur direkten Tablettenverpressung. Da die Kristallite der erfindungsgemäßen Form III jedoch deutlich kleiner ausfallen als jene der Form II, verfügen Sie - analog zu Form I - über eine höhere Rieselfähigkeit und damit auch eine verbesserte Filtrierbarkeit als dies bei Form II der Fall ist. Auch die Schüttdichte der erfindungsgemäßen Form III

ist deutlich höher als jene der Form II (siehe Beispiel 1).

Es kann somit konstatiert werden, dass die erfindungsgemäße Form III die jeweiligen Vorteile von Form II gegenüber Form I und von Form I gegenüber Form II in sich vereint. Somit verfügt Form III über physikalische Eigenschaften, die von großem Vorteil in der technischen Arzneimittelproduktion sind, insbesondere bei der Herstellung von festen Zubereitungen wie zum Beispiel Tabletten, Kapseln und Pulver. Aber auch im Rahmen der Herstellung von flüssigen Zubereitungen bieten diese Eigenschaften Vorteile durch die bessere Handhabbarkeit des erfindungsgemäßen Wirkstoffes im Produktionsprozess. Hervorzuheben ist dabei der so genannte Hausner-Faktor, der Quotient aus Schüttvolumen und Stampfvolumen. Desto näher der Hausner-Faktor bei 1 liegt, desto weniger muss mit Problemen bei der Steuerung der Dosiergenauigkeit gerechnet werden. Gerade bei Wirkstoffen mit hohem Schüttvolumen, also geringer Schüttdichte, ist von einem deutlichen Unterschied zwischen Schütt- und Stampfvolumen zu rechnen und der Hausner-Faktor liegt entsprechend hoch. Ansonsten notwendig werdende an die Herstellung anschließende Schritte wie z.B. Mahlen, Zerkleinern im Luftstrom oder Sieben sind daher für die erfindungsgemäße Form III nicht notwendig. Die Bereitstellung des hierzu geeigneten Equipments nach GMP-Vorschriften entfällt und es wird - in der Regel teure - Produktionszeit eingespart. Dies ist ein eindeutiger wirtschaftlicher Vorteil.

Wie schon in der WO2011/107295A1 für die Anhydratformen Form I und Form II beschrieben, zeigt auch die erfindungsgemäße Anhydratform eine sehr hohe Thermostabilität mit Zersetzungstemperaturen von deutlich über 300°C. So erfolgt die Zersetzung von Form III erst ab einer Temperatur von 391,7°C ± 10°C. Die Bestimmung erfolgte mit Hilfe der simultanen Thermogravimetrie - Differential-Thermoanalyse an einem Linseis L81-077 gekoppelt mit Massenspektroskopie-Messungen mit einer Netzsch STA 449 C (Thermowaage) mit MS- und FTIR-Kopplung über 30-600°C in synthetischer Luft (4 $N_2$ : 1 $O_2$) und einer Heizrate von 10°C/min. Die Daten wurden mit der werksseitigen Software Proteus ausgewertet.

Die thermoanalytischen Daten bestätigen die Annahme der Erfinder, dass die kristalline Form III ebenso wie die Formen I und II vorteilhafte Eigenschaften in Bezug auf Stabilität und Lagerungsbeständigkeit gegenüber dem Dihydrat mit einer Festphasenumwandlung bei 85°C (WO2011/107295A1) aufweist. Auch für andere Hydrate (z.B. Trihydrat, Hexahydrat) ist davon auszugehen, dass eine Festphasenumwandlung bereits bei Temperaturen unter 100°C einsetzt. Diese Eigenschaft begünstigt ferner die pharmazeutische Verarbeitung der erfindungsgemäßen kristallinen Form, indem sie diese gegenüber Verfahrensschritten mit hohem Energieeintrag, z.B. Sterilisation oder Vermahlung, unempfindlich macht. Bei einer Wirkstoffherstellung, die eine Endsterilisation erlaubt, entfällt die GMP-Anforderung einer geschlossenen Sterilproduktion über alle Produktionsschritte. Dies ist ein erheblicher Kostenvorteil.

Die einzelnen Formen unterscheiden sich auch durch die unterschiedliche stöchiometrische Koordination des Natrium-Kations und der 5-Amino-2,3-dihydrophthalazin-1,4-dion Anionen. Während in Form I ein Natrium-Kation von insgesamt 6 5-Amino-2,3-dihydrophthalazin-1,4-dion Anionen in einem trigonalen Prisma koordiniert wird, sind es in der Form II und III nur 5 5-Amino-2,3-dihydrophthalazin-1,4-dion Anionen.

Über die Paulingschen Veknüpfungsregeln (vgl. Linus Pauling (1929): Journal of the American Chemical Society, 51, 1010-1026) lassen sich u. a. Aussagen zur Stabilität ionisch aufgebauter Kristallstrukturen treffen. Die dritte Regel besagt, dass das Auftreten von gemeinsamen Kanten und insbesondere Flächen die Stabilität in Koordinationspolyedern verringern. Hintergrund dafür ist die elektrostatische Abstoßung von Kationen, welche bei Verringerung des Abstands größer wird. So führt eine Flächenverknüpfung der Koordiationspolyeder benachbarter Kationen in einer Kristallstruktur zu einem geringeren Abstand zwischen diesen als eine Kantenverknüpfung oder eine Spitzenverknüpfung der Koordinationspolyeder.

In Form I wird ein Natrium-Kation von insgesamt 6 5-Amino-2,3-dihydrophthalazin-1,4-dion Anionen in einem trigonalen Prisma koordiniert. Diese Koordination führt zu einer Flächenverknüpfung zu jeweils zwei benachbarten Prismen und zu einem effektiven Abstand von 3,395 Å zwischen zwei benachbarten Na-Kationen. In den Formen II und III wird ein Natrium-Kation von nur 5 5-Amino-2,3-dihydrophthalazin-1,4-dion Anionen koordiniert mit einer Kantenverknüpfung zu jeweils zwei direkt benachbarten Polyedern. Dies führt zu einem effektiven Abstand von 3.510 Å zwischen zwei benachbarten Na-Kationen in Form II und 3,578 bzw. 3,595 Å zwischen je zwei benachbarten Na-Kationen in Form III.

Ausgehend von der dritten Paulingschen Verknüpfungsregel und den effektiven Abständen der Na-Kationen in den jeweiligen Kristallstrukturen kann daher davon ausgegangen werden, dass die Koordination von Form III stabiler als die jeweiligen Koordinationen von Form II und Form I ist.

Darüber hinaus zeigt sich die kristalline Form III substantiell stabil in Bezug auf eine Veränderung des Wassergehalts, sodass Formulierungsprobleme infolge Veränderungen des Wirkstoffgewichts bei der pharmazeutischen Weiterverarbeitung (z.B. Tablettierung, Verkapselung etc.) deutlich seltener auftreten.

Die neue kristalline Form stellt weiterhin einen Vorteil gegenüber dem Stand der Technik dar, da die geringere maximale Löslichkeit (Beispiel 2) dort Vorteile bietet, wo eine verzögerte Wirkstoff-Freisetzung angestrebt wird. Form III eignet sich daher besonders zur Verwendung in pharmazeutischen Zubereitungen für orale und topische Applikationen, insbesondere Tabletten, Kapseln, Cremes, Puder und speziellen Retard-Formulierungen für Anwendungen, in welchen eine verzögerte Wirkstofffreisetzung erwünscht ist.

Aufgrund der Ergebnisse aus "Slurry" Experimenten (Beispiel 3) ist davon auszugehen, dass Form III bei

üblichen Lagerungsbedingungen bei Raumtemperatur stabiler gegenüber Umwandlungsprozessen ist als Form I und Form II. Derartige Umwandlungsprozesse sollten nach GPP nur in sehr geringem Umfang pro Zeitraum oder am besten überhaupt nicht stattfinden. Aufgrund dieses Vorteils wird die Lagerbeständigkeit (shelf life) eines Form III enthaltenden Medikaments deutlich erhöht, was die Verwertbarkeit und den Wert für die produzierende und/oder vertreibende pharmazeutische Industrie deutlich erhöht. Zudem werden derart die Ansprüche an den technischen Aufwand für die Lagerung deutlich vermindert.

Beispiel 1: Schüttdichte

Zum Vergleich der Schüttdichte wurden Reinformen des Dihydrats und der Formen I, II und III hergestellt. Als Ausgangsmaterial für die Umkristallisierungen diente Anhydratform I des 5-Amino-2,3-dihydrophthalazin-1,4-dion Natriumsalzes hergestellt nach den Vorgaben der WO2011/107295A1.

*Herstellung:*

Für die Herstellung des Dihydrats wurden 10 g 5-Amino-2,3-dihydrophthalazin-1,4-dion Natriumsalz in 100 mL $H_2O$ (VE) unter Rühren bei Raumtemperatur gelöst, bis augenscheinlich keine Trübung mehr vorhanden war. Anschließend wurden 900 mL 2-Propanol zur Lösung hinzu gegeben. Nach Auftreten einer Eintrübung der Lösung wurde die entstandene Suspension für weitere 4 h gerührt. Die Suspension wurde unter Verwendung eines Büchnertrichters unter Vakuum filtriert. Der verbleibende kristalline Feststoff wurde bei RT bis zur Massenkonstanz getrocknet.

Die Herstellung der Form I des 5-Amino-2,3-dihydrophthalazin-1,4-dion Natriumsalzes erfolgte durch Lösen von 10 g 5-Amino-2,3-dihydrophthalazin-1,4-dion Natriumsalz in 100 mL $H_2O$ (VE) unter Rühren bei Raumtemperatur, bis augenscheinlich keine Trübung mehr vorhanden war. Zur Lösung wurden 900 mL 2-Propanol hinzu gegeben. Nach Auftreten einer Eintrübung der Lösung wurde die entstandene Suspension umgehend unter Verwendung eines Büchnertrichters unter Vakuum filtriert. Der verbleibende kristalline Feststoff wurde bei RT bis zur Massenkonstanz getrocknet.

Die Herstellung der Form II des 5-Amino-2,3-dihydrophthalazin-1,4-dion Natriumsalzes erfolgte nach Herstellungsbeispiel 4 durch Lösen von 10 g 5-Amino-2,3-dihydrophthalazin-1,4-dion Natriumsalz in 100 mL $H_2O$ (VE) unter Rühren bei Raumtemperatur bis augenscheinlich keine Trübung mehr vorhanden war. Zur Lösung wurden 900 mL 2-Propanol hinzu gegeben. Nach Auftreten einer Eintrübung der Lösung wurde die entstandene Suspension für weitere 4 h gerührt. Die Suspension wurde unter Verwendung eines Büchnertrichters unter Vakuum filtriert. Der verbleibende kristalline Feststoff wurde bei 60°C in einem Trockenofen bis zur Massenkonstanz getrocknet.

Die Herstellung der erfindungsgemäßen Form III des 5-Amino-2,3-dihydrophthalazin-1,4-dion Natriumsalzes erfolgte nach Herstellungsbeispiel 2 - Ausführungsform 1. 10 g 5-Amino-2,3-dihydrophthalazin-1,4-dion Natriumsalz wurden unter Rühren bei 90°C in 3,3 1 DMSO gelöst, bis keine mit bloßem Auge zu erkennende Trübung mehr vorhanden war. Die Lösung wurde unter Anlegen von Vakuum ($8x10^{-3}$ mbar $\pm$ $1x10^{-3}$ mbar) bei 90°C weiter gerührt, bis sich eine Suspension bildet. Die Suspension wurde unter Vakuum ($8x10^{-3}$ mbar $\pm$ $1x10^{-3}$ mbar) bei 90°C getrocknet bis zur Entstehung eines Feststoffes. Der Feststoff wurde mit 2-Propanol aufgeschlämmt und unter Verwendung eines Büchnertrichters unter Vakuum filtriert. Es wurde zweimal mit 2-Propanol nachgewaschen. Der kristalline Feststoff wurde anschließend bei 50°C in einem Trockenofen bis zur Massenkonstanz getrocknet.

Alle hergestellten Formen wurden nach der Trocknung 1 Minute lang mittels eines Vortexmischers aufgeschüttelt, um größere Agglomerate schonend zerfallen zu lassen.

*Messung der Schüttdichte:*

Zur Bestimmung der Schüttdichte ("bulk density") des Dihydrats, der Formen I und II, sowie der erfindungsgemäßen Form III wurde ein gläserner Hohlzylinder (Höhe: 20 mm, Durchmesser: 20,5 mm; Volumen: 6,601 $cm^3$; im Weiteren: "Auffangbehälter") mit abnehmbarem Boden verwendet. Eine Probe des jeweils zu bestimmenden Feststoffs wurde in einen Plastiktrichter für Feststoffe (Durchmesser des Auslasses: 11 mm) mit verschließbarem Auslass gefüllt, welcher in 20 mm Höhe über dem Auffangbehälter angebracht wurde. Der verwendete Trichter wurde dabei jeweils bis zur Hälfte gefüllt, wodurch sichergestellt war, dass auf jeden Fall mehr Probenmaterial zum Einsatz kommt, als für die Füllung des Auffangbehälters benötigt werden. Nach Öffnung des Auslasses rieselte der jeweilige Feststoff in den Auffangbehälter, bis dieser über den oberen Rand gefüllt war. Der Überstand des jeweiligen Feststoffs über dem oberen Rand des Auffangbehälters wurde durch Abstreifen mit einem Spatel entfernt und der gefüllte Auffangbehälter auf einer Feinwaage ausgewogen. Die Bestimmung wurde für jede Form 10 Mal wiederholt und der Mittelwert bestimmt (Tabelle 3).

*Ergebnisse:*

Form III weist sowohl gegenüber den bislang bekannten Anhydratformen als auch gegenüber dem Dihydrat

eine höhere Schüttdichte auf, wobei der Unterschied in der Schüttdichte sowohl zum Dihydrat als auch zur Form II hoch signifikant ist ($p < 0,001$; T-Test, ungepaart, 2-seitig).

Tabelle 3: Schüttdichten [kg/$m^3$] der Formen I-III und des Dihydrats (Mittelwert ± Standardabweichung)

| Form I | Form II | Form III | Dihydrat |
|---|---|---|---|
| 201,9 ± 3,9 | 108,6 ± 3,0 | 203,5 ± 6,7 | 72,3 ± 4,1 |

Beispiel 2: Maximale Löslichkeit in Wasser bei RT

Die maximale Löslichkeit der kristallinen Form III in Wasser bei RT bis zur Entstehung einer gesättigten Lösung wurde bestimmt. Danach ist Form III mit einem Wert von 145 mg/mL schwerer löslich als die Formen I und II (vgl. WO2011/107295A1).

Die Ermittlung der maximalen Löslichkeit von Form III in Wasser bei RT erfolgte mithilfe der UV/Vis-Spektroskopie an einem Agilent Cary 300 UV-Vis Spektrometer. Diesem Vorgang liegt ein linearer Zusammenhang zwischen der Konzentration eines gelösten Feststoffes und der ermittelten Absorption bei einer bestimmten Wellenlänge zugrunde (Lambert-Beersches Gesetz).

Es wurde eine Stammlösung von 0,502 g der Form III in 20 mL $H_2O$ (VE) hergestellt. Zur Erstellung einer Drei-Punkt-Eichgeraden wurden aus dieser mittels entsprechender Verdünnung drei Lösungen mit Konzentrationen von 0,5041 mmol/l, 0,2521 mmol/l und 0,1260 mmol/l hergestellt und deren jeweilige Absorption bei 347 nm bestimmt. Bei dieser Wellenlänge zeigen 5-Amino-2,3-dihydrophthalazin-1,4-dion Natriumsalze ein Absorptionsmaximum im UV-Spektrum. Ferner wurde zur Ermittlung der Maximalkonzentration eine übersättigte Lösung von 0,531 g Form III in 2 mL $H_2O$ (VE) hergestellt. 100 $\mu$L der Lösung wurde um das 2000fache verdünnt und es wurde ebenfalls die Absorption bei 347 nm bestimmt.

Der errechnete Wert für die maximale Löslichkeit von Form III in Wasser bei RT liegt entsprechend bei 145 mg/mL (entspricht einer maximalen Konzentration von 0,73 mol/l).

Beispiel 3: Slurry-Experimente ("Aufschlämmexperimente")

Bei Polymorphen kristalliner Feststoffe treten Phasenumwandlungen je nach thermodynamischer Stabilität der einzelnen Formen auf. Hierbei gilt, dass sich die thermodynamisch weniger stabile (metastabile) Form in die thermodynamisch stabilere umwandelt. Verschiedene Umwandlungsprozesse sind bekannt. Neben Festphasenumwandlungen, bei denen es durch einen Energieeintrag (Temperatur, Druck, usw.) in ein Phasengemisch zum Einsetzen der Umwandlung der thermodynamisch instabileren in die stabilere Form kommt, versteht man unter einer lösungsvermittelten Phasenumwandlung die Auflösung der metastabilen Form zugunsten des Kristallkeimbildung und des Kristallwachstums der stabilen Form. Vor diesem Hintergrund können so genannte Slurry-Experimente Aufschluss über die thermodynamische Stabilität von Polymorphen eines Feststoffs geben.

Zum Vergleich der Stabilität unter Einfluss von Lösungsmittel wurden jeweils 50 : 50 Gew.% Festphasengemische der erfindungsgemäßen Form III und Form I sowie der erfindungsgemäßen Form III und Form II hergestellt.

Je 0,05 g des jeweiligen Festphasengemisches wurden in je 5 mL 2-Propanol, Methanol oder Ethanol suspendiert. Das Gemisch aus Form III und Form I wurde bei RT für 3 Tage gerührt. Das Gemisch aus Form III und Form II wurde bei RT für 24 h gerührt. Die Suspensionen wurden unter Verwendung eines Büchnertrichters unter Vakuum filtriert. Der verbleibende kristalline Feststoff wurde an RT vollständig getrocknet. Röntgenpulverbeugungsdiagramme der resultierenden kristallinen Feststoffe wurden mit einem Bragg-Brentano-Diffraktometer (STOE STADI P), ausgestattet mit einem DECTRIS Mythen1K Detektor unter Verwendung einer monochromatisierten (Ge111-Monochromator) Kupferstrahlung Cu K($\alpha$1) (Wellenlänge $\lambda$ = 1,54187 Å) aufgenommen.

*Ergebnisse:*

Für das Gemisch aus Form II und Form III zeigt sich in den aufgezeichneten Pulverdiffraktogrammen (Abb. 6a) für alle angewendeten Lösungsmittel nach 24-stündigem Rühren ein deutlicher Intensitätszuwachs der Reflexe, welche eindeutig Form III zugeordnet werden können. Daher muss davon ausgegangen werden, dass Form III bei RT das thermodynamisch stabilere Polymorph im Vergleich zu Form II ist.

Für das Gemisch aus Form I und Form III zeigt sich in den aufgezeichneten Pulverdiffraktogrammen (Abb. 6b) für alle angewendeten Lösungsmittel nach 3-tägigem Rühren ein deutlicher Intensitätszuwachs der Reflexe, welche eindeutig Form III zugeordnet werden können. Aufgrund dieser Ergebnisse ist Form III ebenfalls das bei RT thermodynamisch stabilere Polymorph im Vergleich zu Form I.

Biologische Wirksamkeit

Die biologische Wirksamkeit von 5-amino-2,3-dihydrophthalazin-1,4-dion Natriumsalz wurde in der Vergangenheit immer wieder unter Beweis gestellt.

Die vergleichbare biologische Wirksamkeit von Form III zu Form I und II und dem Dihydrat konnte *in vitro* auf LPS-induzierten HL60 Zellen (Beispiel 4) gezeigt werden.

Beispiel 4 - Differenzierte HL60-Zellen

Aus der WO2011/107295A1 z. B. ist bekannt, dass sowohl Form I als auch Form II die Sekretion von Zytokinen, insbesondere von TNF-alpha und IL-6, sowohl *in vitro* als auch *in vivo* reduzieren können. Dieskonnte nun in einem *in vitro*-Modell auch für Form III bestätigt werden. Nicht nur die bereits bekannten Formen, sondern auch die erfindungsgemäße Form III führt zur Reduktion der Zytokinsekretion in einem Modell mit LPS induzierten, zu Makrophagen differenzierten HL-60 Zellen (Human promyelocytic leukemia cells).

Zur Makrophagendifferenzierung wurden HL-60 Zellen vorab mit Phorbol-12-myristat-13-acetat (PMA) behandelt. Im Anschluss wurden die Zellen für eine Stunde mit jeweils 1 mmol/l Dihydrat, Form I, II, oder III vorstimuliert, bevor 100 ng/mL Lipopolysaccharid (LPS) für 24 Stunden zugegeben wurde. Als Kontrolle (100%) wurde die besagte LPS-Menge ohne vorherige Wirkstoffgabe verwendet.Die Sekretion von TNF-alpha und IL-6 aus dem Zellüberstand wurde mittels ELISA über jeweils 3 Messungen bestimmt. Tabelle 4 sowie die Abbildungen 7a und 7b zeigen dabei jeweils den gefundenden Mittelwert MW und die Standardabweichung s (GraphPad Prism 5).

Das Dihydrat des 5-Amino-2,3-dihydrophthalazin-1,4-dion Natriumsalzes, sowie dessen Anhydratformen, Form I, II und III zeigten alle eine mehr oder weniger deutlich reduzierteTNFalpha- und IL-6-Sekretion in differenzierten HL-60 Zellen. Demnach verfügt Form III über eine zum Dihydrat und den Formen I und II vergleichbare biologische Wirksamkeit.

Tabelle 4: TNF-alpha- und IL-6-Sekretion in LPS-stimulierten HL-60 Zellen

| Form | TNF-alpha (pg/mL) | | IL-6 (pg/mL) | |
|---|---|---|---|---|
| | MW | s | MW | s |
| Kontrolle | 30,08 | 3,56 | 42,81 | 2,29 |
| Dihydrat | 26,87 | 0,68 | 40,93 | 2,29 |
| Form I | 25,28 | 4,29 | 41,15 | 1,10 |
| FormII | 22,16 | 0,90 | 36,11 | 3,27 |
| FormIII | 22,15 | 4,69 | 34,86 | 2,98 |

Somit konnte gezeigt werden, dass die erfindungsgemäße Form III des 5-Amino-2,3-dihydrophthalazin-1,4-dion Natriumsalzes in einem Standard-*in vitro*-System eine zu den bereits bekannten Formen I und II sowie dem Dihydrat gleichwertige oder sogar bessere biologische Wirkung auf die essentiellen Entzündungsmarker TNF-alpha und IL-6 hat. Daher ist bei therapeutischen Verwendungen eine zum Stand der Technik vergleichbare oder bessere Wirkung zu erwarten.

Herstellung der erfindungsgemäßen kristallinen Form

Nachfolgend wird die Herstellung der kristallinen Form (III) beispielhaft beschrieben.

Ausgang der Synthese ist im Stand der Technik bekanntes 5-Amino-2,3-dihydrophthalazin-1,4-dion, welches sich zum Beispiel nach folgendem Reaktionsschema herstellen lässt:

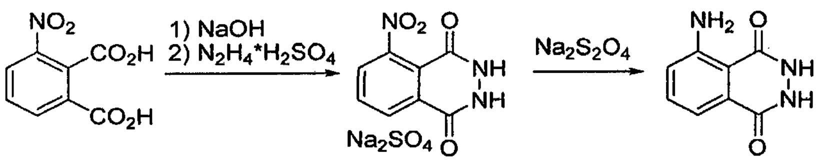


(i) (ii) (iii)

Dargestellt ist die Synthese von 5-Amino-2,3-dihydrophthalazin-1,4-dion (iii) durch Umsetzung von 3-Nitrophthalsäure (i), das in alkalischem Medium durch Hydrazin oder einem seiner Salze, oder andere geeignete Reduktionsmittel, etwa Ammoniumsulfit oder Triethylenglykol, zu 5-Amino-2,3-dihydrophthalazin-1,4-dion über 3-Nitrophthalanhydrid (ii) reduziert werden kann. Geeignete Herstellungsverfahren für 5-Amino-2,3-dihydrophthalazin-1,4-dion finden sich in: Williamson, K. L. In: Macroscale and Microscale Organic Experiments; 2nd ed.; D.C. Heath: Lexington, MA, 1994. Ein weiteres für die Herstellung von 5-Amino-2,3-dihydrophthalazin-1,4-dion geeignetes Verfahren, welches sich eines Raney-Nickel-Katalysators bedient, findet sich beispielsweise in der US 6,489,326 B1.

Ein bevorzugtes spezielleres Verfahren zur mengenunabhängigen Herstellung des Ausgangsproduktes 5-Amino-2,3-dihydrophthalazin-1,4-dion wird in der WO2011/107295A1 beschrieben: dabei werden 1 Äquivalent 3-Nitrophthalsäure und 1,1 Äquivalente Hydrazinhydrat vorgelegt und mit 1,5 Äquivalenten Ethylenglycol vermischt. Dabei wird die Temperatur bis auf 110-200°C erhöht und das Wasser durch Destillation entfernt. Wenn sich kein Wasser mehr bildet, wird die Mischung auf 100°C abgekühlt und 6 Äquivalente Wasser hinzugefügt. Nach Abkühlen der Mischung auf Raumtemperatur und Rühren über Nacht wird das Präzipitat filtriert, mit Wasser gewaschen und anschließend bis zur Massenkonstanz getrocknet.

Im Anschluss können Anhydratform I oder II des 5-Amino-2,3-dihydrophthalazin-1,4-dion Natriumsalzes durch Vermischen von 5-Amino-2,3-dihydrophthalazin-1,4-dion in Natronlauge und Eintropfen dieser Lösung in einen niedermolekularen flüssigen Alkohol hergestellt werden, welcher das Löslichkeitsprodukt des entstandenen 5-Amino-2,3-dihydrophthalazin-1,4-dion Natriumsalzes so herabsenkt, dass Letzteres auszufällen beginnt. Das durch Ausfällen entstandene Präzipitat wird im Anschluss getrocknet. WO2011/107295A1 zum Beispiel stellt verschiedene detaillierte Verfahren zur Herstellung der Anhydratformen I und II bereit.

Gegenstand der Erfindung sind ebenfalls Verfahren zur Herstellung der Anhydratform III durch Lösen einer beliebigen Anhydratform von 5-Amino-2,3-dihydrophthalazin-1,4-dion Natriumsalz (Reinform oder Gemisch) bei mindestens 90°C unter Rühren in DMSO (Dimethylsulfoxid). Das Rühren wird solange fortgeführt bis die Lösung nach zirka einer Stunde vollständig klar ist. Anschließend wird die Lösung bei möglichst gleichbleibender Temperatur (± 20°C) weiter gerührt bis zur Entstehung einer Suspension. Anschließend wird die Lösung weiterhin ohne Rühren bei möglichst gleichbleibender Temperatur (± 20°C) gehalten, bis das DMSO vollständig abgedampft ist. Die Verfahren sind in den folgenden Herstellungsbeispielen näher beschrieben.

Herstellungsbeispiel 1 - Ausführungsform 1

Eine mögliche Herstellung von Form III beinhaltet die Zugabe einer beliebigen Anhydratform (Form I, Form II oder eine Mischform aus Form I und Form II) von 5-Amino-2,3-dihydrophthalazin-1,4-dion Natriumsalz zu DMSO im Gewichtsverhältnis von 5-Amino-2,3-dihydrophthalazin-1,4-dion Natriumsalz zu DMSO von 1 zu 300 bis 1 zu 30.000, bevorzugt 1 zu 300 bis 1 zu 3.000, besonders bevorzugt 1 zu 300 bis 1 zu 350. Die Mischung wird bis zur vollständigen Auflösung, also bis keine mit bloßem Auge zu erkennenden Trübungen mehr vorhanden sind, bei einer Temperatur von 90°C bis 189°C, bevorzugt 100°C bis 170°C, besonders bevorzugt 120°C bis 150°C gerührt, wobei 189°C dem Siedepunkt von DMSO entsprechen.

Die Lösung wird bei 90°C bis 189°C, bevorzugt 100°C bis 170°C, besonders bevorzugt 120°C bis 150°C weiter gerührt, bis sich eine Suspension bildet, die durch das erneute Auftreten von Trübungen gekennzeichnet ist, wobei bevorzugt der Temperaturbereich des vorhergehenden Auflösungsschrittes beibehalten wird, bevorzugt im Rahmen von ± 10°C, besonders bevorzugt ± 1°C.

Die Suspension wird bei 90°C bis 189°C, bevorzugt 100°C bis 170°C, besonders bevorzugt 120°C bis 150°C ohne Rühren getrocknet bis zur Entstehung eines feinpulvrigen Feststoffes, wobei bevorzugt weiterhin der Temperaturbereich des Auflösungsschrittes beibehalten wird, bevorzugt im Rahmen von ± 10°C, besonders bevorzugt ± 1°C.

Herstellungsbeispiel 1 - Ausführungsform 2

Eine bevorzugte Ausführungsform zur Herstellung von Form III beinhaltet das vorab Aufheizen von DMSO auf eine Temperatur von 90°C bis 189°C, bevorzugt 100°C bis 170°C, besonders bevorzugt 120°C bis 150°C, bevor eine beliebige 5-Amino-2,3-dihydrophthalazin-1,4-dion Natriumsalz-Anhydratform (Form I, Form II oder eine Mischform aus Form I und Form II) im Verhältnis von 5-Amino-2,3-dihydrophthalazin-1,4-dion Natriumsalz zu DMSO von 1 zu 300 bis 1 zu 30.000, bevorzugt 1 zu 300 bis 1 zu 3.000, besonders bevorzugt 1 zu 300 bis 1 zu 350 hinzugefügt wird, wobei 189°C dem Siedepunkt von DMSO entsprechen.

Die Mischung wird bis zur vollständigen Auflösung, also bis keine mit bloßem Auge zu erkennenden Trübungen mehr vorhanden sind, bei einer Temperatur von 90°C bis 189°C, bevorzugt 100°C bis 170°C, besonders bevorzugt 120°C bis 150°C gerührt.

Die Lösung wird bei 90°C bis 189°C, bevorzugt 100°C bis 170°C, besonders bevorzugt 120°C bis 150°C gerührt, bis sich eine Suspension bildet, die durch das erneute Auftreten von Trübungen gekennzeichnet ist, wobei bevorzugt

der Temperaturbereich des vorhergehenden Auflösungsschrittes beibehalten wird, bevorzugt im Rahmen von ± 10°C, besonders bevorzugt ± 1°C.

Die Suspension wird bei 90°C bis 189°C, bevorzugt 100°C bis 170°C, besonders bevorzugt 120°C bis 150°C ohne Rühren getrocknet bis zur Entstehung eines feinpulvrigen Feststoffes, wobei bevorzugt weiterhin der Temperaturbereich des Auflösungsschrittes beibehalten wird, bevorzugt im Rahmen von ± 10°C, besonders bevorzugt ± 1°C.

Herstellungsbeispiel 1 - Ausführungsform 3

In einer besonders bevorzugten Ausführungsform des Herstellungsbeispiels lässt sich kristalline Form III wie folgt herstellen:
100 mL DMSO in einem ausreichend großen Becherglas, bevorzugt in einem 200 mL Becherglas, auf mindestens 90°C, bevorzugt mindestens 100°C, besonders bevorzugt mindestens 120°C aufheizen,.

Zugabe von 300 mg 5-Amino-2,3-dihydrophthalazin-1,4-dion Natriumsalz Anhydrat zu DMSO unter ständigem Rühren und Beibehaltung der gewählten Temperatur, bevorzugt im Rahmen von ± 10°C, besonders bevorzugt ± 1°C.

Rühren der 5-Amino-2,3-dihydrophthalazin-1,4-dion Natriumsalz - DMSO - Mischung unter weiterer Beibehaltung der Temperatur, bevorzugt im Rahmen von ± 10°C, besonders bevorzugt ± 1°C, bis zur vollständigen Auflösung, bevorzugt für weniger als 24 Stunden, besonders bevorzugt für weniger als 12 Stunden.

Rühren der 5-Amino-2,3-dihydrophthalazin-1,4-dion Natriumsalz - Lösung in DMSO unter weiterer Beibehaltung der Temperatur, bevorzugt im Rahmen von ± 10°C, besonders bevorzugt ± 1°C, bis zur Bildung einer Suspension, bevorzugt für weniger als 20 Tage, besonders bevorzugt für weniger als 10 Tage.

Trocknen der Suspension unter Beibehaltung der Temperatur, bevorzugt im Rahmen von ± 10°C, besonders bevorzugt ± 1°C, jedoch ohne zu rühren, bis zur Entstehung eines feinpulvrigen Feststoffes, bevorzugt für weniger als 10 Tage, besonders bevorzugt für weniger als 5 Tage.

Substanz auswiegen und Ausbeute ermitteln, welche bevorzugt mehr als 200 mg, besonders bevorzugt annähernd 300 mg beträgt.

Herstellungsbeispiel 2 - Ausführungsform 1

Eine weitere mögliche Herstellung von Form III beinhaltet die Zugabe einer beliebigen Anhydratform von 5-Amino-2,3-dihydrophthalazin-1,4-dion Natriumsalz (Form I, Form II oder eine Mischform aus Form I und Form II) zu DMSO im Verhältnis von 5-Amino-2,3-dihydrophthalazin-1,4-dion Natriumsalz zu DMSO von 1 zu 300 bis 1 zu 30.000, bevorzugt 1 zu 300 bis 1 zu 3.000, besonders bevorzugt 1 zu 300 bis 1 zu 350. Die Mischung wird bis zur vollständigen Auflösung, also bis keine mit bloßem Auge zu erkennenden Trübungen mehr vorhanden sind, bei einer Temperatur von 90°C bis 189°C, bevorzugt 100°C bis 170°C, besonders bevorzugt 120°C bis 150°C gerührt.

Die Lösung wird unter Anlegen von Vakuum im Bereich von $5x10^{-3}$ - $20x10^{-3}$ mbar, bevorzugt $5x10^{-3}$ - $10x10^{-3}$ mbar, besonders bevorzugt $8x10^{-3}$ - $10x10^{-3}$ mbar bei 70 bis 189°C, bevorzugt 90°C bis 170°C, besonders bevorzugt 120°C bis 150°C weiter gerührt, bis sich eine Suspension bildet, die durch das erneute Auftreten von Trübungen gekennzeichnet ist, wobei zunächst bevorzugt der Temperaturbereich des vorhergehenden Auflösungsschrittes beibehalten wird, bevorzugt im Rahmen von ± 20°C, besonders bevorzugt ± 5°C.

Die Suspension wird bei Vakuum im Bereich von $5x10^{-3}$ - $20x10^{-3}$ mbar, bevorzugt $5x10^{-3}$ - $10x10^{-3}$ mbar, besonders bevorzugt $8x10^{-3}$ - $10x10^{-3}$ mbar bei 70°C bis 189°C, , bevorzugt 90°C bis 170°C, besonders bevorzugt 120°C bis 150°C getrocknet bis zur Entstehung eines Feststoffes, wobei bevorzugt weiterhin der Temperaturbereich des Auflösungsschrittes beibehalten wird, bevorzugt im Rahmen von ± 20°C, besonders bevorzugt ± 5°C.

Der Feststoff wird mit 2-Propanol aufgeschlämmt und unter Verwendung eines Büchnertrichters unter Vakuum filtriert. Es wird zweimal mit 2-Propanol nachgewaschen. Der feinpulvrige Feststoff wird anschließend bei 50°C in einem Trockenofen bis zur Massenkonstanz getrocknet.

Herstellungsbeispiel 2 eignet sich insbesondere bei der Verwendung größerer Ansatzmengen, z.B. für die Herstellung von Mengen im industriellen Maßstab.

Herstellungsbeispiel 3 kristalline Form III

Um die Ausbeute der Synthese zu erhöhen, beinhaltet eine weitere mögliche Herstellung von Form III die Zugabe von 5-Amino-2,3-dihydrophthalazin-1,4-dion Natriumsalz zu einem DMSO-$H_2O$-Gemisch (99/1 % v/v bis 1/99 % v/v). Aufgrund der Mischbarkeit von DMSO und $H_2O$ und der höheren maximalen Löslichkeit von 5-Amino-2,3-dihydrophthalazin-1,4-dion Natriumsalz in $H_2O$ verglichen mit DMSO kann ein höherer Anteil von 5-Amino-2,3-dihydrophthalazin-1,4-dion Natriumsalz in Lösung gebracht werden. Die weitere Synthese erfolgt vorzugsweise analog zu den vorangegangen Herstellungsbeispielen.

Die Löslichkeit von 5-Amino-2,3-dihydrophthalazin-1,4-dion Natriumsalz bei variablem Wasseranteil an dem

DMSO-$H_2O$-Gemisch bei Raumtemperatur ist in Abb. 8 dargestellt. Mit zunehmendem Wasseranteil an dem Gemisch steigt die maximale Löslichkeit von 5-Amino-2,3-dihydrophthalazin-1,4-dion Natriumsalz kontinuierlich an. Die höheren $H_2O$-Volumenanteile an dem DMSO-$H_2O$-Gemisch sind nur für den initialen Lösungsschritt von Bedeutung. Bei dem darauffolgenden Erhitzungsschritt (im Hinblick auf eine sinnvolle Ausbeute an Form III im bevorzugten Temperaturbereich > 100°C, siehe Herstellungsbeispiel 1 - Ausführungsformen 1 und 2) verdampft der $H_2O$-Anteil aus dem Lösungsgemisch. Ohne einen DMSO-Anteil mit mindestens einem Gewichtsverhältnis von 5-Amino-2,3-dihydrophthalazin-1,4-dion Natriumsalz zu DMSO von 1 zu 300 (siehe Herstellungsbeispiel 1 - Ausführungsform 1) bildet sich während des finalen Trocknungsschrittes keine oder zumindest keine phasenreine 5-Amino-2,3-dihydrophthalazin-1,4-dion Natriumsalz Form III. Das optimale Mischungsverhältnis von DMSO zu $H_2O$ für den initialen Auflösungsschritt ergibt sich daher aus den jeweiligen industriellen Produktionsbedingungen. Allgemein bevorzugt wird ein Wasseranteil an dem DMSO-$H_2O$-Gemisch von 30 % bis 80%, weiter bevorzugt von 40% bis 70% und am meisten bevorzugt von 50% bis 60%.

Auch Herstellungsbeispiel 3 eignet sich für Verwendung größerer Ansatzmengen und somit für die Herstellung von Mengen im industriellen Maßstab.

Durch den ersten Schritt des eben gezeigten Herstellungsbeispiels, nämlich die Wahl eines geeigneten DMSO-$H_2O$-Verhältnisses kann je nach beabsichtigter Verwendung die maximale Löslichkeit von 5-Amino-2,3-dihydrophthalazin-1,4-dion Natriumsalz eingestellt werden. Diese Möglichkeit ist bei den bisher beschriebenen Herstellungsprozessen für 5-Amino-2,3-dihydrophthalazin-1,4-dion Natriumsalze nicht gegeben. Damit stellt dieser optionale Herstellungsschritt für Form III für sich genommen bereits einen Vorteil gegenüber dem Stand der Technik dar.

Mögliche Anwendungsbeispiele umfassen die industrielle Wirkstoffproduktion und die Herstellung topischer Applikationsformen. In der industriellen Wirkstoffproduktion unter validierten GMP-Bedingungen kann die Einstellung der maximalen Löslichkeit einen erheblichen Vorteil in Bezug auf die Genauigkeit der Menge und die Phasenreinheit der gewünschten Kristallform darstellen. Für die Herstellung von topischen Applikationsformen kann dies von Vorteil sein, da hier oft nicht die maximale Löslichkeit in Wasser als erstrebenswert gilt.

Herstellungsbeispiel 4 kristalline Form II

Eine mögliche Herstellung von Form II beinhaltet zunächst die Herstellung einer Reinform des Dihydrats von 5-Amino-2,3-dihydrophthalazin-1,4-dion Natriumsalz, beispielsweise nach US 6489326 B1. Alternativ kann eine beliebige Form von 5-Amino-2,3-dihydrophthalazin-1,4-dion Natriumsalz unter Rühren in Wasser gelöst werden, bis augenscheinlich keine Trübung mehr vorhanden ist. Hierbei beträgt das Verhältnis von 5-Amino-2,3-dihydrophthalazin-1,4-dion Natriumsalz zu Wasser 1 zu 2 bis 50, bevorzugt 1 zu 5 bis 20, besonders bevorzugt 1 zu 10. Anschließend wird 2-Propanol zur Lösung hinzu gegeben und die entstandene Suspension weiterhin gerührt. Hierbei beträgt das Verhältnis der bereits bestehenden Lösung zu 2-Propanol 1 zu 2 bis 50, bevorzugt 1 zu 4 bis 20, besonders bevorzugt 1 zu 8-10. Die Rührdauer beträgt mindestens 1 Stunde, bevorzugt mindestens 2 h, besonders bevorzugt mindestens 4 h. Die Suspension wird im Anschluss filtriert, bevorzugt unter Vakuum.

Der verbleibende kristalline Feststoff (Dihydrat) wird dann bei einer Temperatur von 60°C bis 100°C, bevorzugt 60°C bis 90°C, besonders bevorzugt 60°C bis 75°C, höchst bevorzugt 60°C bis 65°C in einem Trockenofen bis zur Massenkonstanz getrocknet.

Erfindungsgemäß sollen die in den Herstellungsbeispielen und Ausführungsformen zu Form III gezeigten Modifikationen der Herstellungsanleitung frei miteinander kombinierbar sein, solange sich diese Modifikationen logisch nicht ausschließen.

Medizinische Verwendung:

Aufgrund der erfindungsgemäß gefundenen, zuvor beschriebenen physikochemischen und biologischen Eigenschaften eignet sich die erfindungsgemäße Form III analog den Formen I und II als Wirkstoff zur Verwendung in der Medizin, insbesondere zur Verwendung als antientzündliches und immunregulierendes Mittel, z.B. für die Behandlung von Zuständen mit überschießenden Immunreaktionen und zur Behandlung von Zuständen mit immundefizientem Hintergrund.

Als Zustände mit überschießenden Immunreaktionen seien beispielhaft genannt Abstoßungsreaktionen nach Transplantationen; aktive Autoimmunerkrankungen bzw. Krankheiten mit einer Autoimmunkomponente, insbesondere aktive Rheumatoide Arthritis, schubförmige Multiple Sklerose, lupoide Hepatitis, Polyarteriitis nodosa, Morbus Crohn, Colitis ulcerosa, Dermatomyositis. Morbus Behçet, Morbus Behçet Uveitis, idiopathische thrombozytopenische Purpura, Myasthenia gravis, Lambert-Eaton-Syndrom, Polymyositis, Psoriasis, Psoriasis Arthritis, Morbus Bechterew, paroxysmale nächtliche Hämoglobinurie, ankylosierende Spondylitis, Autoimmun-Thyreoiditiden wie z.B. Hashimoto-Thyreoiditis, Ord-Thyreoiditis oder Morbus Basedow, Lupus erythematodes, Vitiligo, autoimmune Enzephalomyelitis, idiopathische Opticus-Neuritis, sympathische Ophthalmie, anteriore Uveitis, Retinadegeneration, periphere ulzerative Keratitis, bullöses Pemphigoid, chronische Urtikaria, Morbus Duhring, erworbene bullöse Epidermolyse, Alopecia areata, Autoimmunenteropathie, Polyendokrine Autoimmunerkrankungen wie z.B. APECED (Autoimmune Polyendocrinopathy Candidiasis Ectodermal Dystrophy), Schmidts Syndrom und XPID (X-linked Polyendocrinopathy Immunodeficiency and Diarrhea Syndrome), chronische Gastritis, Dermatomyositis, Diabetes mellitus Typ 1, Diabetes mellitus Typ 2, endokrine Orbitopathie, Glomerulonephritis, Goodpasture-Syndrom, Granulomatose mit Polyangiitis, Guillain-Barre-Syndrom, Lichen sclerosus, Lichen mucosae, lineare IgA-Dermatose, mikroskopische Polyangiitis, myalgische Enzephalomyelitis, Narkolepsie, PANS (Pediatric Autoimmune Neuropsychiatric Syndrome), wie z.B. PANDAS (Pediatric Autoimmune Neuropsychiatric Disorders Associated with Streptococcal infections), Pemphigus foliaceus, Pemphigus seborrhoicus, Pemphigus vulgaris, Polychondritis, Polymyalgia rheumatica, rheumatisches Fieber, SAPHO-Syndrom (Synovitis, Akne, Pustulitis, Hyperostose, Osteitis), Sarkoidose, Sjögren-Syndrom, Sklerodermie, Stiff-Man-Syndrom, Purpura Schönlein-Henoch, Zöliakie, akute disseminierte Enzephalomyelitis, Antiphospholipid-Syndrom, autoimmune Kardiomyopathie, autoimmune hämolytische Anämie, autoimmune Innenohrerkrankung, autoimmunes lymphoproliferatives Syndrom, autoimmune Pankreatitis, autoimmunes polyendokrines Syndrom, autoimmune Progesteron-Dermatitis, Chagas-Krankheit, chronisch inflammatorische demyelinisierende Neuropathie, chronisch rekurrente multifokale Osteomyelitis, COPD (chronisch-obstruktive Lungenkrankheit), Churg-Strauss-Syndrom, Kälte-Agglutinin-Krankheit, Lipomatosis dolorosa, Endometriose, eosinophile Fasciitis, Hashimoto-Enzephalopathie, Akne inversa, interstitielle Zystitis, Kawasaki-Syndrom, Sharp-Syndrom, Neuromyotonie, Opsoklonus-Myoklonus-Syndrom, primäre biliäre Zirrhose, Raynaud-Syndrom, Restless-Legs-Syndrom, transverse Myelitis und Vaskulitis;

aplastische Anämie; Pemphigus; Pemphigoid; endogene Uveitis; nephrotisches Syndrom und atopische Dermatitis;

sowie septische Zustände z.B. ausgelöst durch Infektionen mit gram-negativen oder grampositiven Bakterien, z.B. MRSA (Methicillin-resistenter Staphylokokkus aureus), oder mykotischen Erregern, und Systemisches inflammatorisches Response-Syndrom (SIRS) ausgelöst durch andere (z.B. immunologische oder chemische) Faktoren.

Als Zustände mit immundefizientem Hintergrund seien beispielhaft genannt häufige grippale Infekte; rezidivierende Atemwegsinfekte; rezidivierende Infekte der ableitenden Harnwege; Müdigkeit; Schwäche; Konzentrationsstörungen unbekannter Genese; Rekonvaleszenz;

chronische Virusinfektionen, insbesondere humane Immundefizienzviren (z.B. HIV-1, HIV-2), Hepatitis B, Hepatitis C, Enzephalitis, Herpes zoster, Herpes simplex, Innenohrinfekte, Varizellen, Masern, Zytomegalie, Epstein-Barr, Adenoviren, Humane Papillomaviren und Parvoviren, wie z.B. Amdoviren, Bocaviren, Dependoviren, Erythroviren und Parvovirus spec.;

verschiedene onkologische Erkrankungen, insbesondere Haarzellenleukämie, Myeloische Leukämie, Multiples Myelom, Follikuläre Lymphome, Kaposi-Sarkom, Kutanes T-Zell-Lymphom, Nasopharynxkarzinom, Karzinoid, Nierenkarzinom, Harnblasenkarzinom, Basalzellkarzinome, metastasierende Karzinome und Malignes Melanom;

septische Granulomatose, Neutropenie; Feigwarzen; Keratosen;

Autoimmunerkrankungen; insbesondere nicht aktive Stadien, wie zum Beispiel schubförmige Multiple Sklerose zwischen den Schüben; Radiogene Kolitis, Divertikelkrankheit;

Allergien, insbesondere Heuschnupfen, Polymorphe Lichtdermatose, Ekzem, Neurodermitis; Enteritis; Colitis;

sowie vor, während und nach Chemo- und Strahlentherapien.

Zusammenfassend lässt sich sagen, dass die erfindungsgemäße Form sich prinzipiell zur Verwendung bei der Behandlung aller entzündlichen Erkrankungen eignet, welche mit einem mehr oder weniger deutlichen Anstieg proinflammatorischer Zytokine, insbesondere dem Anstieg von IL-6 und TNF-alpha, verbunden sind. Dies ist neben den bislang genannten zum Beispiel auch der Fall im Rahmen der Wundheilung, z.B. nach chirurgischen Eingriffen, bei sich verselbstständigenden Immunprozessen, wie z.B. Keratitis sicca oder bei akuten und chronischen Entzündungen unklarer Genese, wie z.B. Tendovaginitis oder Osteoarthritis.

## Pharmazeutische Zubereitungen und Applikationsformen

Pharmazeutische Zubereitungen des erfindungsgemäßen Wirkstoffs, einzeln oder in Kombination mit Adjuvanzien und Standardtherapien, können erfindungsgemäß in flüssiger und fester Form formuliert und in jeder pharmazeutisch geeigneten Weise verabreicht werden, vor allem, aber nicht ausschließlich, intravenös, intramuskulär, topisch (z. B. konjunktival als Augentropfen, oder z.B. transdermal als Salbe oder Substanz-eluierende Wundauflage), parenteral (z.B. subkutan, intramuskulär, intravenös, intraarteriell oder intradermal), vaginal, rektal, nasal, oder oral, einschließlich sublingual und bukkal, sowie in Form von Substanz-eluierenden Implantaten.

Flüssige Formen können sein: z.B. Lösungen (z.B. für Injektionen und Infusionen), Säfte, Sirups, Tropfen, Tees, Suspensionen in wässrigen oder nicht-wässrigen Flüssigkeiten, Emulsionen, insbesondere Öl-in-Wasser-Flüssigemulsionen oder Wasser-in-Öl-Flüssigemulsionen und Sprays.

Feste Formen, insbesondere für die orale Verabreichung, können sein: Tabletten, Dragees, Kapseln, Pillen, Puder, Pulver, Granulate. oder weitere, dem Fachmann geläufige und als geeignet erscheinende Formen, z.B. Suppositorien.

Für topische Applikationen des erfindungsgemäßen Polymorphs sind Cremes, Emulsionen, Lotionen, Gele,

Pasten, Puder, Salben und Suspensionen geeignet.

Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird.

Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Ein Granulat wird hergestellt, indem die für das Pulvergemisch in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Pflanzen-Schleim (z.B. Akazie) oder Lösungen aus Zellulose- oder Polymermaterialen benetzt und durch ein Sieb gepresst wird.

Alternativ kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, deren Salzen (Stearate), Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden.

Kapseln werden hergestellt, indem ein Pulvergemisch wie oben beschrieben hergestellt und geformte Gelatinehüllen damit befüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Zur Herstellung einer Darreichungsform als Zäpfchen mit den erfindungsgemäßen Verbindungen werden Wachse mit einem niedrigen Schmelzpunkt sowie ein Gemisch aus Fettsäureglyceriden wie Kakaobutter zuerst zum Schmelzen gebracht, dann der erfindungsgemäße Wirkstoff durch Rühren oder andere Mischmethoden homogen darin dispergiert. Die geschmolzene homogene Mischung wird dann in geeignete Gussformen überführt, abkühlen gelassen und somit verfestigt.

Die erfindungsgemäße Anhydratform kann mit allen herkömmlich bekannten Trägerstoffen gemischt werden, bei festen Darreichungsformen z.B. mit pflanzlichen und tierischen Fetten, Wachsen, Paraffinen, Stärke, Tragant, Zellulose-Derivaten, Polyethylenglykolen, Silikonen, Bentoniten, Kieselsäure, Talkum, Zinkoxid oder Gemischen der vorgenannten Substanzen. So lässt sich beispielsweise bei der oralen Verabreichung in Form einer Tablette oder Kapsel die Wirkstoffkomponente mit einem oralen, nicht-toxischen und pharmazeutisch verträglichen inerten Trägerstoff, wie z.B. Ethanol, Glycerin, Wasser u.a. kombinieren.

Bei flüssigen Darreichungsformen und Emulsionen sind als Trägerstoffe z.B. Lösungsmittel, Solubilisierungsmittel, Emulgatoren wie z.B. Wasser, Ethanol, Isopropanol, Ethylcarbonat, Ethylacetat, Benzylalkohol, Benzylbenzoat, Propylenglykol, 1,3-Butylglykol, Baumwollsaatöl, Erdnussöl, Olivenöl, Rizinusöl, Sesamöl, Glycerol-Fettsäureester, Polyethylglykole, Fettsäureester von Sorbitan oder Gemische der vorgenannten Substanzen geeignet.

Erfindungsgemäße Suspensionen können herkömmliche bekannte Trägerstoffe wie Verdünnungsmittel (z.B. Wasser, Ethanol oder Propylenglykol), ethoxylierte Isostearylalkohole, Polyoxyethylen und Polyoxyethylensorbitanester, mikrokristalline Zellulose, Bentonite, Agar-Agar, Tragant oder Gemische der vorgenannten Substanzen enthalten.

Hilfsstoffe können zum Beispiel auch Einfluss auf die Verteilung eines Wirkstoffs in unterschiedlichen Geweben und Organen nehmen oder die Wirkdauer oder Wirkgeschwindigkeit von Arzneiformen verändern, etwa durch Beschleunigung der Resorption (beispielsweise durch Dimethylsulfoxid, Nikotinsäure, Hyaluronidase, Taurin) oder durch Verzögerung des Wirkungseintritts, z.B. in Depotarzneiformen beispielsweise durch Polyactid-co-Glycolid (PLGA).

Bei topischen Applikationsformen spricht man von Eindringungsverstärkern ("penetration enhancers"). Hierzu zählen z.B. Isopropylmyristat, Ölsäure, Natriumlaurylsulfat und 1, 2-Propandiol.

Flüssige Darreichungsformen umfassen Lösungen, Suspensionen und Emulsionen. Beispiele sind isotonische Kochsalzlösungen, Ringerlösungen, Ringer-Lactat-Lösungen, Ringer-Acetat-Lösungen, Wasser und Wasser-Propylenglykol-Lösungen für parenterale Injektionen oder der Zusatz von Süßungsmitteln und Trübungsmitteln für orale Lösungen, Suspensionen und Emulsionen. Flüssige Darreichungsformen können auch Lösungen für intranasale Verabreichung und Augentropfen umfassen.

Des Weiteren können Pufferlösungen Bestandteile pharmazeutischer Zusammensetzungen sein. Bevorzugte Puffersysteme können aus der Gruppe bestehend aus Formiat, Lactat, Benzoesäure, Oxalat, Fumarat, Anilin, Acetat-Puffer, Zitrat-Puffer, Glutamat-Puffer, Phosphat-Puffer, Succinat, Pyridin, Phthalat, Histidin, MES (2-(N-Morpholino)ethansulfonsäure), Maleinsäure, Cacodylat (Dimethylarsenat), Carbonsäure, ADA (N-(2-Acetamido)iminodiessigsäure), PIPES (4-piperazin-bis-ethansulfonsäure); BIS-TRIS-Propan (1,3-Bis[tris(hydroxymethyl)-methylamino]-propan), Ethylendiamin, ACES (2-[(2-Amino-2-oxoethyl)amino]ethansulfosäure), Imidazol, MOPS (3-(N-Morphin)-propansulfonsäure), Diethylmalonsäure, TES (2-[Tris(hydroxymethyl)methyl]aminoethansulfonsäure) und HEPES (N-2-Hydroxylethylpiperazin-N'-2-ethansulfosäure-Puffer) sowie weitere Puffer mit einem pKa zwischen 3,8 bis 7,7 ausgewählt werden.

Bevorzugt werden Carbon-Säurepuffer wie Acetat- und Dicarbonsäure-Puffer wie Fumarat, Tartrat und Phthalat sowie Tricarbonsäure-Puffer wie Citrat. Eine weitere Gruppe bevorzugter Puffer sind anorganische Puffer wie Sulfat-, Borat-, Carbonat-, Oxalat-, Kalziumhydroxid- und Phosphat-Puffer. Noch eine weitere Gruppe bevorzugter Puffer sind Stickstoffhaltige Puffer wie Imidazol, Diethylendiamin und Piperazin. Des Weiteren bevorzugt werden Sulfonsäure-Puffer wie TES, HEPES, ACES, PIPES, TAPS ([(2-Hydroxy-1,1bis(hydroxymethyl)ethyl)amino]-1-propansulfonsäure), EPPS (4-(2-Hydroxyethyl)piperazin-1-propansulfonsäure), MOPS (4-Morpholinepropansulfonsäure) und BES (N,N-Bis(2-hydroxyethyl)-2-aminoethansulfonsäure). Eine weitere Gruppe bevorzugter Puffer sind Glycin, Glycylglycin, Glycylglycylglycin, N,N-Bis(2-hydroxyethyl)glycin und N-[2-Hydroxy-1,1-bis(hydroxymethyl)ethyl]-glycin (Tricin). Bevorzugt werden auch Aminosäurepuffer wie Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N.N.N-Trimethyllysin, 3-Methylhistidin, 5-Hydroxylysin, o-Phosphoserin, [Gamma]-Carboxyglutamat, [Epsilon]-N-Acetyllysin, [Omega]-N-Methylarginin, Citrullin, Ornithin und deren Derivate.

Pharmazeutische Hilfsstoffe zur Anwendung in der je nach Anwendungsart gewünschten Applikationsform können beispielsweise sein: Natriumcitrat, Kalziumphosphat, Kalziumcarbonat zusammen mit geeigneten Tablettensprengmitteln, z.B. bei oraler Applikation.

Zu den Sprengmitteln gehören, ohne darauf beschränkt zu sein, Stärke, in kaltem Wasser lösliche Stärken wie Carboxymethylstärke, Zellulosederivate wie Methylzellulose und Natriumcarboxymethylcellulose, mikrokristalline Zellulosen und kreuzvernetzte mikrokristalline Zellulosen wie Croscarmellose-Natrium, natürliche und synthetische Gummi wie Guar, Agar, Karaya (Indischer Tragant), Johannisbrotkernmehl, Tragant, Tonerden wie Bentonit, Xanthangummi, Alginate wie Alginsäure und Natriumalginat, aufschäumende Gemische u.a. Die Quellung unterstützen beispielsweise Stärke, Cellulose-Derivate, Alginate, Polysaccharide, Dextrane, quervernetztes Polyvinylpyrrolidon. Die Menge der Sprengmittel in der Zusammensetzung kann zwischen 1 und 40 Gewichts-% variieren, bevorzugt zwischen 3 und 20 Gewichts-%, besonders bevorzugt zwischen 5 und 10 Gewichts-%.

Substanzen, die durch eine chemische Reaktion mit Wasser Gas entwickeln (Natriumhydrogencarbonat, Zitronen- und Weinsäure) oder Substanzen, welche als Hydrophylierungsmittel die Benetzung der Kristallite verbessern und damit ihre Auflösung z.B. in Wasser vermitteln (Lösungsvermittler, z. B. Polyethylenglycolsorbitanfettsäureester).

Hilfsstoffe sind auch Substanzen, die als Bindemittel eingesetzt werden können, wie z.B. Stärke (z.B. aus Weizen, Mais, Reis oder Kartoffel), Gelatine, Zuckerstoffe wie z.B. Glucose, Sucrose oder Beta-Lactose, Süßstoffe (z.B. aus Mais), natürliche und synthetische Gummi, wie z.B. Akazia, Tragant oder Ammoniumkalziumalginat, Natriumalginat, Polyethylenglykol, Polyvinylpyrrolidon, Magnesiumaluminiumsilicat, Wachse, Zellulosederivate wie z.B. Carboxymethylzellulose, Natriumcarboxymethylzellulose, Hydroxypropylcarboxymethylzellulose, oder als Verdünner, z.B. Zuckerstoffe (z.B. Mannitol).

Zu den Hilfsstoffen zählen beispielhaft ferner Schmiermittel, Gleitmittel, Geschmacks- oder Aromastoffe, Antioxidantien, Farbstoffe, Konservierungsstoffe und oberflächenaktive Substanzen, wie z.B. Natriumlaurylsulfat oder Polysorbat 80.

Schmiermittel werden bei der Tablettenherstellung gewöhnlich kurz vor der Verpressung zugegeben, da sie auf der Oberfläche der Granula sowie zwischen ihnen und den Bauteilen der Tablettenpresse vorhanden sein sollen. Die Menge des Schmiermittels kann zwischen 0,05 und 15 Gewichts-% der Zusammensetzung variieren, bevorzugt zwischen 0,2 und 5 Gewichts-%, besonders bevorzugt zwischen 0,3 und 3 Gewichts-%, höchstbevorzugt zwischen 0,3 und 1,5 Gewichts-%.

Zu den in diesen Darreichungsformen verwendeten Schmiermitteln gehören Natriumoleat, Metallstearate wie z.B. Natriumstearat, Kalziumstearat, Kaliumstearat und Magnesiumstearat, Stearinsäure, Natriumbenzoat, Natriumacetat, Natriumchlorid, Borsäure, Wachse mit einem hohen Schmelzpunkt, Polyethylenglykole u.a.

Geeignete Gleitmittel umfassen z.B. Silikondioxid, Talkum, Magnesiumstearat, Natriumstearat. Die Menge des Gleitmittels in der Zusammensetzung kann zwischen 0,01 und 10 Gewichts-% variieren, bevorzugt zwischen 0,1 und 7 Gewichts-%, besonders bevorzugt zwischen 0,2 und 5 Gewichts-%, höchst bevorzugt zwischen 0,5 und 2 Gewichts-%.

Geeignete Beispiele für Geschmacks- oder Aromastoffe sind ätherische Öle, Vitamine, sowie galenische Hilfsstoffe, ausgewählt aus Zuckern, Zuckeraustauschstoffen, Zuckerersatzstoffen, Säuerungsmitteln, Lösungsvermittlern wie z.B. Wasser, Glykol, Glycerin, Verdickern, Süßungsmitteln, Farbstoffen oder Konservierungsmitteln oder Kombinationen hiervon, auch in Abhängigkeit von der galenischen Darreichungsform.

Zu den geeigneten Aromen und Geschmacksstoffen gehören vor allem ätherische Öle, die als Aroma- bzw. als Geschmacksstoffe verwendbar sind. Dabei handelt es sich im Allgemeinen um flüchtige Extrakte aus Pflanzen oder Pflanzenteilen mit dem dafür charakteristischen Geruch, die vor allem durch Wasserdampfdestillation aus Pflanzen oder

Pflanzenteilen gewonnen werden können.

Beispielhaft können hier genannt werden: ätherische Öle bzw. Aromastoffe und Extrakte aus Salbei, Nelken, Kamille, Anis, Sternanis, Thymian, Teebaum, Pfefferminze, Minze, (Menthol, Cineol), Eukalyptus, Mango, Feigen, Lavendel, Kamillenblüten, Kiefernnadeln, Zypresse, Orangen, Rosenholz, Pflaume, Johannisbeere, Kirsche, Birkenblätter, Zimt, Limetten, Orangen, Grapefruit, Mandarine, Wacholder, Baldrian, Zitronenmelisse, Zitronengras, Palmarosa, Moosbeere, Granatapfel, Rosmarin, Ingwer, Ananas, Guave, Sonnenhut (Echinacea), Efeublättern, Heidelbeere, Kaki, Melonen u. ä. oder Mischungen hiervon wie z.B. Mischungen aus Menthol, Pfefferminz- und Sternanisöl oder Menthol und Kirscharoma.

Diese Aroma- bzw. Geschmacksstoffe können in Mengen von 0,0001 bis 10 Gewichts-% (insbesondere als Mischung), vor allem 0,001 bis 6 Gewichts-%, bevorzugt 0,001 bis 4 Gewichts-%, besonders bevorzugt 0,01 bis 1 Gewichts-%, bezogen auf die Gesamtzubereitung vorliegen. Anwendungsbedingt oder einzelfallabhängig kann es von Vorteil sein, hiervon abweichende Mengen einzusetzen.

Der Zusatz von Antioxidantien ist besonders bei topischen Applikationen vorteilhaft. Geeignete Beispiele hierfür sind Natriummetabisulfit, alpha-Tocopherol, Ascorbinsäure, Maleinsäure, Natriumascorbat, Ascorbylpalmitat, butyliertes Hydroxyanisol, butyliertes Hydroxytoluol, Fumarsäure oder Propylgallat. Bevorzugtes Antioxidantium ist Natriummetabisulfit.

Färbemittel können z.B. Lebensmittelfarbstoffe sein. Diese können auch auf einem geeigneten Adsorptionsmittel wie Tonerde oder Aluminiumoxid adsorbiert sein. Die Menge der Farbstoffe kann zwischen 0,01 und 10 Gewichts-% der Zusammensetzung variieren, bevorzugt zwischen 0,05 und 6 Gewichts-%, besonders bevorzugt zwischen 0,1 und 4 Gewichts-%, höchst bevorzugt zwischen 0,1 und 1 Gewichts-%.

Geeignete Farbstoffe sind z.B. Kurkumin, Riboflavin, Riboflavin-5'-phosphat, Tartrazin, Alkannin, Chinolingelb WS, Echtgelb AB, Riboflavin-5'-Natriumphosphat, Gelb 2G, Gelborange S, Orange GGN, Echtes Karmin, Citrus red # 2, Azorubin, Amaranth, Cochenille rot A, Ponceau SX-rot, Ponceau 6R, Erythrosin, Rot 2G, Allurarot AC, Indanthron, Patentblau V, Indigotin I, Brilliantblau FCF, Chlorophylle und Chlorophylline, kupferhaltige Komplexe der Chlorophylle und Chlorophylline, Grün S, Fast Green FCF, Einfaches Zuckerkulör, Sulfitlaugen-Zuckerkulör, Ammoniak-Zuckerkulör, Ammonsulfit-Zuckerkulör, Brilliantschwarz BN, Ruß, Pflanzenkohle, Braun FK, Braun HT, Alpha-Carotin, Beta-Carotin, Gamma-Carotin, Annatto, Bixin, Norbixin, Paprikaextrakt, Capsanthin, Capsorubin, Lycopin, Beta-apo-8'-Carotinal, Beta-apo-8'-Carotinsäureethylester, Flavoxanthin, Lutein, Cryptoxanthin, Rubixanthin, Violaxanthin, Rhodoxanthin, Canthaxanthin, Zeaxanthin, Citranaxanthin, Astaxanthin, Betanin, Anthocyane, Safran, Kalziumcarbonat, Titandioxid, Eisenoxide, Eisenhydroxide, Aluminium, Silber, Gold, Litholrubin BK, Tannin, Orcein, Eisengluconat und Eisenlactat.

Konservierungsstoffe für flüssige Darreichungsformen können bei Bedarf eingesetzt und dabei ausgewählt werden aus Kaliumsorbat, Methyl-Ethylparaben, Natriumbenzoat und ähnlichen dem Fachmann zu diesem Zweck bekannten Substanzen oder Gemischen hiervon.

Typische Beispiele für Konservierungsstoffe, die für eine topische Applikation geeignet sind, sind Benzylbenzoate, Benzoesaure, Benzylalkohol, Benzalkoniumchlorid, N-Cetyl-N,N,-Trimethylammoniumbromid (Cetrimid, Fa. Merck), Chlorhexidin, Chlorbutanol, Chlorcresol, Imidurea, die Parabene, wie Methyl-, Ethyl-, Propyl- oder Butylparaben, Natriummethylparaben, Natriumpropylparaben, Kaliumsorbat, Natriumbenzoat, Natriumpropionat, Phenol, Phenoxyethanol, Phenylethylalkohol, Phenylmercuriacetat, Phenylmercuriborat, Phenylmercurinitrate, Sorbinsäure oder Thiomersal (Natriumethylmercurithiosalicylat). Bevorzugt sind Methylparaben, Propylparaben sowie Natriummethylparaben und Natriumpropylparaben.

Bei topischen Applikationen kommen als oberflächenaktive Solubilisatoren (Lösungsvermittler) beispielsweise Diethylenglykolmonoethylether, Polyethylpropylenglykol-Copolymere, Cyclodextrine, Glycerylmonostearate wie z.B. Solutol HS 15 (Macrogol-15-hydroxystearat von BASF, PEG-660-15-hydroxystearate), Sorbitanester, Polyoxyethylensorbitansäureester, Polyvinylalkohol, Natriumlaurylsulfat, (anionische) Glycerylmonooleate, etc. zum Einsatz.

Als Emulgatoren kommen beispielsweise die folgenden anionischen und nichtionischen Emulgatoren in Frage: anionische Emulgatorwachse, Cetylalkohol, Cetylstearylalkohol, Stearinsäure, Ölsäure, Polyoxyethylen-Polyoxypropylen-Blockpolymere, Anlagerungsprodukte von 2 bis 60 Mol Ethylenoxid an Rizinusöl und/oder gehärtetes Rizinusöl, Wollwachsöl (Lanolin), Sorbitanester, Polyoxyethylenalkylester, Polyoxyethylen-Sorbitanfettsäureester oder Polyvinylalkohol. Bevorzugt sind Glycerinmonooleat, Stearinsäure und Phospholipide wie z.B. Lezithin.

Als Triglyceride kommen mittelkettige und hochmolekulare Triglyceride in Frage. Mittelkettige Triglyceride sind Glycerinester der Fettsäuren mit nur 6-12 Kohlenstoffatomen, wie z.B. Capryl-Caprinsäuretriglycerid. Hochmolekulare Triglyceride sind Glycerinfettsäureester mit langkettigen Fettsäuren. Sie sind z.B. aus verschiedenen natürlichen Fetten hergestellte Triglyceridgemische. Bevorzugt werden mittelkettige Triglyceride eingesetzt, insbesondere Capryl-Caprinsäuretriglycerid.

Als pH-regulierendes Mittel bei topischen Darreichungsformen kommen z.B. Natriumhydroxid, Salzsäure, Puffersubstanzen, wie z.B. Natriumdihydrogenphosphat oder Dinatriumhydrogenphosphat in Frage.

Creme-Zubereitungen können ferner weitere Hilfs- und Zusatzstoffe enthalten, wie z.B. Fettungsmittel, Lösungsmittel, Konsistenzgeber oder Hydrotrope, zur Verbesserung des Fließverhaltens. Dabei können von den vorgehend

angegebenen Additiven bzw. Zusatzstoffen jeweils einzelne oder auch mehrere Stoffe derselben Gruppe im Gemisch anwesend sein.

Als Fettungsmittel eignen sich z.B. Ölsäuredecylester, hydriertes Rizinusöl, leichtes Mineralöl, Mineralöl, Polyethylenglykol, Natriumlaurylsulfat.

Als Lösungsmittel kommen Maiskeimöl, Baumwollsaatöl, Erdnussöl, Sesamöl, Sojabohnenöl, Ethyloleat, Glycerin, Isopropylmyristat, Isopropylpalmitat, Polyethylenglykol oder Polypropylenglykol in Frage.

Als Konsistenzgeber eignen sich beispielsweise Cetylalkohol, Cetylesterwachs, hydriertes Rizinusöl, mikrokristalline Wachse, nicht-ionische Emulgatorwachse, Bienenwachs, Paraffin oder Stearylalkohol.

Geeignete Hydrotope sind Alkohole, wie beispielsweise Ethanol, Isopropylalkohol oder Polyole, wie z.B. Glycerin.

<u>Anwendungsbeispiele:</u>

Als mögliche Anwendungen sind beispielhaft jedoch nicht ausschließend die Folgenden genannt.

Parenterale Anwendungen eignen sich beispielsweise für akute und perakute Zustände mit Hospitalisierung, wie z.B. septische Zustände, oder im Rahmen von geplanten chirurgischen Eingriffen. Geeignete Wirkstoffdosierungen der erfindungsgemäßen Form III reichen von 1 μg bis 100 mg /kg Körpergewicht, bevorzugt von 50 μg bis 10 mg /kg Körpergewicht, besonders bevorzugt von 100 μg bis 5 mg /kg Körpergewicht. Die Applikation kann einmal oder mehrmals täglich oder über eine Dauerinfusion erfolgen, wobei die Gesamttagesdosis bevorzugt bei maximal 100 mg/kg Körpergewicht, besonders bevorzugt bei maximal 50 mg/kg Körpergewicht liegt. Im Rahmen von geplanten chirurgischen Eingriffen empfiehlt es sich mit der Dosierung mindestens 12 h, bevorzugt 24 h, besonders bevorzugt mindestens 48 h vor dem eigentlichen Eingriff zu starten. Die Behandlungsdauer richtet sich nach dem Zustand des Patienten und der Art der Erkrankung. Die Behandlung sollte mindestens bis zum Ausbleiben der Symptome, bevorzugt noch mindestens 2 Tage nach dem Ausbleiben der Symptome, besonders bevorzugt noch mindestens 5 Tage nach dem Ausbleiben der Symptome fortgesetzt werden.

Anwendungen *per os*, wie z.B. Tabletten oder Kapseln, eignen sich beispielsweise für akute, subakute, chronische oder rezidivierende Zustände, wie z.B. Rheumatoide Arthritis. Geeignete Wirkstoffdosierungen der erfindungsgemäßen Form III reichen von 1 μg bis 100 mg /kg Körpergewicht, bevorzugt von 50 μg bis 10 mg /kg Körpergewicht, besonders bevorzugt von 100 μg bis 5 mg /kg Körpergewicht. Die Einnahme kann einmal oder mehrmals täglich erfolgen, wobei die Gesamttagesdosis bevorzugt bei maximal 100 mg/kg Körpergewicht, besonders bevorzugt bei maximal 50 mg/kg Körpergewicht liegt. Der Abstand zwischen zwei Einnahmen sollte bevorzugt mindestens eine Stunde, besonders bevorzugt mindestens 2 Stunden betragen. Die Behandlungsdauer richtet sich nach dem Zustand des Patienten und der Art der Erkrankung. Die Behandlung sollte mindestens bis zum Ausbleiben der Symptome, bevorzugt noch mindestens 5 Tage nach dem Ausbleiben der Symptome, besonders bevorzugt noch mindestens 14 Tage nach dem Ausbleiben der Symptome fortgesetzt werden.

Topische Anwendungen, wie z.B. Augentropfen oder Salben, eignen sich beispielsweise in Form von Augentropfen zur Behandlung von entzündlichen Erkrankungen des Auges, und beispielsweise in Form einer Salbe oder Creme zur Behandlung von Wunden oder entzündlichen Hauterkrankungen. Geeignete Wirkstoffanteile der erfindungsgemäßen Form III an der pharmazeutischen Zusammensetzung reichen von 0,05 bis 20 %, bevorzugt von 0,1 bis 10%, besonders bevorzugt von 0,2 bis 5%. Die Anwendung kann einmal oder mehrmals täglich erfolgen, wobei der Abstand zwischen zwei Anwendungen bevorzugt mindestens eine Stunde, besonders bevorzugt mindesten 2 Stunden betragen sollte. Die Behandlungsdauer richtet sich nach dem Zustand des Patienten und der Art der Erkrankung. Die Behandlung sollte mindestens bis zum Ausbleiben der Symptome, bevorzugt noch mindestens 3 Tage nach dem Ausbleiben der Symptome, besonders bevorzugt noch mindestens 10 Tage nach dem Ausbleiben der Symptome fortgesetzt werden.

Die erfindungsgemäße kristalline Form III von 5-Amino-2,3-dihydro-1,4-phthalazindion Natriumsalz kann auch in Kombination mit anderen mindestens einem weiteren bekannten Wirkstoffen und/oder Standardtherapien verwendet werden.

Diese Wirkstoffe für Kombinationen können aus der Gruppe ausgewählt werden, die steroidale und nichtsteroidale antiinflammatorische Wirkstoffe, Immunmodulatoren, Immunsuppressiva, Antibiotika, antiinfektive Wirkstoffe, antivirale Wirkstoffe, antimykotische Wirkstoffe, Analgetika, Lokalanästhetika, Antikoagulantia, Thrombozytenaggregationshemmer, Muskelrelaxantien, Tonika und anabole Wirkstoffe umfasst. Eine solche Wirkstoffkombination kann zu prophylaktischen und/oder therapeutischen Zwecken bei einer Person verwendet werden, bei der eine solche Verabreichung sinnvoll erscheint.

Geeignete Beispiele für steroidale antiinflammatorische Wirkstoffe umfassen Corticosteroide, Glucocorticoide, Cortison, Cortison Acetat, Hydrocortison, Hydrocortison Acetat, Dexamethason, Betamethason, Prednison, Prednisolon, Methylprednisolon, Deltason, Triamcinolon, Tixocortol Pivalat, Mometason, Amcinonid, Budesonid, Desonid, Fluocinonid, Fluocinolon, Halcinonid, Fluocortolon, Hydrocortison-17-Valerat, Halometason, Alclometason Dipropionat, Betamethason Valerat, Betamethason Dipropionat, Prednicarbat, Clobetason-17-Butyrate, Clobetasol-17-Propionat, Fluocortolon Caproat, Fluocortolon Pivalat, Flupredniden Acetat, Hydrocortison-17-Butyrat, Hydrocortison-17-Aceponat, Hydrocortison-17-Buteprat, Ciclesonid, Flunisolid, Fluticason Furoat, Fluticason Propionat, Triamcinolon Acetonid, Beclomethason Dipropionat.

Geeignete Beispiele für nicht-steroidale antiinflammatorische Wirkstoffe (NSAIDs) umfassen Acetylsalizylsäure, Salizylsäure und Salicylate, Paracetamol (Acetaminophen), Salsalat, Diflunisal, Ibuprofen, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Aceclofenac, Nabumeton, Piroxicam, Meloxicam, Tenoxicam, Droxicam, Lornoxicam, Isoxicam, Phenylbutazon, Mefenaminsäure, Meclofenaminsäure, Flufenaminsäure, Tolfenaminsäure, Celexoxib, Rofecoxib, Valdecoxib, Parecoxib, Lumiracoxib, Etoricoxib, Firocoxib, Nimesulid, Clonixin, Licofelon, H-Harpagid, Flunixin, Tiaprofensäure.

Geeignete Beispiele für Immunmodulatoren (IMIDs) umfassen Thalidomid, Lenalidomid, Pomalidomid und Apremilast.

Geeignete Beispiele für antivirale Wirkstoffe umfassen Ancriviroc, Aplaviroc, Cenicriviroc, Enfuvirtid, Maraviroc, Vicriviroc, Amantadin, Rimantadin, Pleconaril, Idoxuridin, Aciclovir, Brivudin, Famciclovir, Penciclovir, Sorivudin, Valaciclovir, Cidofovir, Ganciclovir, Valganciclovir, Sofosbusvir, Foscarnet, Ribavirin, Taribavirin, Filibuvir, Nesbuvir, Tegobuvir, Fosdevirin, Favipiravir, Merimepodib, Asunaprevir, Balapiravir, Boceprevir, Ciluprevir, Danoprevir, Daclatasvir, Narlaprevir, Telaprevir, Simeprevir, Vaniprevir, Rupintrivir, Fomivirsen, Amenamevir, Alisporivir, Bevirimat, Letermovir, Laninamivir, Oseltamivir, Peramivir, Zanamivir.

Geeignete Beispiele für immunstimulierende Wirkstoffe umfassen die Interferone ($\alpha$-, $\beta$-, $\gamma$-, $\tau$-Interferon), Interleukine, CSF, PDGF, EGF, IGF, THF, Levamisol, Dimepranol, Inosin.

Geeignete Beispiele für immunsuppressive Wirkstoffe umfassen die Gruppe der Glucocorticoide wie zuvor beschrieben; Zytostatika wie alkylierende Wirkstoffe (wie Cyclophosphamid), Antimetaboliten wie Methotrexat, Azathioprin, Mercaptopurin, Fluorouracil, Leflunomid, Proteinsynthesehemmer und bestimmte Antibiotika wie Dactinomycin, Anthracycline, Mitomycin C, Bleomycin und Mithramycin, interkalierende Wirkstoffe wie Mitoxantron; Antikörper wie Muromonab-CD3, Rituximab, Ustekinumab, Alemtuzumab, Natalizumab, Basiliximab und Daclizumab; auf Immunophiline wirkende Agentien wie Ciclosporin, Tacrolimus und Sirolimus; und nicht-klassifizierte immunsuppressive Wirkstoffe wie $\beta$--Interferon, $\gamma$--Interferon, Opioide, TNF-bindende Proteine wie Infliximab, Etanercept, Adalimumab; oder Curcumin, Catechine, Mycophenolsäure, Fingolimod, Myriocin und Fumarsäuredimethylester.

Geeignete Beispiele für Antibiotika umfassen Imipenem, Meropenem, Ertapenem, Cephalosporine, Aztreonam, Penicilline wie Penicillin G und Penicillin V, Piperacillin, Mezlocillin, Ampicillin, Amoxicillin, Flucloxacillin, Methicillin, Oxacillin, Clavulansäure, Sulbactam, Tazobactam, Sultamicillin, Fosfomycin, Teicoplanin, Vancomycin, Bacitracin, Colistin, Gramicidin, Polymyxin B, Tyrothricin, Teixobactin, Fosmidomycin, Amikacin, Gentamicin, Kanamycin, Neomycin, Netilmicin, Streptomycin, Tobramycin, Chloramphenicol, Fusidinsäure, Cethromycin, Narbomycin, Telithromycin, Clindamycin, Lincomycin, Daptomycin, Dalfopristin, Quinupristin, Azithromycin, Clarithromycin, Erythromycin, Roxithromycin, Linezolid, Doxycyclin, Minocyclin, Tetracyclin, Oxytetracyclin, Tigecyclin, Norfloxacin, Enoxacin, Ciprofloxacin, Ofloxacin, Levofloxacin, Moxifloxacin, Metronidazol, Tinidazol, Aminocumarin, Sulfadiazin, Sulfadoxin, Sulfamethoxazol, Sulfasalazin, Pyrimethamin, Trimethoprim, Rifampicin.

Antiinfektive Wirkstoffe ist ein Überbegriff für Verbindungen, die zur Behandlung bakterieller, viraler, fungaler, protozoaler und Wurminfektionen verwendet werden und umfasst Antibiotika, antivirale Wirkstoffe, Antimykotika, Antiprotozoenmittel und Anthelminthika.

Geeignete Beispiele für Muskelrelaxantien umfassen Tercuronium, 1-Ethylcarbamoyl-3-(3-trifluoromethylphenyl)pyrrolidin, Metaxalon, Methocarbamol, Meprobamat, Baclofen, Carisoprodol, Chlorzoxanzon, Cyclobenzaprin, Dantrolen, Diazepam, Orphenadrin, Chinin, Rocuronium, Succinylcholin, Decamethonium, Pancuronium, Veruronium, Rapacuronium, Dacuronium, Duador, Malouetin, Dipyrandium, Pipercuronium, Chandonium, HS-342, Atracurium, Mivacurium, Doxacurium, d-Tubocurarin, Dimethyltubocurarin, Gallamin, Alcuronium, Anatruxonium, Diadonium, Fazadinium, Tropeinium, Cisatrucurium.

Geeignete Beispiele für Antimykotika umfassen Abafungin, Amphotericin B, Candicidin, Filipin, Hamycin, Natamycin, Nystatin, Rimocidin, Bifonazol, Butoconazol, Clotrimazol, Econazol, Fenticonazol, Isoconazol, Ketoconazol, Luliconazol, Miconazol, Omoconazol, Oxiconazol, Sertaconazol, Sulconazol, Tioconazol, Albaconazol, Efinaconazol, Epoxiconazol, Fluconazol, Isavuconazol, Itraconazol, Posaconazol, Propiconazol, Ravuconazol, Terconazol, Voriconazol, Amorolfin, Butenafin, Nafitifin, Terbinafin, Anidulafungin, Caspofungin, Micafungin, Benzoesäure, Ciclopirox, Flucytosin, Griseofulvin, Haloprogin, Tolnaftat, Undecylensäure, Kristallviolett, Peru-Balsam.

Geeignete Beispiele für Antiprotozoenmittel umfassen Metronidazol, Tinidazol, Ornidazol, Atovaquon, Clioquinol, Chlorquinaldol, Emetin, Pentamidinisethionat, Eflornithin, Nitrofural, Halofuginon, Miltefosin, Chloroquin, Hydroxychloroquin, Mepacrin, Primaquin, Amodiaquin, Pamaquin, Piperaquin, Proguanil, Cyclohunailembonat, Chinin, Mefloquin, Pyrimethamin, Artmether, Artemisinin, Artesunat, Dihydroartemisinin, Halofantrin, Lumefantrin, Sulfadoxin.

Geeignete Beispiele für Anthelminthika umfassen Mebendazol, Praziquantel, Albendazol, Diethylcarbamazin, Flubendazol, Ivermectin, Levamisol, Metrifonat, Niclosamid, Oxyclozanid, Oxamniquin, Oxantel, Piperazin, Pyrantel,

Pyrantelpamoat, Monopantel, Derquantel, Pelletierinsulfat, Pyrvinium, Thiabendazol, Fenbendazol, Triclabendazol, Abamectin, Suramin, Emodepsid, Pyrviniumembonat, Aminoacetonitril.

Geeignete Beispiele für Lokalanästhetika umfassen Lidocain, Lignocain, Menthol, Articain, Bupivacain, Ropivacain, Benzocain, Chloroprocain, Kokain, Cyclomethycain, Dimetocian, Larocain, Piperocain, Propoxycain, Procain, Novocain, Proparacain, Tetracain, Amethocain, Cinchocain, Dibucain, Etidocain, Levobupivacain, Meplavacain, Prilocain, Trimecain, Saxitoxin, Neosaxitoxin, Tetrodotoxin, Eugenol.

Geeignete Beispiele für Analgetika umfassen die zuvor gelisteten NSAIDs; Opioidanalgetika wie Morphin, Fentanyl, Methadon, Oxycodon, Carfetanyl, Dihydroetorphin, Ohmefentanyl, Etorphin, Sufentanil, Remifentanil, Alfentanil, Buprenorphin, Hydromorphon, Levomethadon, Hydrocodon, Pintramid, Nalbuphin, Tapentadol, Pentazocin, Dihydrokodein, Kodein, Pethidin, Tramadol, Tilidin, Meptazinol, Naloxon, Naltrexon, Diprenorphin, Loperamid, Apomorphin; Epibatidin; Skopolamin; Ziconitid; Cannabinoide wie Tetrahydrocannabinol, Cannabidiol, Marinol; Flupirtin; Ketamin und die zuvor gelisteten Lokalanästhetika.

Geeignete Beispiele für Antikoagulantia umfassen die Heparine, Cumarine wie Phenprocoumon (Marcumar) und Warfarin, Apixaban, Rivaroxaban, Edoxaban, Dabigatran, Ximelagatran, Hirudin, Lepirudin, Bivalirudin, Citrat, EDTA, Fondaparinux, Argatroban, Otamixaban.

Geeignete Beispiele für Thrombozytenaggregationshemmer umfassen Abciximab, Acetylsalicylsäure, Dipyridamol, Clopidogrel, Eptifibatid, Ilomedin, Prostacyclin, Prasugrel, Ticagrelor, Ticlopidin, Tirofiban.

Tonika ist ein Überbergriff für Wirkstoffe, die den Körper stärken, seinen Tonus erhöhen oder seine physiologischen Funktionen wiederherstellen. Sie können pflanzlichen oder tierischen Ursprungs sein.

Anabole Wirkstoffe können den anabolen Stoffwechsel und eine Stärkung des zellulären Kollagengerüsts befördern. Ein weitreichender Missbrauch dieser Substanzen als Dopingmittel im Sport und beim Bodybuilding ist jedoch bekannt. Daher wird die Kombination mit 5-Amino-2,3-dihydro-1,4-phthalazindion Natriumsalz Form III nur insoweit empfohlen, als dies durch die jeweiligen nationalen Gesetzgebungen gedeckt ist.

Die Standardtherapien für die zuvor genannten Wirkstoffe kann der Fachmann leicht dem Stand der Technik entnehmen. Es wird bevorzugt, dass sich die jeweiligen Applikationsformen und Dosierungen der zuvor genannten Wirkstoffkombinationen an bereits etablierten Standardtherapien für den Kombinationswirkstoff neben der erfindungsgemäßen kristallinen Form III von 5-Amino-2,3-dihydro-1,4-phthalazindion Natriumsalz orientieren.

Abkürzungsverzeichnis:

| | |
|---|---|
| % v/v | Volumenprozent |
| Abb. | Abbildung |
| ca. | circa |
| $cm^{-1}$ | Wellenzahl |
| $cm^3$ | Kubikzentimeter |
| DMSO | Dimethylsulfoxid |
| FT-IR | Fourier-Transformation Infrared |
| g | Gramm |
| Gew.% | Gewichtsprozent (Massenanteil) |
| GMP | Gute Herstellungspraxis (Good Manufacturing Practice) |
| GPP | Gute Pharmazeutische Praxis (Good Pharmacy Practice) |
| h | Stunde |
| $H_2O$ | Wasser |
| IL | Interleukin |
| kg | Kilogramm |
| l | Liter |
| LPS | Lipopolysaccharid |
| $m^3$ | Kubikmeter |
| mbar | Millibar |
| mg | Milligramm |

(fortgesetzt)

| | |
|---|---|
| % v/v | Volumenprozent |
| mL | Milliliter |
| mmol | Millimol |
| mol | Mol |
| nm | Nanometer |
| pg | Pikogramm |
| PMA | Phorbol-12-myristat-13-acetat |
| REM | Rasterelektronenmikroskop |
| RT | Raumtemperatur |
| s | Standardabweichung |
| TNF | Tumornekrosefaktor |
| u.a. | unter anderem |
| VE | vollentsalzt |
| vgl. | vergleiche |
| XRPD | Röntgenpulverdiffraktogramm (X-ray powder diffractogram) |
| z.B. | Zum Beispiel |

KURZE BESCHREIBUNG DER ABBILDUNGEN:

Abb. 1 beschreibt ein Pulverdiffraktogramm der erfindungsgemäßen kristallinen Form III, wobei **Abb. 1a** die Auftragung der Rietveld-Verfeinerung der Kristallstruktur von Form III über den vollen aufgenommenen Bereich von 3 - 80,085 °2θ zeigt während in **Abb. 1b** ein Ausschnitt der Auftragung im Bereich von 3 - 35 °2θ dargestellt ist.

Abb. 2 zeigt eine vergleichende Darstellung der Packungsmotive in der Kristallstruktur der Formen I und II sowie der erfindungsgemäßen Form III in jeweiliger Richtung der Elementarzellachsen, dargestellt als Kugel-Stab-Modell und als Kalottenmodell.

**Abb. 2a** zeigt die a-Achse im Kugel-Stab-Modell,
**Abb. 2b** zeigt die a-Achse im Kalottenmodell,
**Abb. 2c** zeigt die b-Achse im Kugel-Stab-Modell,
**Abb. 2d** zeigt die b-Achse im Kalottenmodell,
**Abb. 2e** zeigt die c-Achse im Kugel-Stab-Modell,
**Abb. 2f** zeigt die a-Achse im Kalottenmodell.

Abb. 3 zeigt ein Festkörper-FT-IR-Spektrum der erfindungsgemäßen Form III, wobei **Abb. 1a** die Auftragung des vollen aufgenommenen Bereichs von 4000 - 400 $cm^{-1}$ zeigt während in **Abb. 1b** ein Ausschnitt der Auftragung im Bereich von 1800 - 400 $cm^{-1}$ dargestellt ist.

Abb. 4 zeigt ein Raman-Spektrum der Form III im Bereich von 90 - 1800 $cm^{-1}$.

Abb. 5 zeigt REM Aufnahmen der Formen I und II, sowie der erfindungsgemäßen Form III.

**Abb. 5a** zeigt eine vergleichende Darstellung aller drei Formen in 2500-facher Vergrößerung.
**Abb. 5b** zeigt eine vergleichende Darstellung der sehr ähnlichen Formen II und III in 1.000-, 10.000, 25.000- und 50.000-facher Vergrößerungen.

**Abb. 6**: Pulverdiffraktogramme von Suspensionen einer 50:50 Gew% Mischung der Formen II und III in 2-Propanol,

Ethanol und Methanol nach 24 h (**Abb. 6a**) bzw. einer 50:50 Gew% Mischung der Formen I und III in 2-Propanol, Ethanol und Methanol nach 3 Tagen (**Abb. 6b**) Äquilibrierungsphase. Zum Vergleich ist jeweils ein Diffraktogramm des Ausgangsgemisches dargestellt.

**In Abb. 6a** sind die in ihren Intensitäten zunehmenden, für Form III singulären Reflexe durch Pfeile markiert. (|) zeigen die Reflexpositionen der jeweiligen Form.
In **Abb. 6b** sind die in ihrer Intensität abnehmenden, für Form I singulären Reflexe durch Pfeile markiert. (|) zeigen die Reflexpositionen der jeweiligen Form.

Abb. 7 zeigt die Sekretion der Zytokine TNF-alpha und IL-6 von differenzierten HL-60 Zellen in pg/mL nach jeweiliger Vorstimulation für 1 h mit 5-Amino-2,3-dihydrophthalazin-1,4-dion Natriumsalz Dihydrat, den Anhydratformen I, II und III oder der Negativkontrolle sowie anschließender Stimulation mit 100 ng/mL Lipopolysaccharid (LPS) für 24 h (Mittelwert basiert auf jeweils 3 Messungen).

**Abb. 7a** zeigt hierbei die TNF-alpha-Sekretion von differenzierten HL-60 Zellen in pg/mL und
**Abb. 7b** zeigt die IL-6-Sekretion von differenzierten HL-60 Zellen in pg/mL.

Abb. 8 zeigt die Löslichkeit von 5-Amino-2,3-dihydrophthalazin-1,4-dion Natriumsalz in mol/l in einem DMSO-$H_2O$-Gemisch mit zunehmendem $H_2O$-Anteil. Mit zunehmendem $H_2O$-Anteil steigt die Löslichkeit kontinuierlich an.

## Patentansprüche

1. Kristalline Form zu 5-Amino-2,3-dihydrophthalazin-1,4-dion Natriumsalz, **gekennzeichnet durch** mittels Röntgenpulverdiagrammen ermittelte Kristallographie-Werte:

   d-Werte: 13,131; 7,987; 7,186; 6,566; 6,512; 5,372; 3,994; 3,662; 3,406; 3,288; 3,283; 3,222; 3,215; 3,127; 2,889 und
   2-Theta-Werte: 6,73; 11,07; 12,31; 13,48; 13,59; 16,49; 22,24; 24,29; 26,14; 27,10; 27,14; 27,67; 27,72; 28,52; 30,93.

2. Kristalline Form nach Anspruch 1, **gekennzeichnet durch** einen Kristallwassergehalt ≤ 0,4 %.

3. Verfahren zur Herstellung der kristallinen Form nach Anspruch 1 oder 2, **gekennzeichnet durch** Lösen eines 5-Amino-2,3-dihydrophthalazin-1,4-dion Natriumsalz Anhydrates in DMSO, anschließendem Rühren dieser Lösung bis zur Entstehung einer Suspension und anschließendes Trocknen dieser Suspension bis zum völligen Abdampfen von DMSO.

4. Verfahren gemäß Anspruch 3 zur Herstellung der kristallinen Form nach Anspruch 1 oder 2, **gekennzeichnet durch** Lösen des 5-Amino-2,3-dihydrophthalazin-1,4-dion Natriumsalz Anhydrates in DMSO bei mindestens 90°C, anschließendem Rühren dieser Lösung bei gleichbleibender Temperatur bis zur Entstehung einer Suspension und anschließendes Trocknen dieser Suspension bei gleichbleibender Temperatur bis zum völligen Abdampfen von DMSO.

5. Verfahren gemäß Anspruch 3 oder 4 zur Herstellung der kristallinen Form nach Anspruch 1 oder 2, **dadurch gekennzeichnet, dass** das 5-Amino-2,3-dihydrophthalazin-1,4-dion Natriumsalz Anhydrat in einem Verhältnis von 1 zu 300 bis 1 zu 30.000 zu DMSO zugegeben wird.

6. Verfahren gemäß Anspruch 3 oder 5 zur Herstellung der kristallinen Form nach Anspruch 1 oder 2, **dadurch gekennzeichnet, dass** DMSO auf eine Temperatur von mindestens 90°C aufgeheizt wird, bevor ein 5-Amino-2,3-dihydrophthalazin-1,4-dion Natriumsalz Anhydrat zugegeben wird.

7. Verfahren gemäß einem der Ansprüche 3 - 6 zur Herstellung der kristallinen Form nach Anspruch 1 oder 2, **dadurch gekennzeichnet, dass** nach dem initialen Auflöseschritt für das weitere Rühren und den Trocknungsschritt ein Vakuum angelegt wird.

8. Verfahren gemäß einem der Ansprüche 3 - 7 zur Herstellung der kristallinen Form nach Anspruch 1 oder 2, **dadurch gekennzeichnet, dass** für das weitere Rühren und den Trocknungsschritt ein Temperaturbereich von 70°C bis

189°C angewandt wird.

9. Verfahren gemäß einem der Ansprüche 3 - 8 zur Herstellung der kristallinen Form nach Anspruch 1 oder 2, **dadurch gekennzeichnet, dass** für den initialen Lösungsschritt ein DMSO-$H_2O$-Gemisch verwendet wird.

10. Pharmazeutische Zubereitung, **gekennzeichnet dadurch, dass** sie die kristalline Form nach Anspruch 1 oder 2 enthält.

11. Pharmazeutische Zubereitung gemäß Anspruch 10, **dadurch gekennzeichnet, dass** die kristalline Form in Kombination mit anderen Wirkstoffen und/oder pharmazeutisch geeigneten Hilfsstoffen vorliegt.

12. Kristalline Form zu 5-Amino-2,3-dihydrophthalazin-1,4-dion Natriumsalz nach Anspruch 1 oder 2 zur prophylaktischen oder therapeutischen Verwendung in der Medizin.

13. Kristalline Form zu 5-Amino-2,3-dihydrophthalazin-1,4-dion Natriumsalz nach Anspruch 1 oder 2 zur prophylaktischen oder therapeutischen Verwendung gemäß Anspruch 12 als Immunmodulator.

14. Kristalline Form zu 5-Amino-2,3-dihydrophthalazin-1,4-dion Natriumsalz nach Anspruch 1 oder 2 zur prophylaktischen oder therapeutischen Verwendung gemäß Anspruch 12 oder 13 bei einer überschießenden Immunreaktion oder bei Zuständen mit immundefizientem Hintergrund.

15. Kristalline Form zu 5-Amino-2,3-dihydrophthalazin-1,4-dion Natriumsalz nach Anspruch 1 oder 2 oder hergestellt nach dem in Anspruch 9 definierten Verfahren zur Verwendung bei der Behandlung mit einer topischen Darreichungsform.

## Claims

1. Crystalline form of 5-amino-2,3-dihydrophthalazine-1,4-dione sodium salt, **characterized by** the crystallography values determined by means of x-ray powder diagrams :

   D values: 13.131; 7.987; 7.186; 6.566; 6.512; 5.372; 3.994; 3.662; 3.406; 3.288; 3.283; 3.222; 3.215; 3.127; 2.889 and
   2-theta values: 6.73; 11.07; 12.31; 13.48; 13.59; 16.49; 22.24; 24.29; 26.14; 27.10; 27.14; 27.67; 27.72; 28.52; 30.93.

2. Crystalline form according to claim 1, **characterized by** a content of water of crystallization ≤ 0.4%.

3. Method for producing the crystalline form according to claim 1 or 2, **characterized by** solving of a 5-amino-2,3-dihydrophthalazine-1,4-dione sodium salt anhydrate in DMSO, subsequent stirring of this solution until a suspension is generated and subsequent drying of this suspension until DMSO is completely evaporated.

4. Method according to claim 3 for producing the crystalline form according to claim 1 or 2, **characterized by** solving of the 5-amino-2,3-dihydrophthalazine-1,4-dione sodium salt anhydrate in DMSO at at least 90°C, subsequent stirring of this solution under maintenance of temperature, until a suspension is generated, and subsequent drying of this suspension under maintenance of temperature until DMSO is completely evaporated.

5. Method according to claim 3 or 4 for producing the crystalline form according to claim 1 or 2, **characterized by** the addition of the 5-amino-2,3-dihydrophthalazine-1,4-dione sodium salt anhydrate to DMSO in a ratio of 1 : 300 to 1 : 30,000.

6. Method according to claims 3 or 5 for producing the crystalline form according to claim 1 or 2, **characterized by** DMSO being heated to a temperature of at least 90°C before a 5-amino-2,3-dihydrophthalazine-1,4-dione sodium salt anhydrate is added.

7. Method according to any of claims 3 to 6 for producing the crystalline form according to claim 1 or 2, **characterized by** applying a vacuum after the initial dissolution step for further stirring and the drying step.

8. Method according to any of claims 3 to 7 for producing the crystalline form according to claim 1 or 2, **characterized by** using a temperature range from 70°C to 189°C for further stirring and the drying step.

9. Method according to any of claims 3 to 8 for producing the crystalline form according to claim 1 or 2, **characterized by** the use of a DMSO-$H_2O$ mixture for the initial dissolution step.

10. Pharmaceutical preparation **characterized by** containing the crystalline form according to claim 1 or 2.

11. Pharmaceutical preparation according to claim 10, **characterized by** providing the crystalline form in combination with other active ingredients and/or pharmaceutically acceptable adjuvants.

12. Crystalline form of 5-amino-2,3-dihydrophthalazine-1,4-dione sodium salt according to claim 1 or 2 for prophylactic or therapeutic use in medicine.

13. Crystalline form of 5-amino-2,3-dihydrophthalazine-1,4-dione sodium salt according to claim 1 or 2 for prophylactic or therapeutic use according to claim 12 as immunomodulator.

14. Crystalline form of 5-amino-2,3-dihydrophthalazine-1,4-dione sodium salt according to claim 1 or 2 for prophylactic or therapeutic use according to claim 12 or 13 in an excessive immune response or in conditions with immunodeficient background.

15. Crystalline form of 5-amino-2,3-dihydrophthalazine-1,4-dione sodium salt according to claim 1 or 2 or produced according to the method as defined in claim 9 for use in treatment with a topical dosage form.

## Revendications

1. Forme crystalline du sel de sodium de 5-amino-2,3-dihydrophthalazine-1,4-dione, **caractérisée** en des valeurs de cristallographie déterminées au moyen de diagrammes de diffraction des rayons X sur poudre:

   valeurs d: 13,131; 7,987; 7,186; 6,566; 6,512; 5,372; 3,994; 3,662; 3,406; 3,288; 3,283; 3,222; 3,215; 3,127; 2,889
   et
   valeurs 2-théta: 6,73; 11,07; 12,31; 13,48; 13,59; 16,49; 22,24; 24,29; 26,14; 27,10; 27,14; 27,67; 27,72; 28,52; 30,93.

2. Forme crystalline selon la revendication 1, **caractérisée** en un contenu en eau de cristallisation ≤ 0,4%.

3. Procédé de production de la forme cristalline selon la revendication 1 ou 2, **caractérisé en ce qu'**un anhydrate du sel de sodium de 5-amino-2,3-dihydrophthalazine-1,4-dione est dissous dans le DMSO, cette solution étant ensuite mélangée jusqu'à une suspension soit formée et cette suspension étant ensuite séchée jusqu'à l'évaporation totale du DMSO.

4. Procédé selon la revendication 3 pour la production de la forme cristalline selon la revendication 1 ou 2, **caractérisé en ce que** l'anhydrate du sel de sodium de 5-amino-2,3-dihydrophthalazine-1,4-dione est dissous dans le DMSO à une température d'au moins 90°C, cette solution étant ensuite mélangée à température constante jusqu'à une suspension soit formée et cette suspension étant ensuite séchée à température constante jusqu'à l'évaporation totale du DMSO.

5. Procédé selon la revendication 3 ou 4 pour la production de la forme cristalline selon la revendication 1 ou 2, **caractérisé en ce que** l'anhydrate du sel de sodium de 5-amino-2,3-dihydrophthalazine-1,4-dione est ajouté au DMSO dans un rapport de 1:300 à 1:30.000.

6. Procédé selon la revendication 3 ou 5 pour la production de la forme cristalline selon la revendication 1 ou 2, **caractérisé en ce que** le DMSO est chauffé à une température d'au moins 90°C avant qu'un anhydrate du sel de sodium de 5-amino-2,3-dihydrophthalazine-1,4-dione soit ajouté.

7. Procédé selon une des revendication 3 à 6 pour la production de la forme cristalline selon la revendication 1 ou 2,

**caractérisé en ce qu'**un vide est appliqué pour l'agitation suivante après l'étape initiale de dissolution.

8. Procédé selon une des revendication 3 à 7 pour la production de la forme cristalline selon la revendication 1 ou 2, **caractérisé en ce qu'**une plage de température de 70°C à 189°C est appliquée pour l'agitation suivante et l'étape de séchage.

9. Procédé selon une des revendication 3 à 8 pour la production de la forme cristalline selon la revendication 1 ou 2, **caractérisé en ce qu'**un mélange DMSO/$H_2O$ est utilisé pour l'étape initiale de dissolution.

10. Préparation pharmaceutique, **caractérisée en ce qu'**elle contient la forme cristalline selon la revendication 1 ou 2.

11. Préparation pharmaceutique selon la revendication 10, **caractérisée en ce que** la forme cristalline est présente en combinaison avec d'autres principes actifs et/ou substances auxiliaires pharmaceutiquement acceptables.

12. Forme crystalline du sel de sodium de 5-amino-2,3-dihydrophthalazine-1,4-dione selon la revendication 1 ou 2 pour l'utilisation prophylactique ou thérapeutique en médicine.

13. Forme crystalline du sel de sodium de 5-amino-2,3-dihydrophthalazine-1,4-dione selon la revendication 1 ou 2 pour l'utilisation prophylactique ou thérapeutique selon la revendication 12 comme immunomodulateur.

14. Forme crystalline du sel de sodium de 5-amino-2,3-dihydrophthalazine-1,4-dione selon la revendication 1 ou 2 pour l'utilisation prophylactique ou thérapeutique selon la revendication 12 ou 13 dans une réponse immunitaire excessive ou dans des conditions sur fond d'immunodéficience.

15. Forme crystalline du sel de sodium de 5-amino-2,3-dihydrophthalazine-1,4-dione selon la revendication 1 ou 2 ou produite par le procédé défini dans la revendication 9 pour l'utilisation dans le traitement avec une forme galénique topique.

Abb. 1:

**Abb. 1a:**

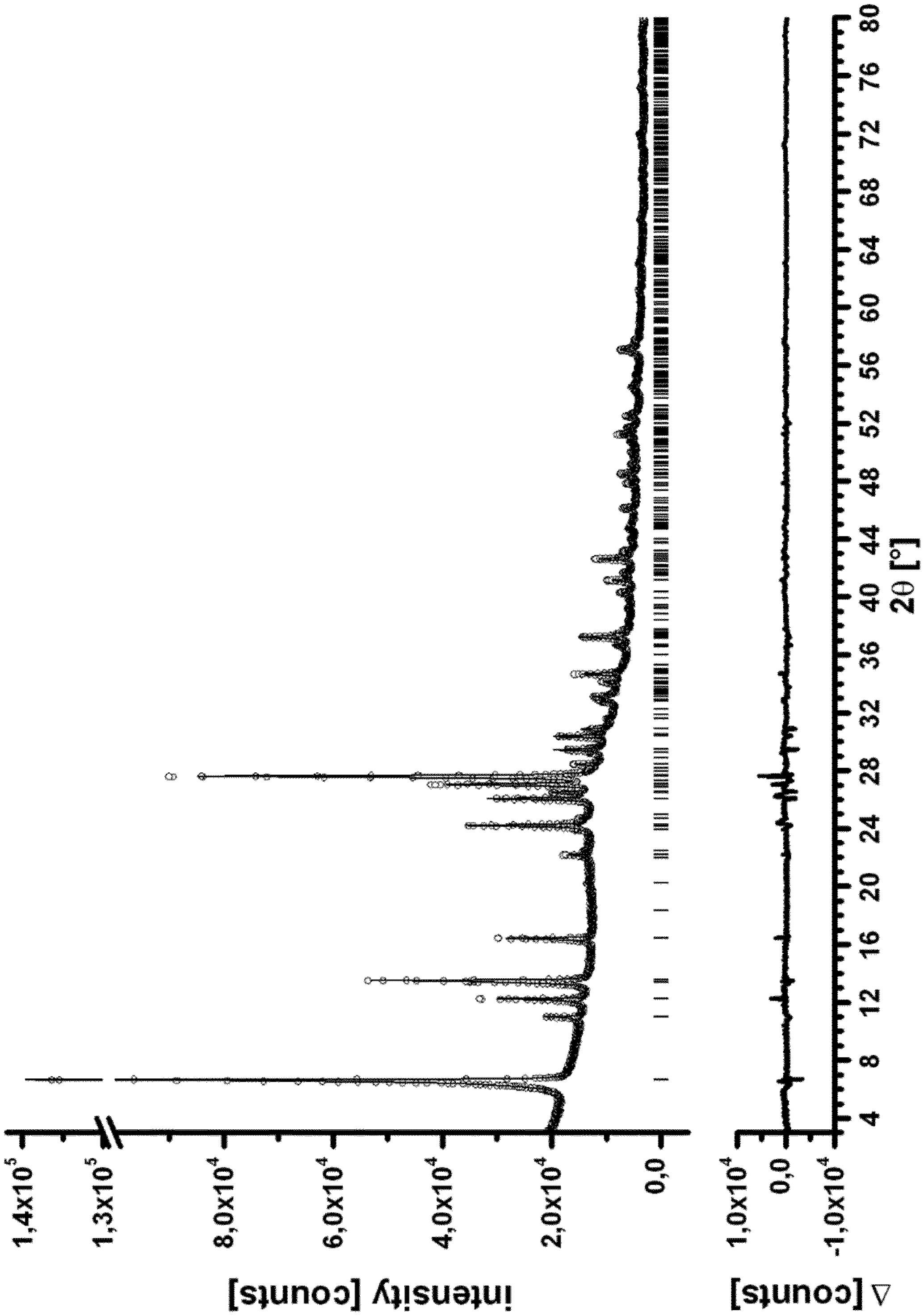

intensity [counts]
1,4x10^5
1,3x10^5
8,0x10^4
6,0x10^4
4,0x10^4
2,0x10^4
0,0
Δ [counts]
1,0x10^4
0,0
-1,0x10^4
4
8
12
16
20
24
28
32
36
40
44
48
52
56
60
64
68
72
76
80
2θ [°]

**Abb. 1b:**

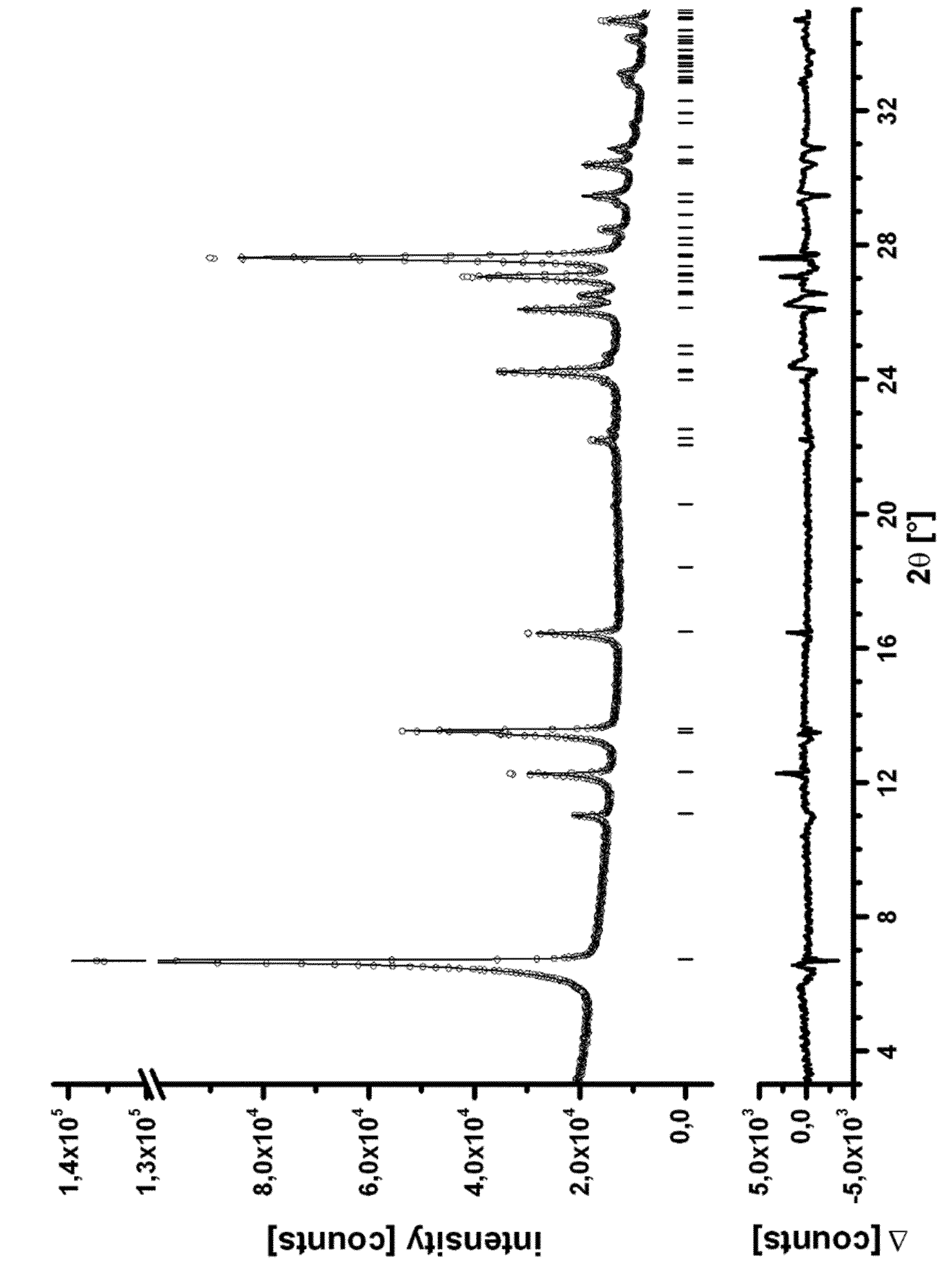
intensity [counts]
1,4x10$^5$
1,3x10$^5$
8,0x10$^4$
6,0x10$^4$
4,0x10$^4$
2,0x10$^4$
0,0
Δ [counts]
5,0x10$^3$
0,0
-5,0x10$^3$
4
8
12
16
20
24
28
32
2θ [°]

Abb. 2:

**Abb. 2a:**

Form I

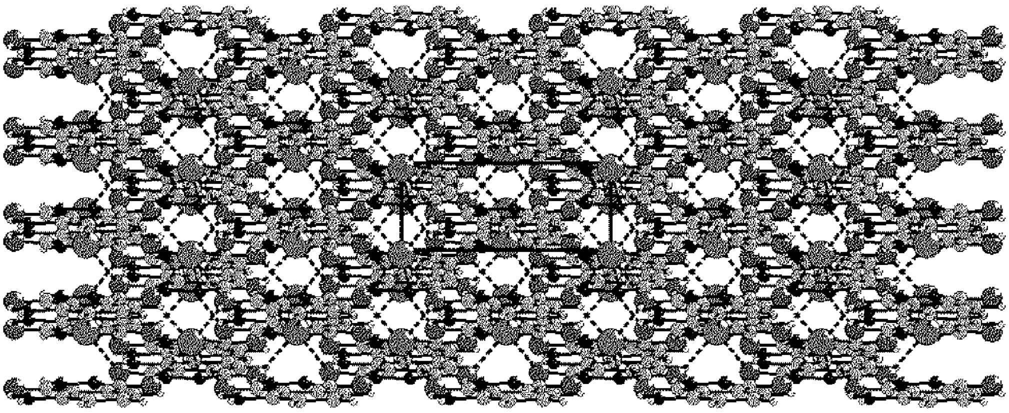

Form II

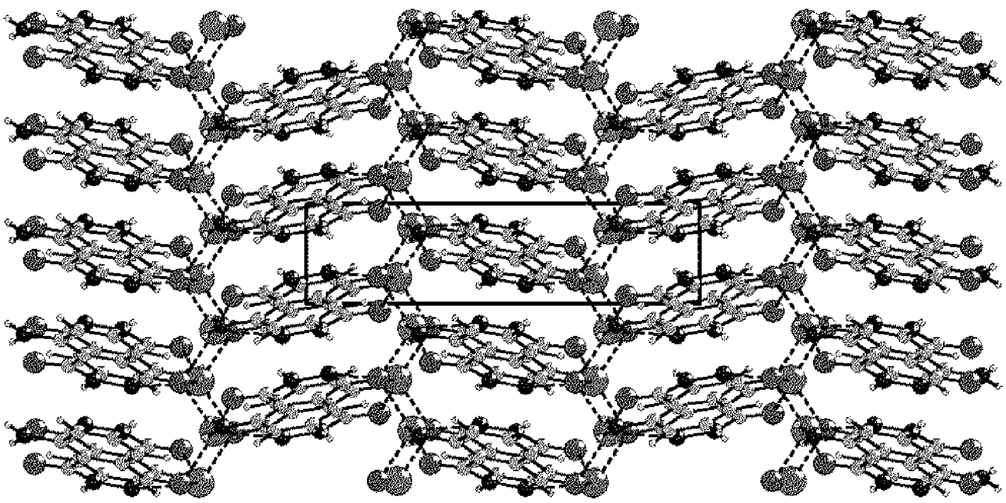

Form III

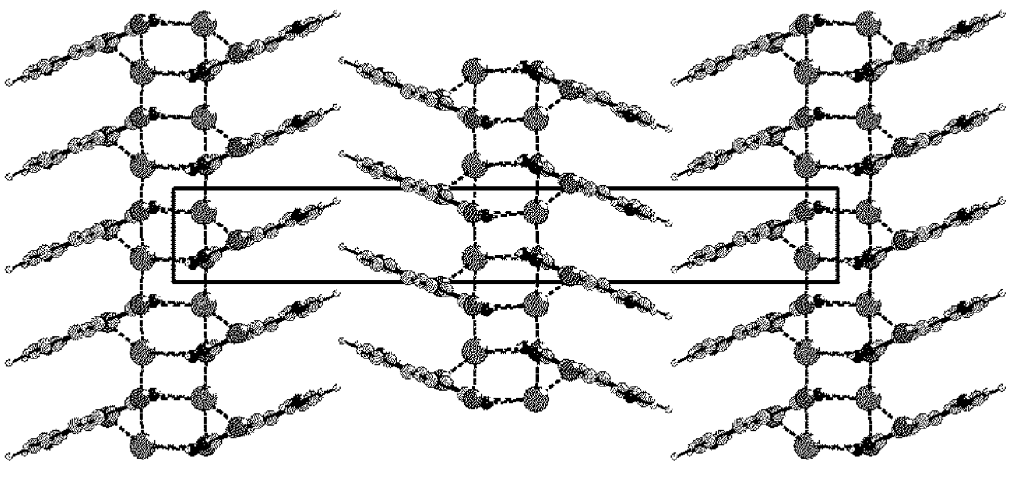

**Abb. 2b:**

Form I

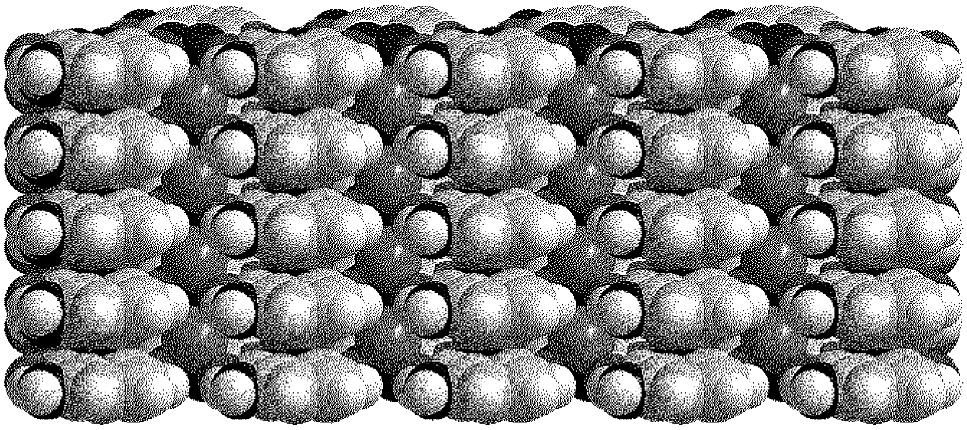

Form II

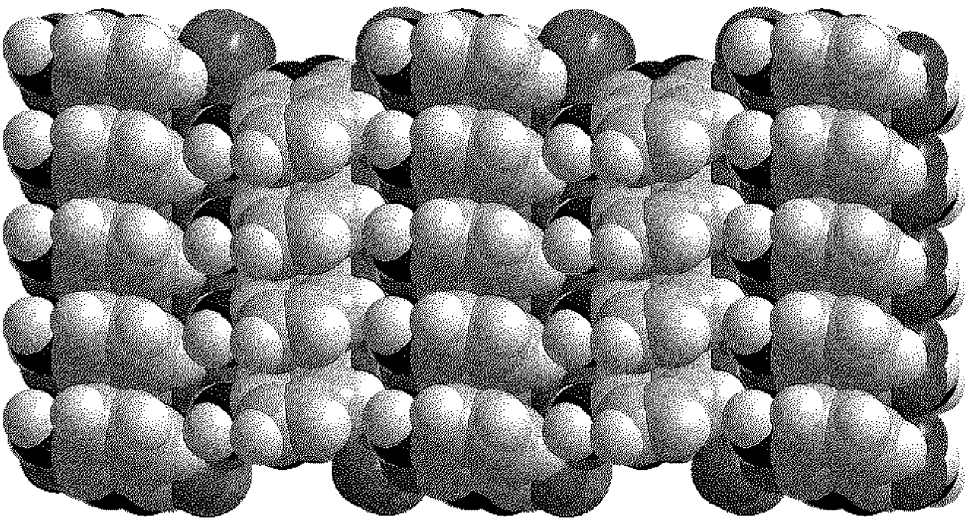

Form III

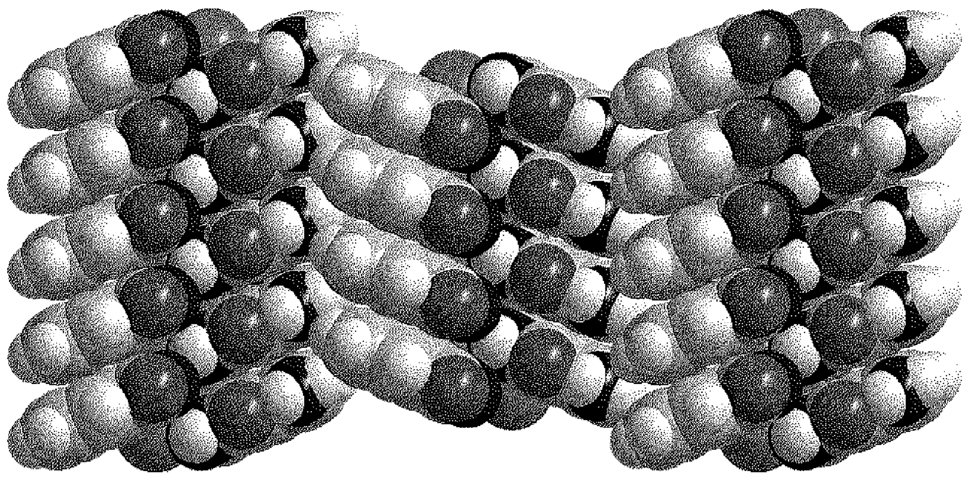

**Abb. 2c:**

Form I

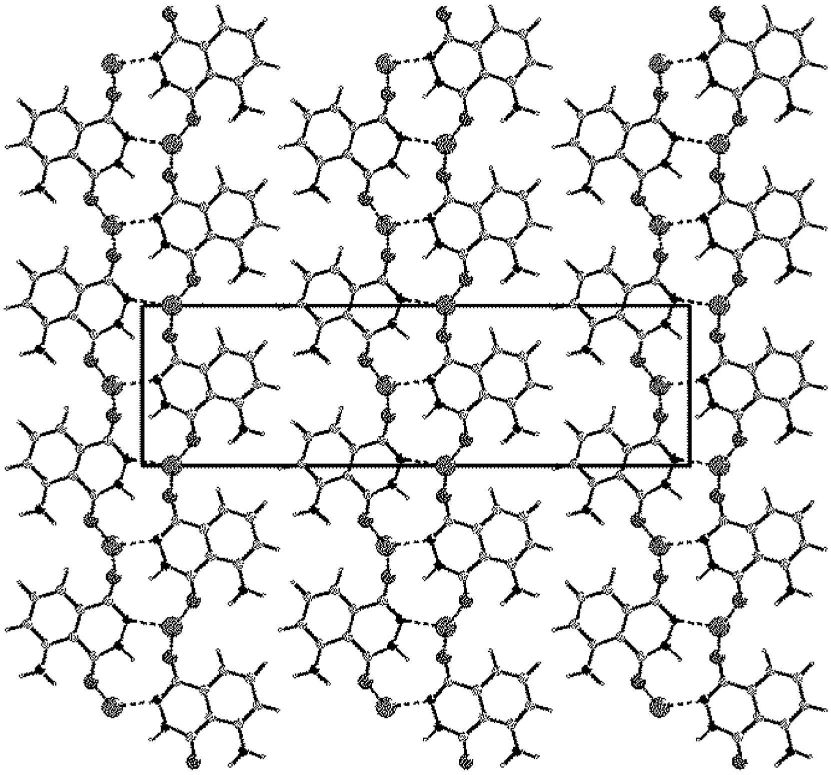

Form II

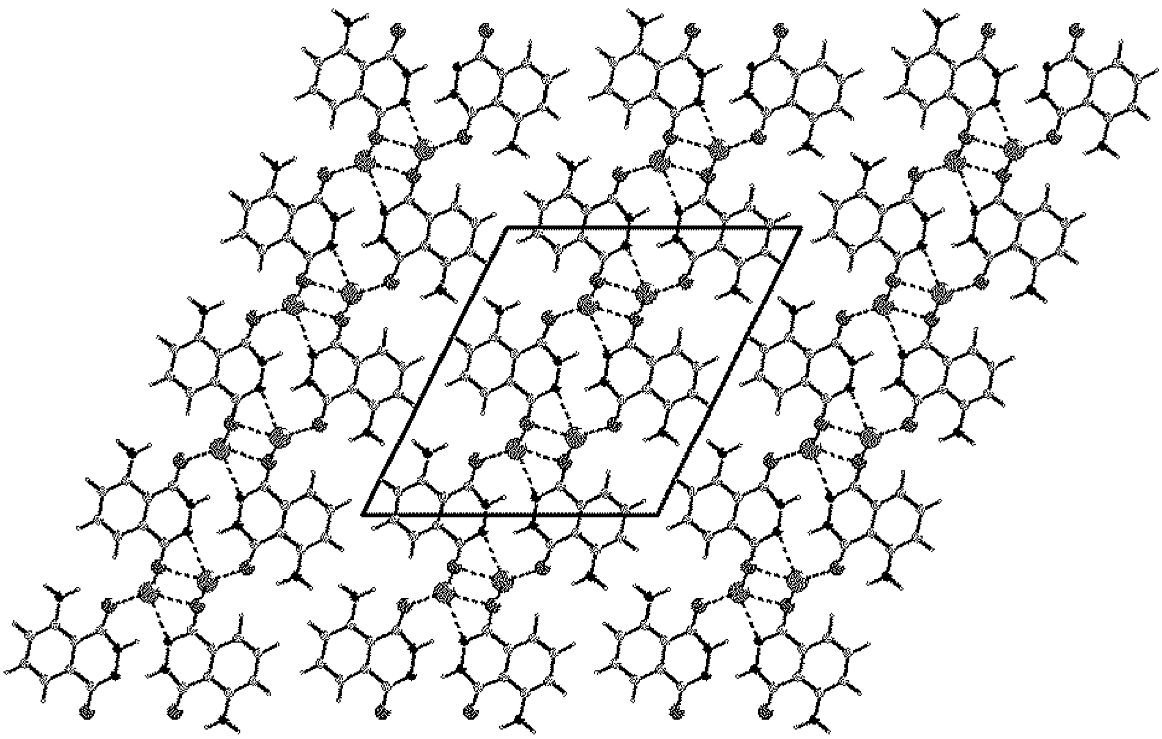

**Abb. 2c (Fortsetzung):**

Form III

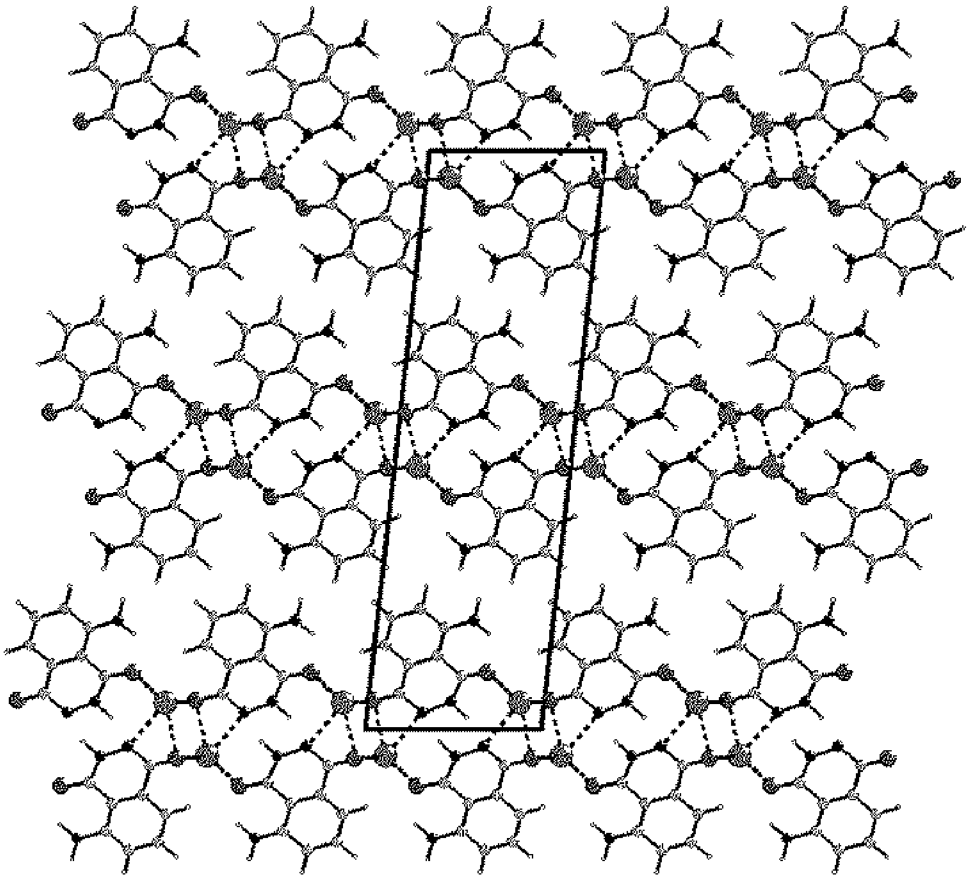

**Abb. 2d:**

Form I

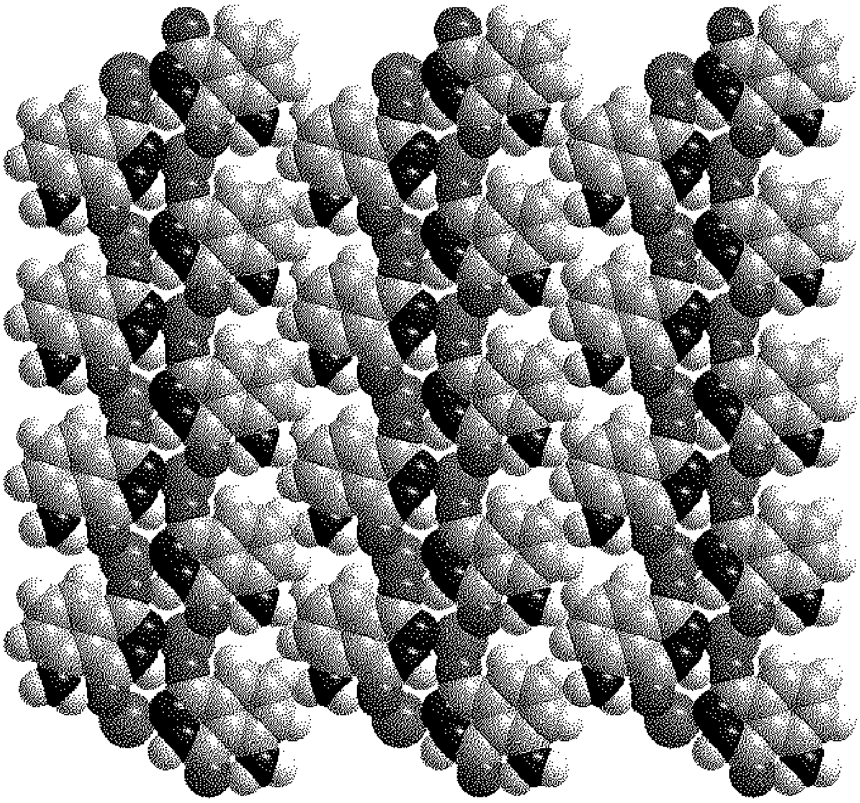

Form II

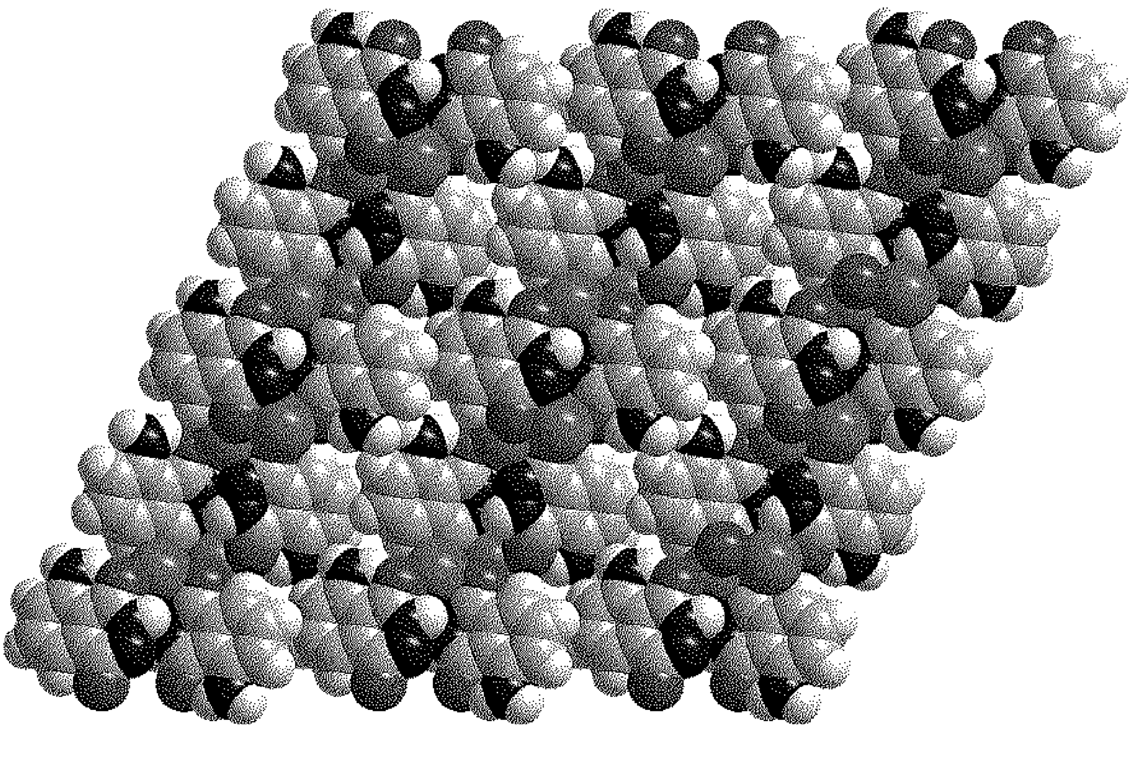

**Abb. 2d (Fortsetzung):**

Form III

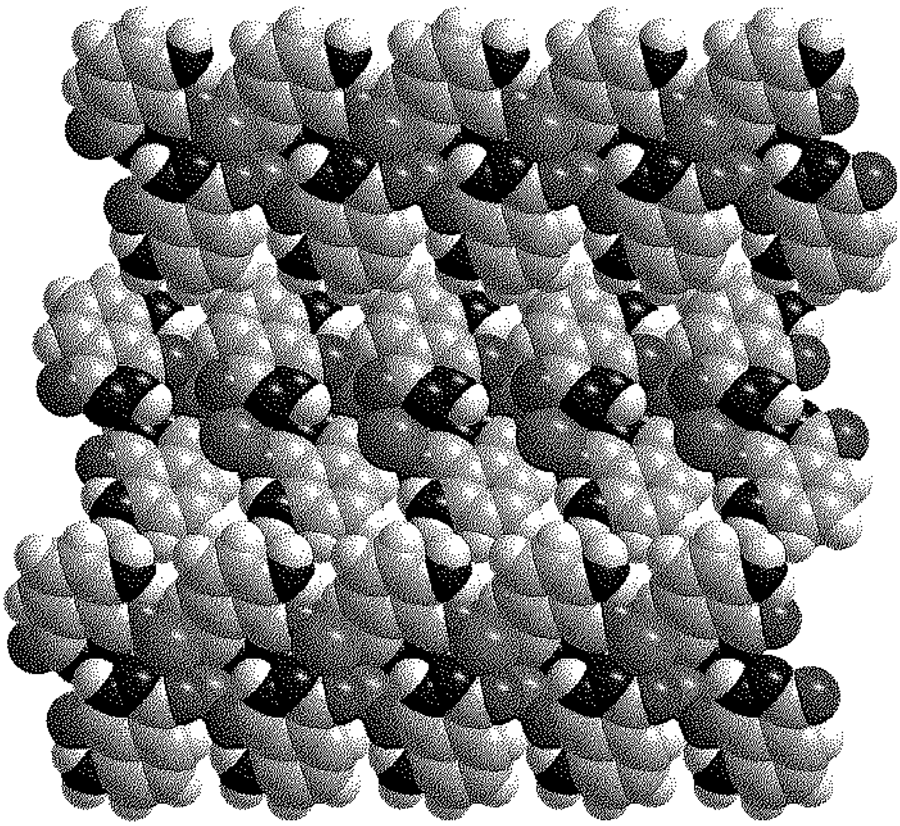

**Abb. 2e:**

Form I

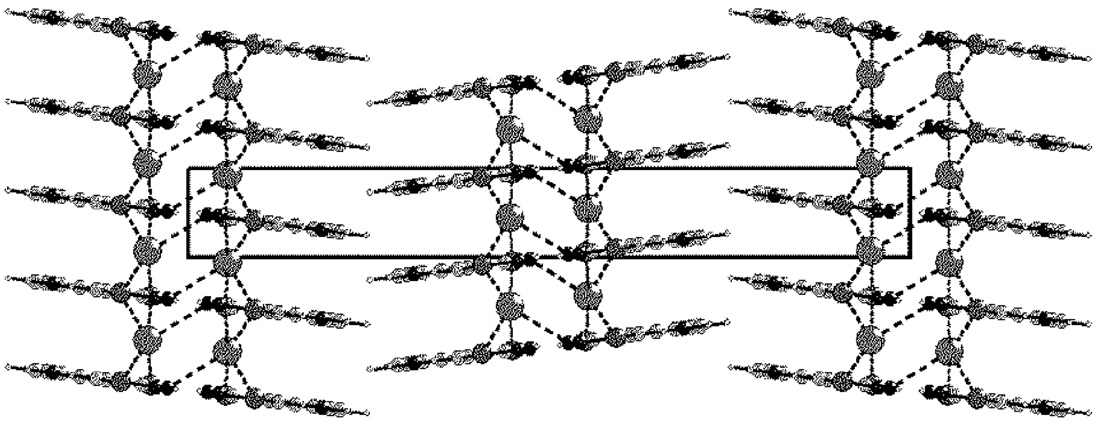

Form II

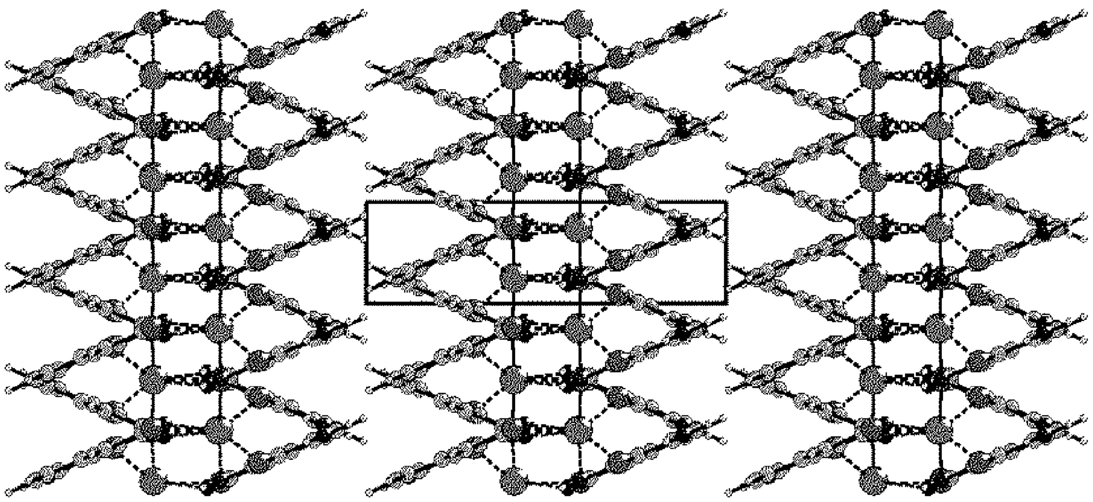

Form III

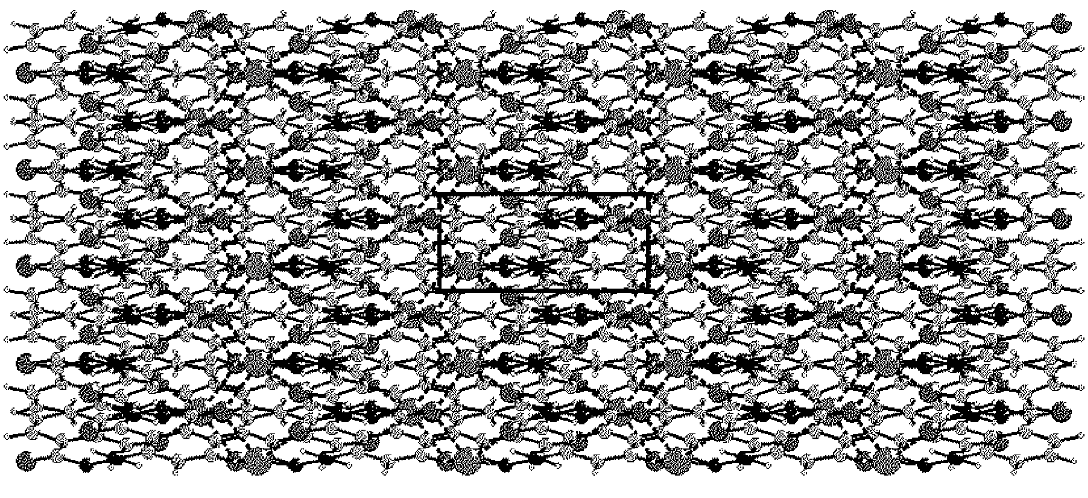

**Abb. 2f:**

Form I

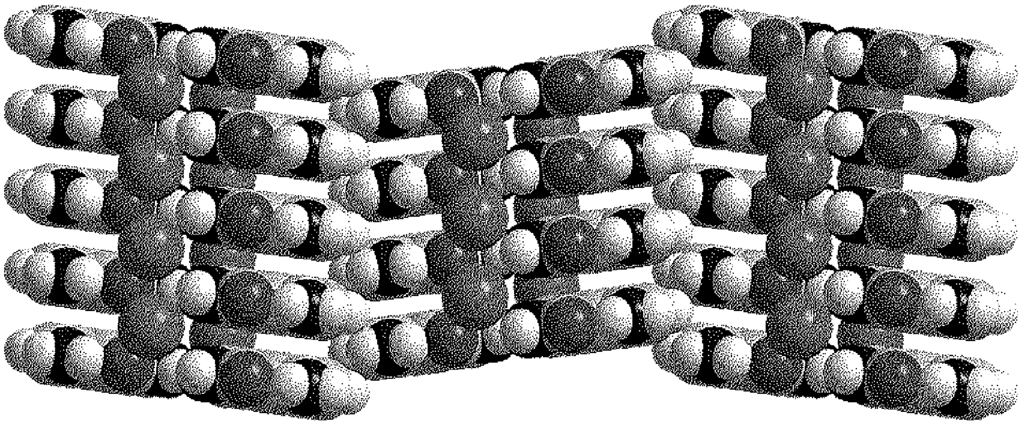

Form II

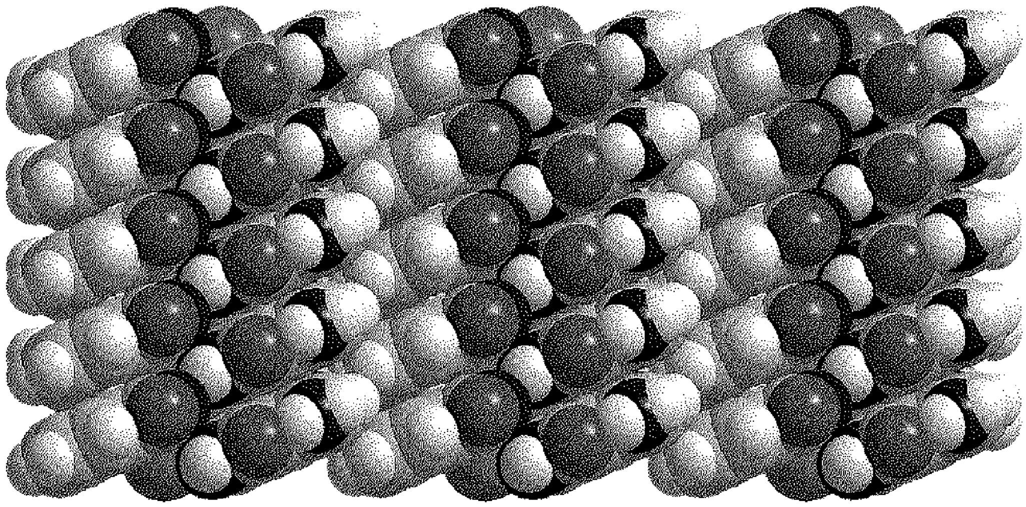

Form III

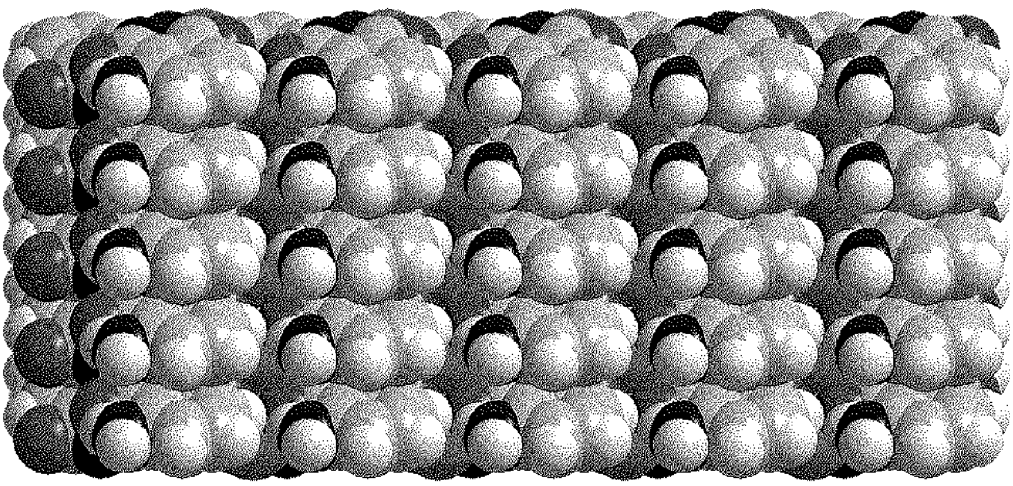

Abb. 3:

**Abb. 3a:**

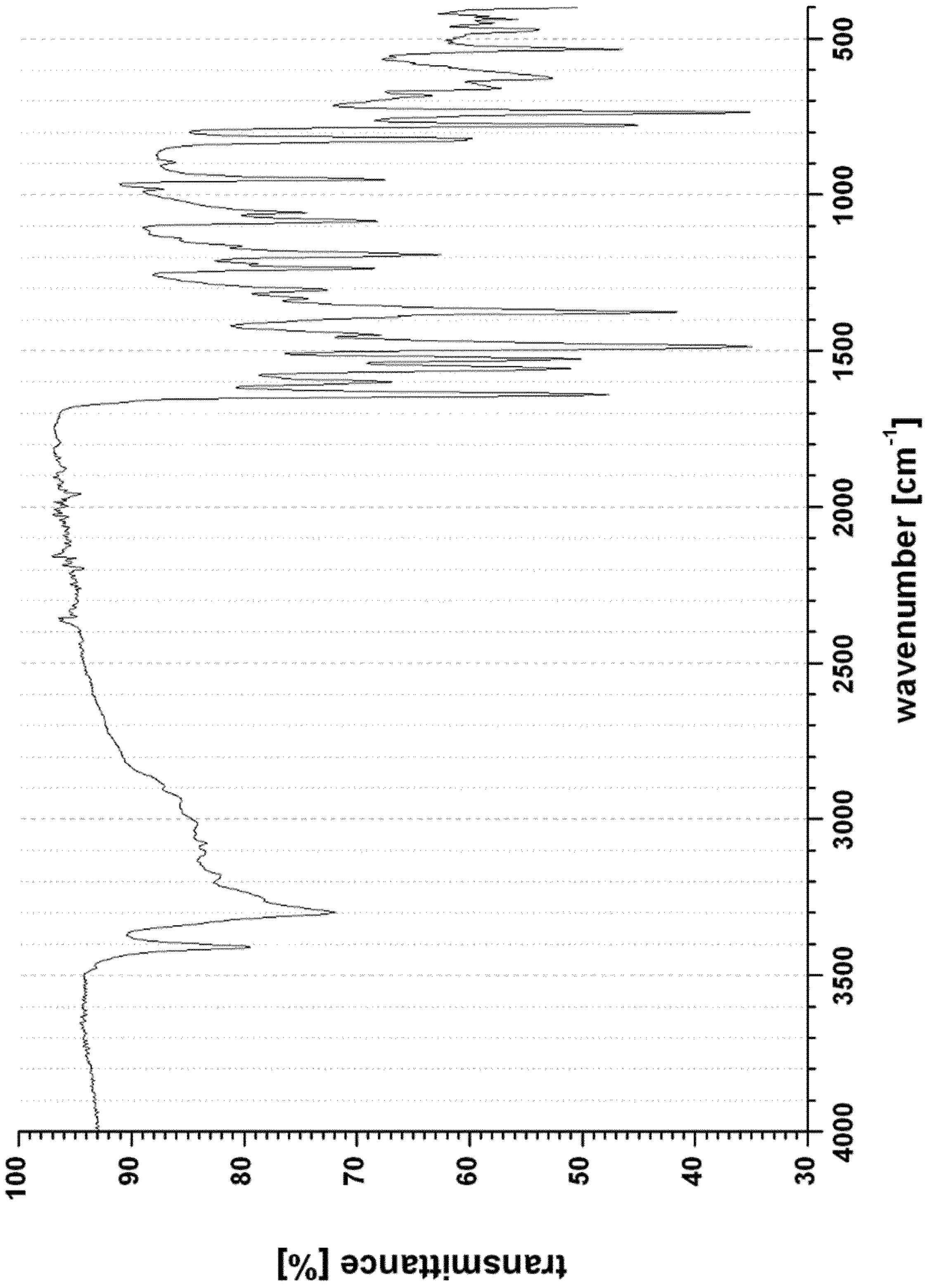
transmittance [%]
wavenumber [cm$^{-1}$]
100
90
80
70
60
50
40
30
4000
3500
3000
2500
2000
1500
1000
500

**Abb. 3b:**

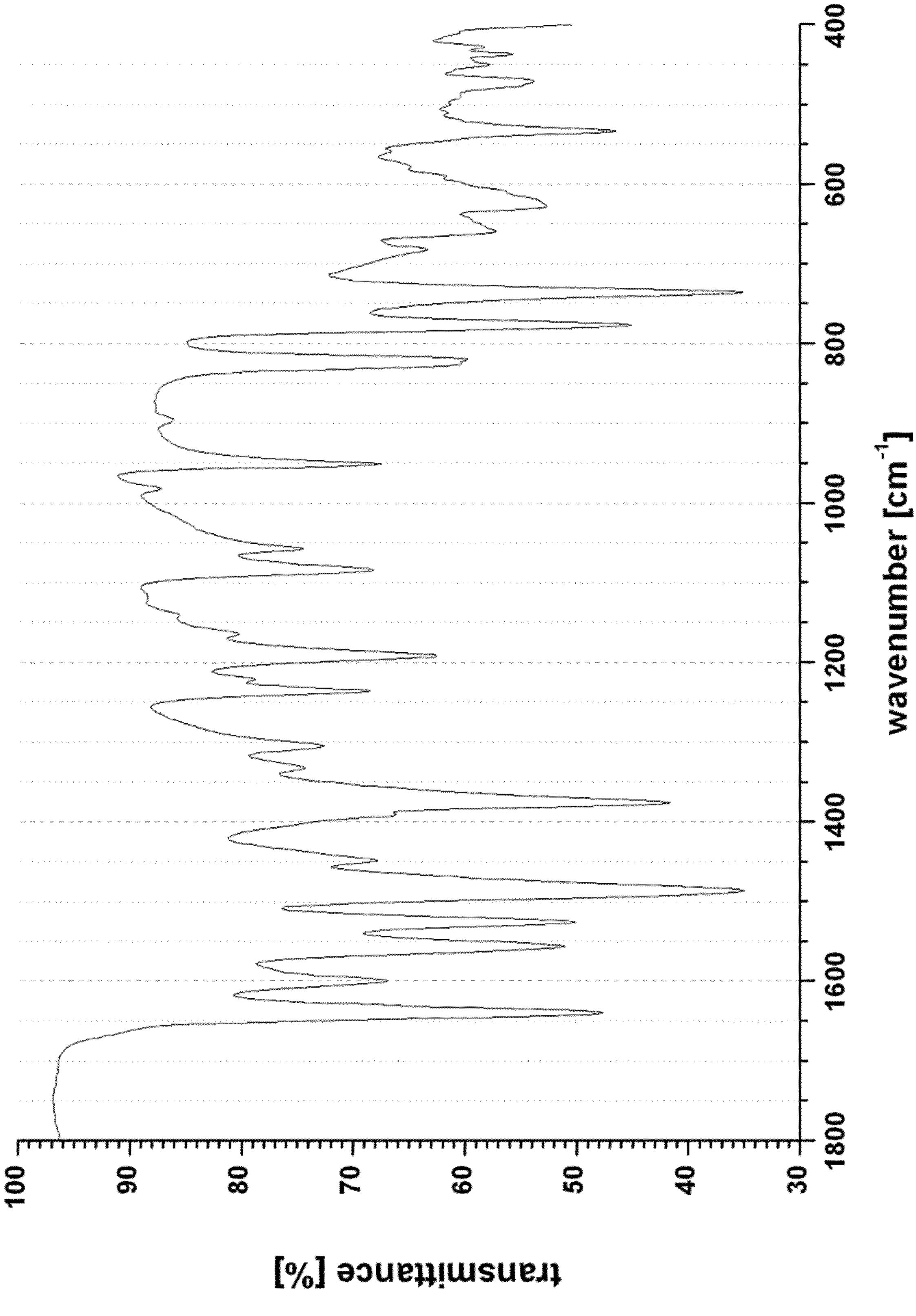
transmittance [%]
100
90
80
70
60
50
40
30
wavenumber [$cm^{-1}$]
1800
1600
1400
1200
1000
800
600
400

Abb. 4:

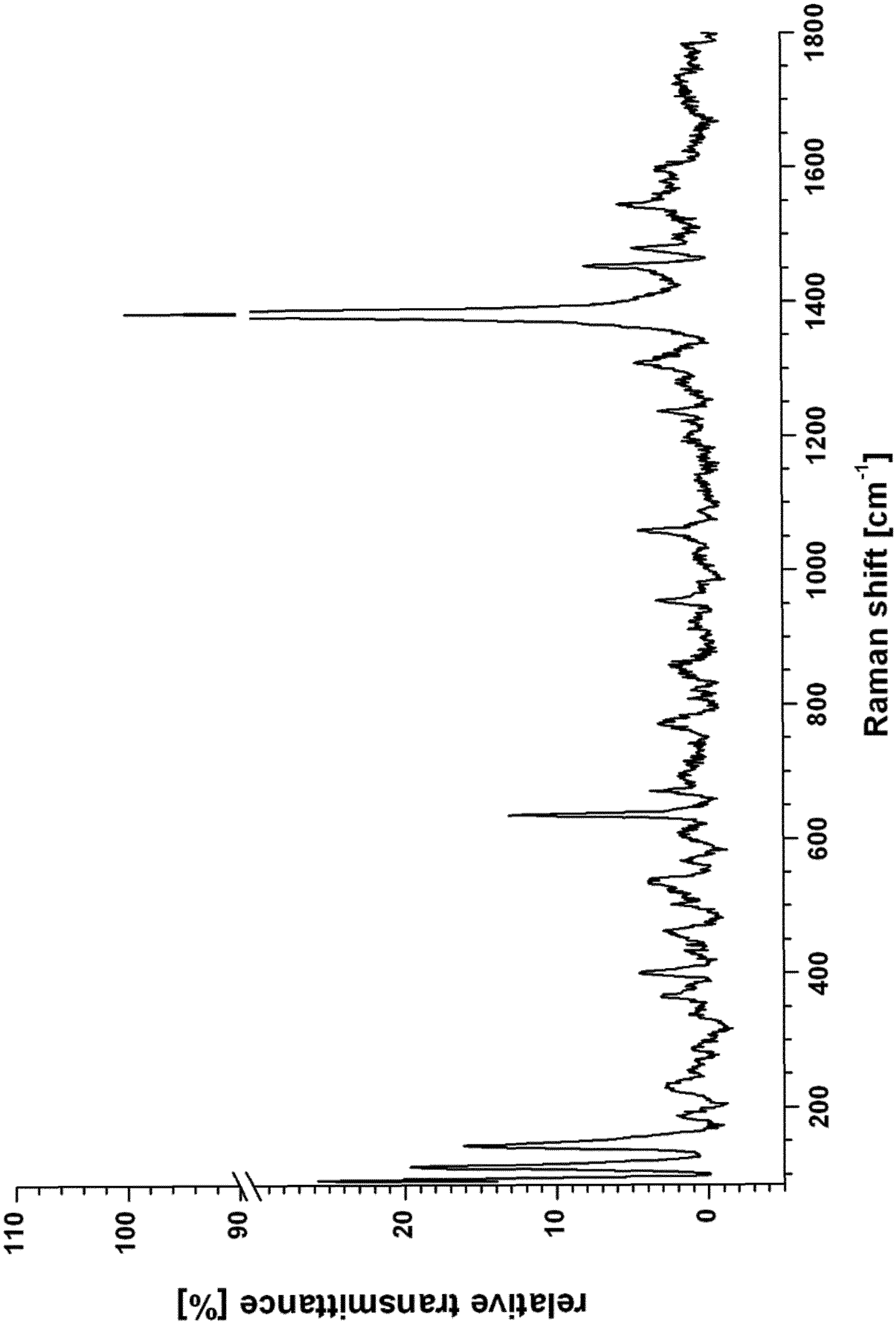
relative transmittance [%]
110
100
90
20
10
0
Raman shift [cm$^{-1}$]
200
400
600
800
1000
1200
1400
1600
1800

Abb. 5:

**Abb. 5a:**

Form I

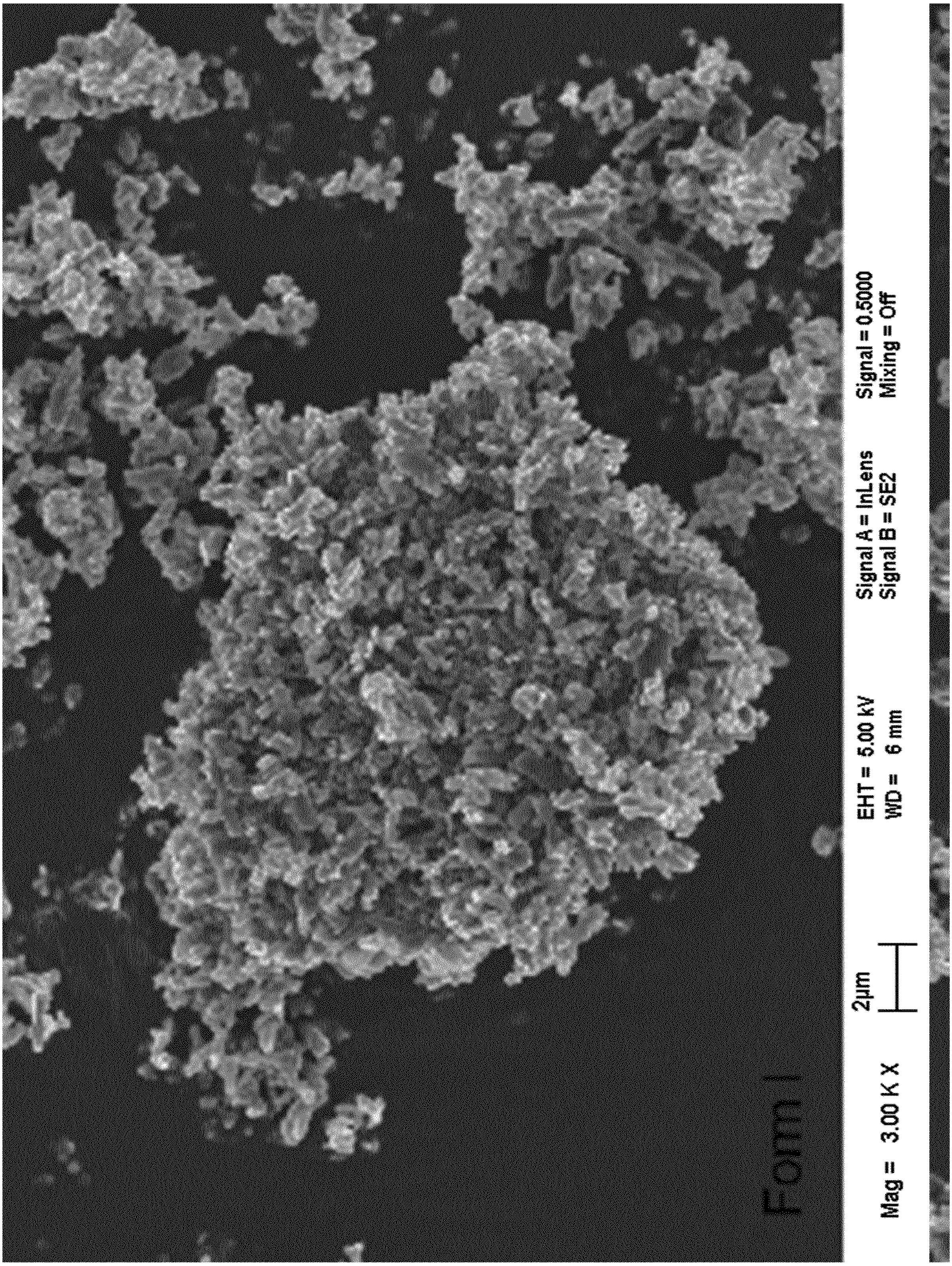

**Abb. 5a (Fortsetzung):**

Form II

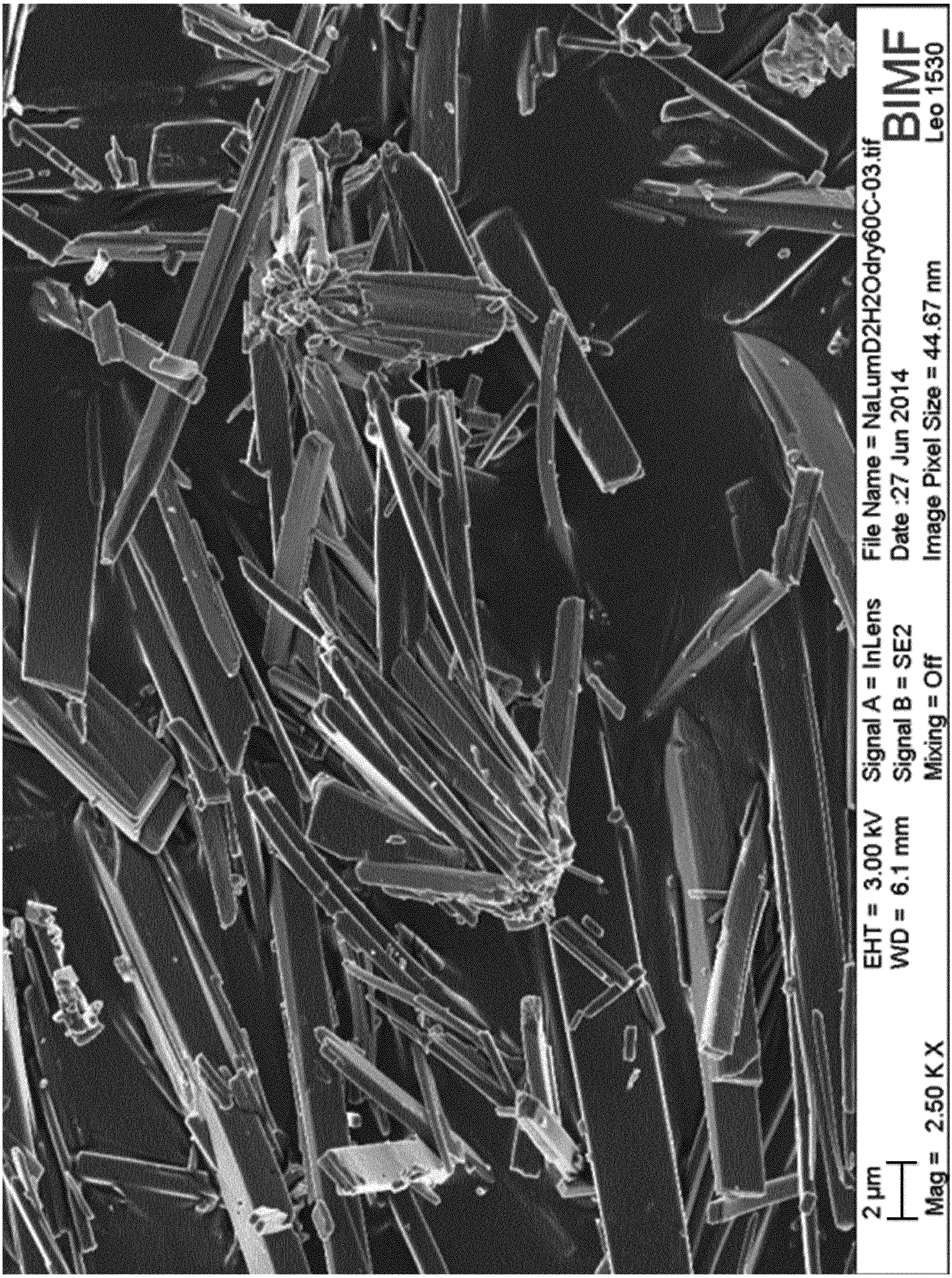

**Abb. 5a (Fortsetzung):**

Form III

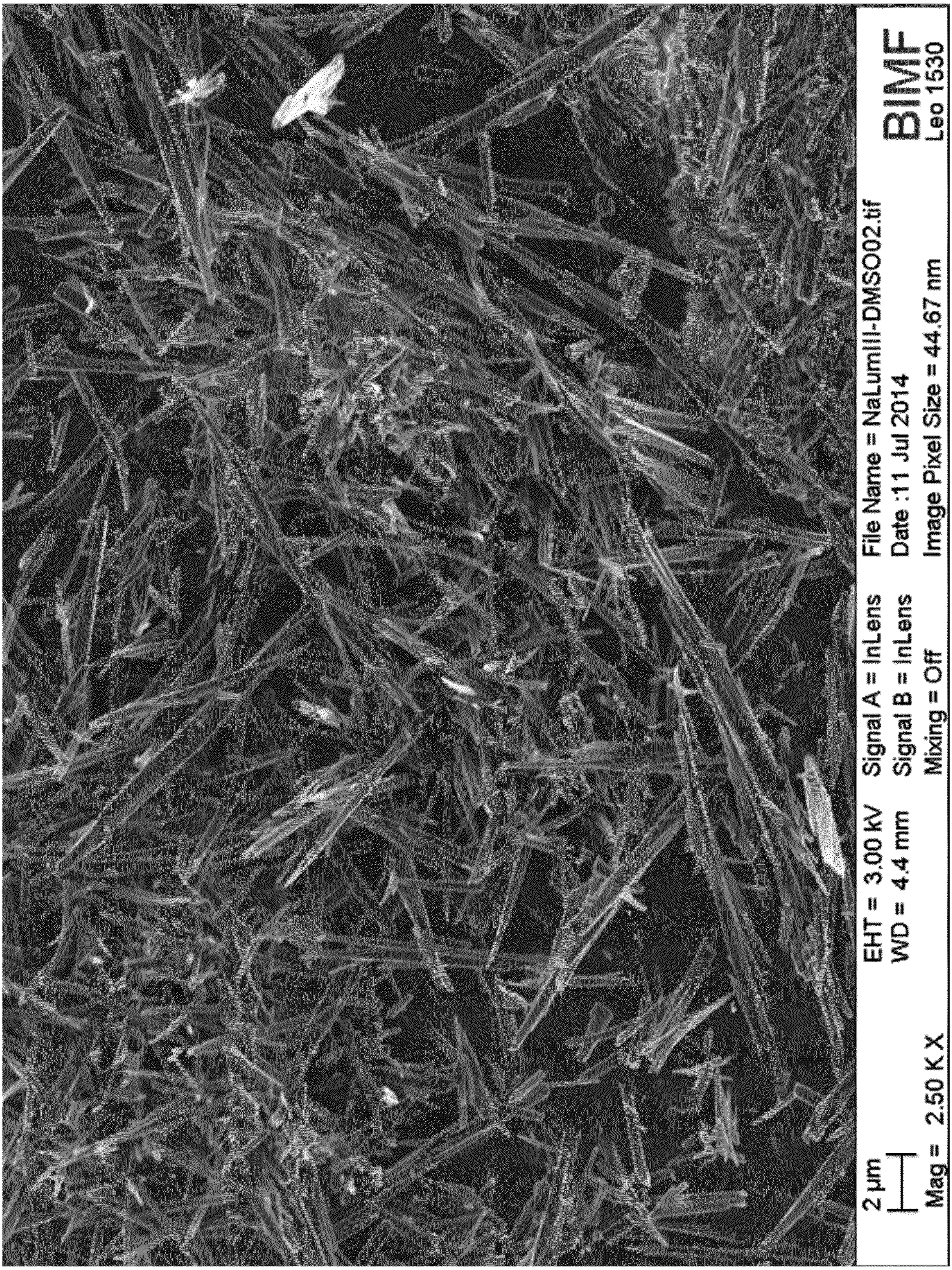

**Abb. 5b:**

Form II

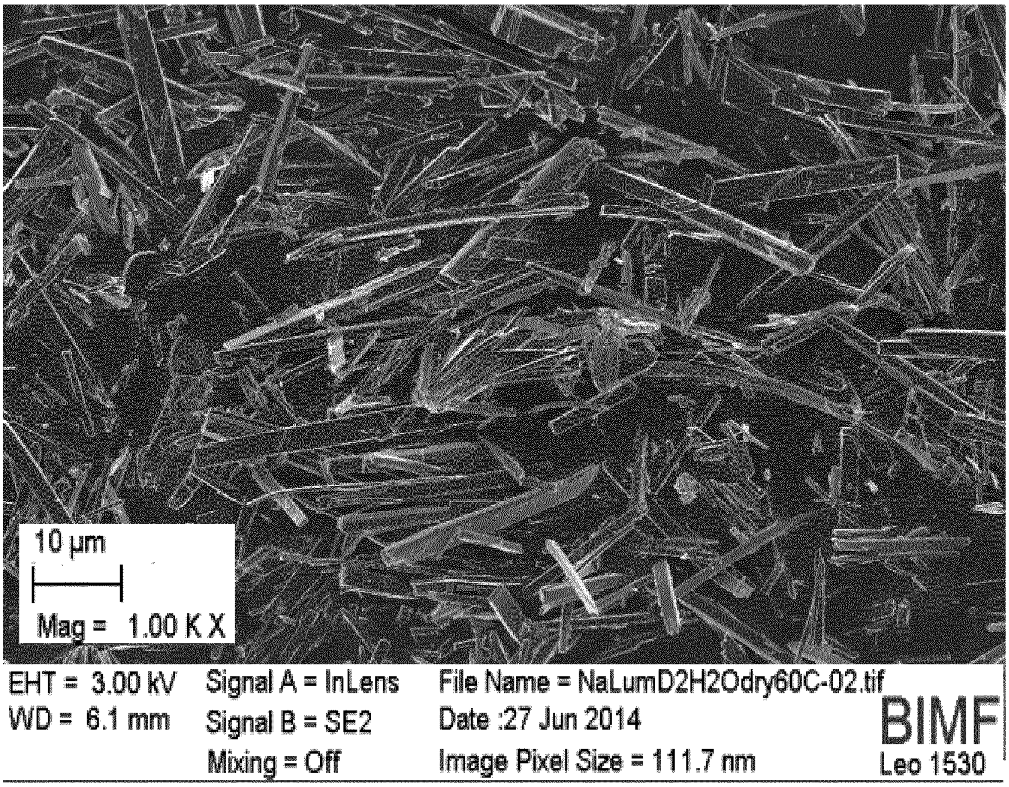


Form III

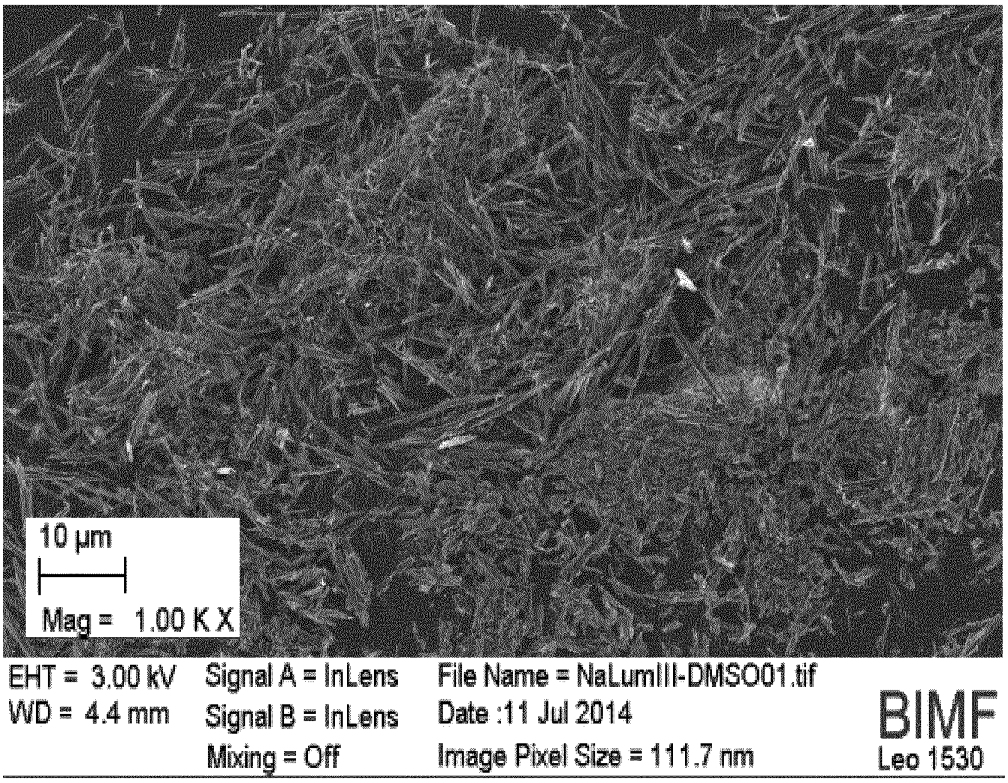

**Abb. 5b (Fortsetzung):**

Form II

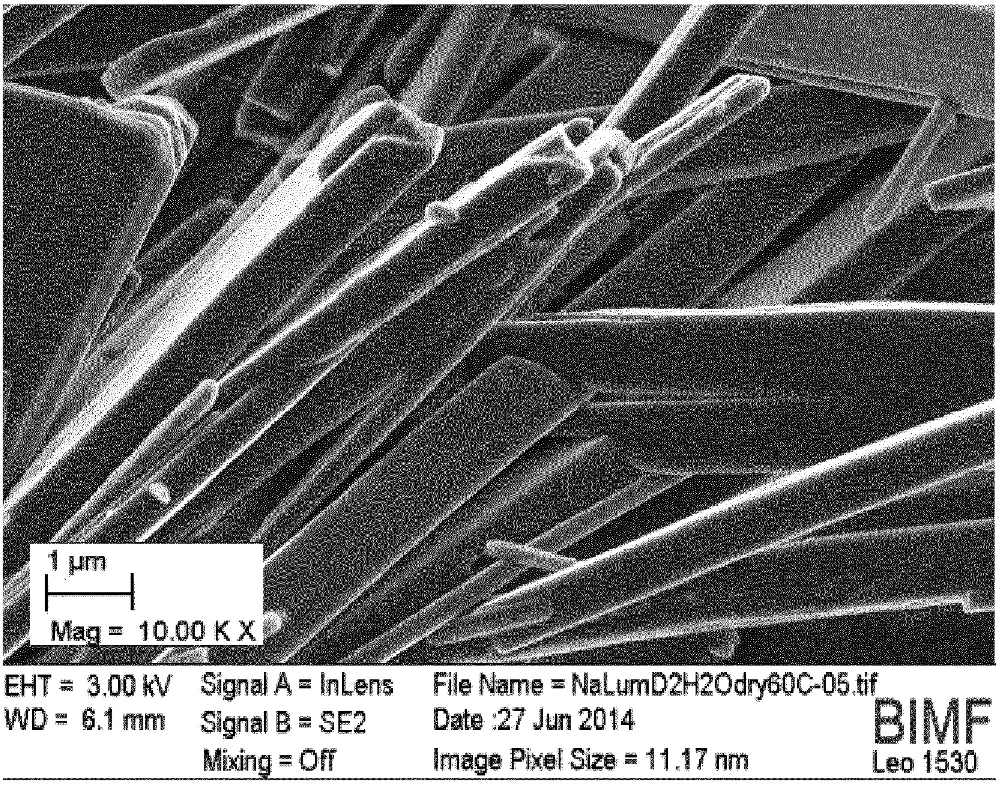


Form III

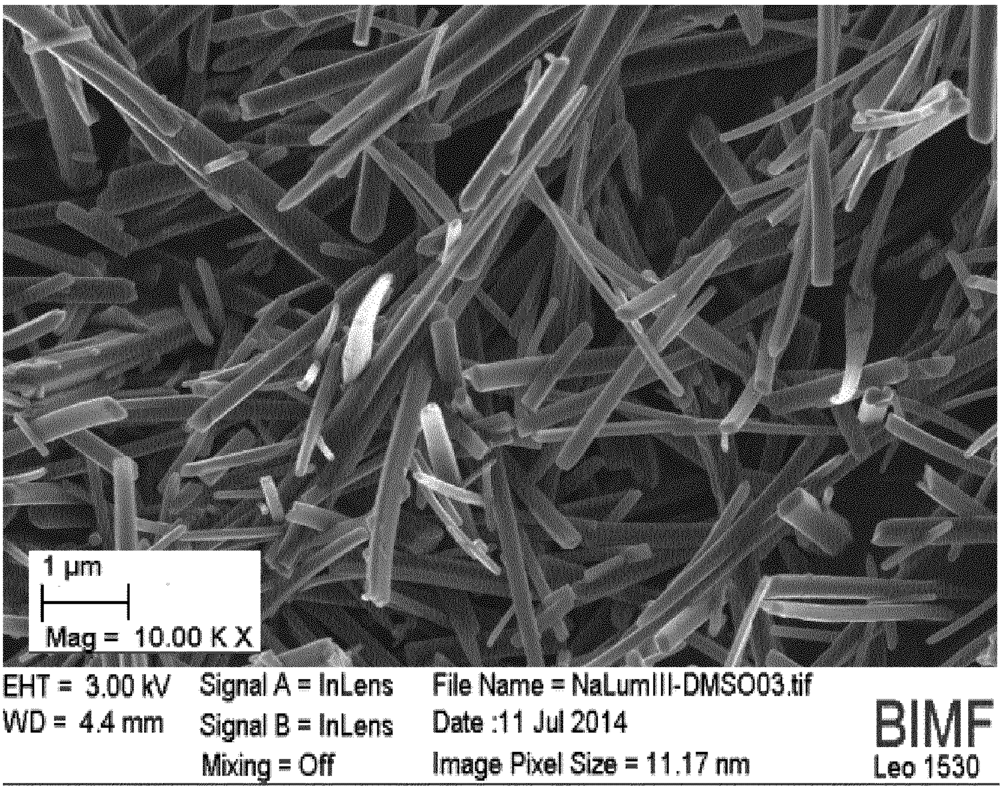

**Abb. 5b (Fortsetzung):**

Form II

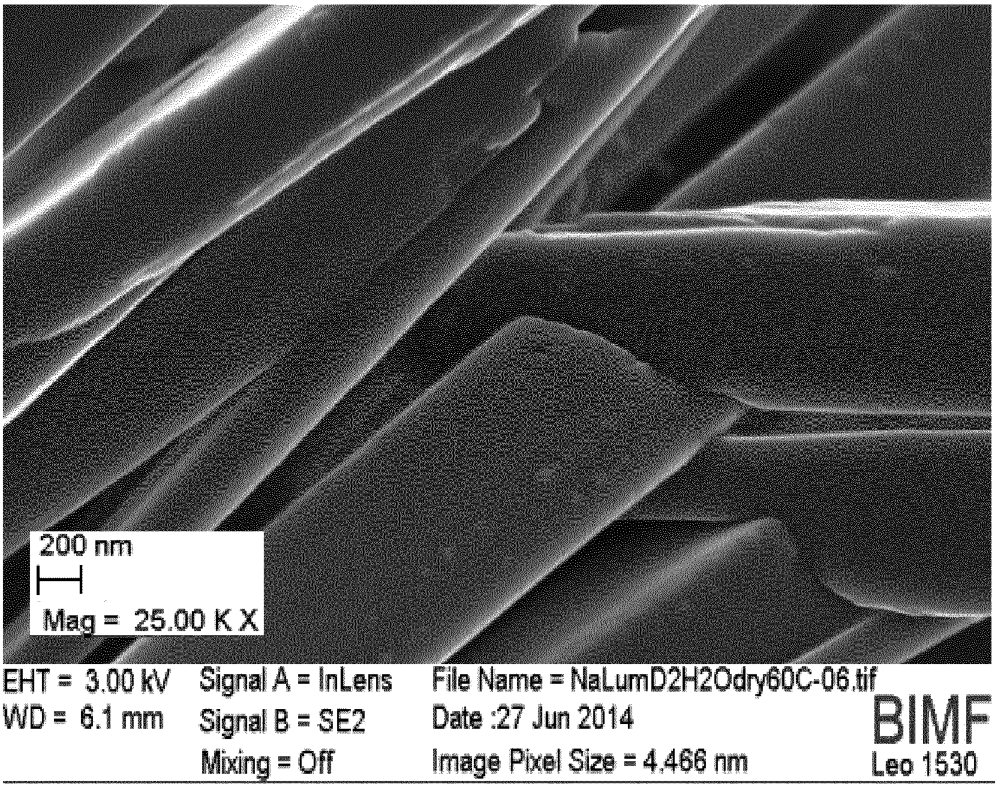


Form III

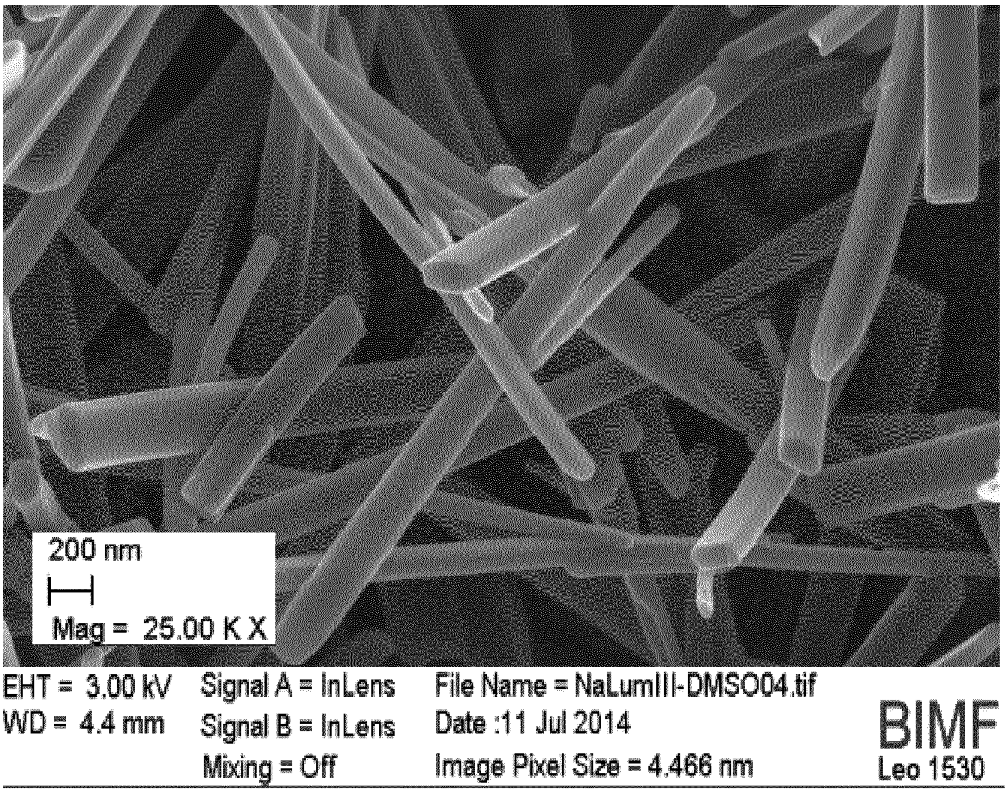

**Abb. 5b (Fortsetzung):**

Form II

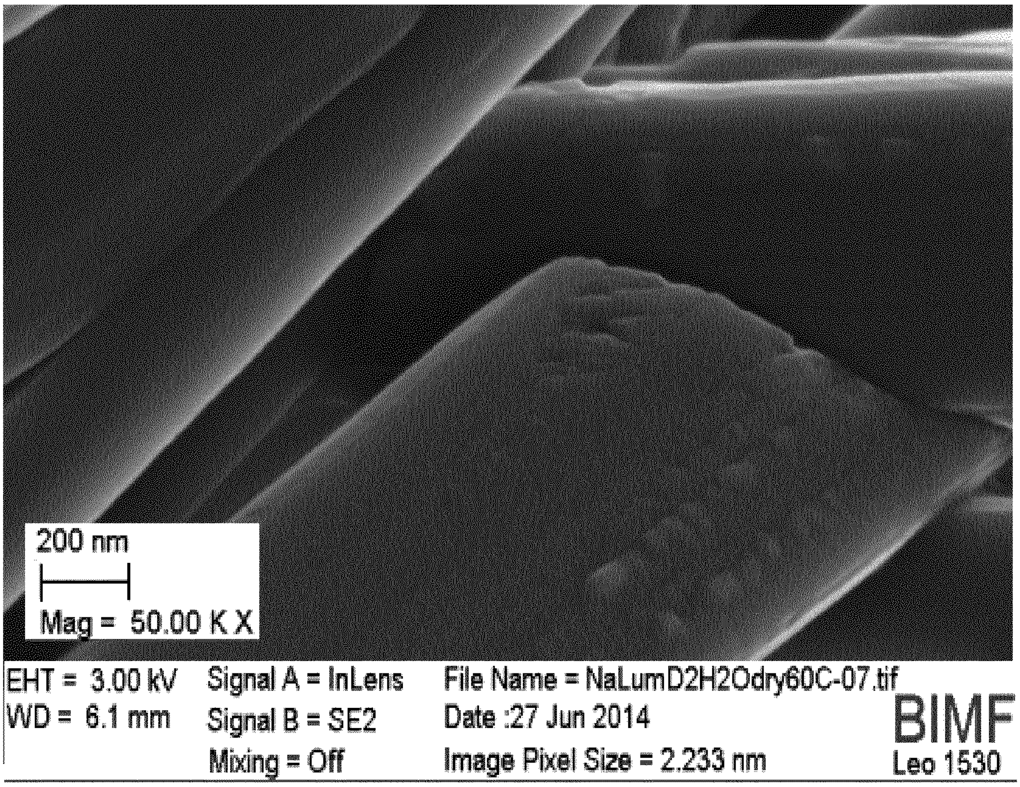


Form III

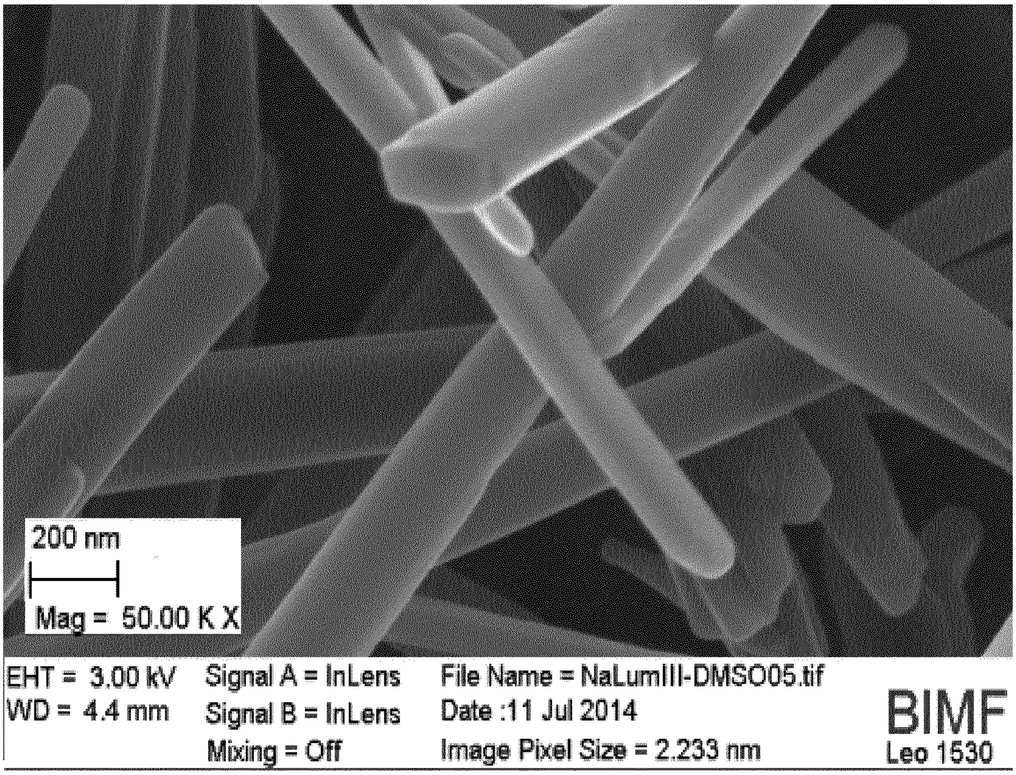

Abb. 6:

**Abb. 6a:**

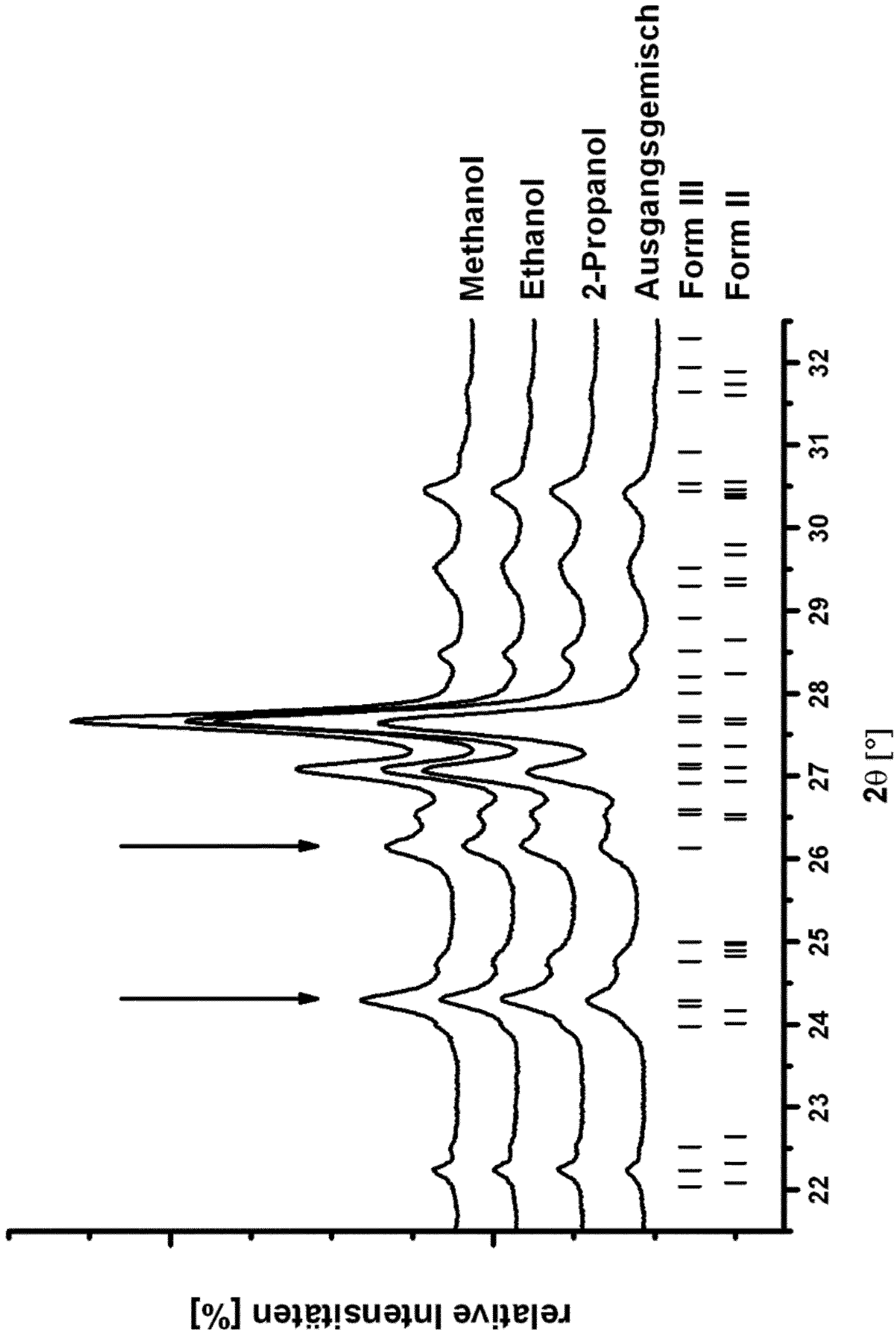
Methanol
Ethanol
2-Propanol
Ausgangsgemisch
Form III
Form II
22
23
24
25
26
27
28
29
30
31
32
2θ [°]
relative Intensitäten [%]

**Abb. 6b:**

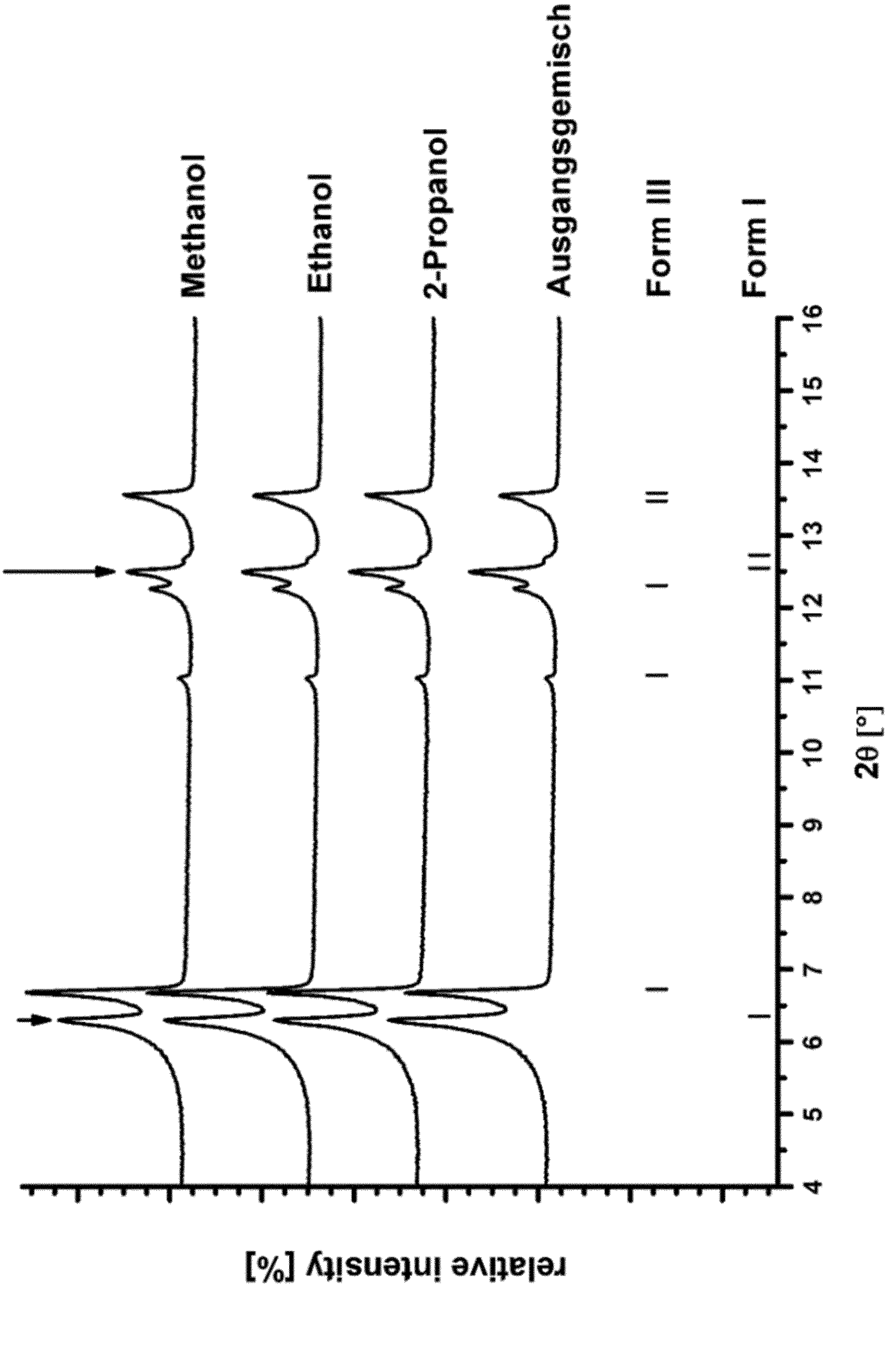
Methanol
Ethanol
2-Propanol
Ausgangsgemisch
Form III
Form I
4
5
6
7
8
9
10
11
12
13
14
15
16
2θ [°]
relative intensity [%]

Abb. 7:

**Abb. 7a:**

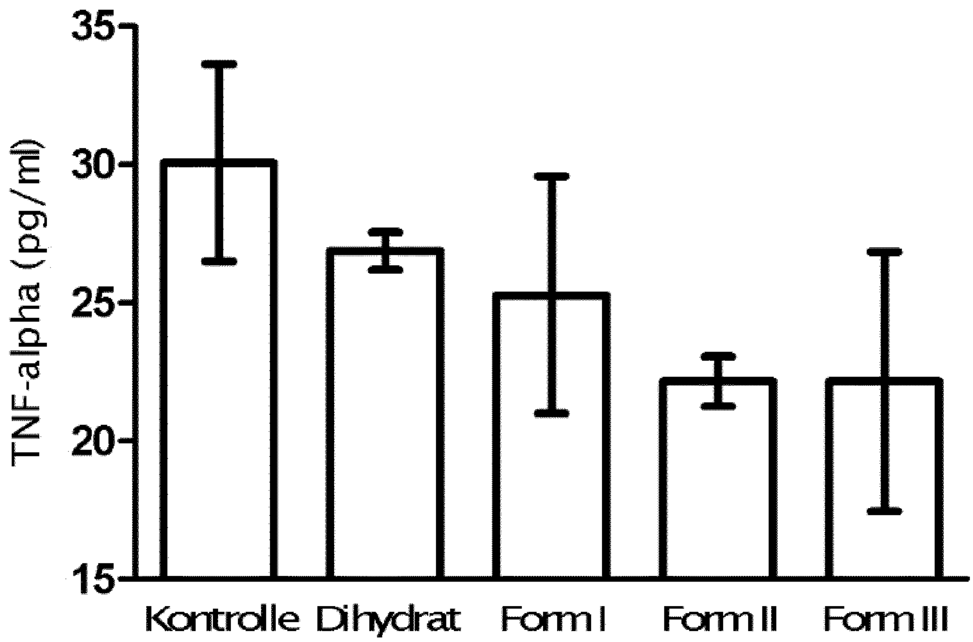


**Abb. 7b:**

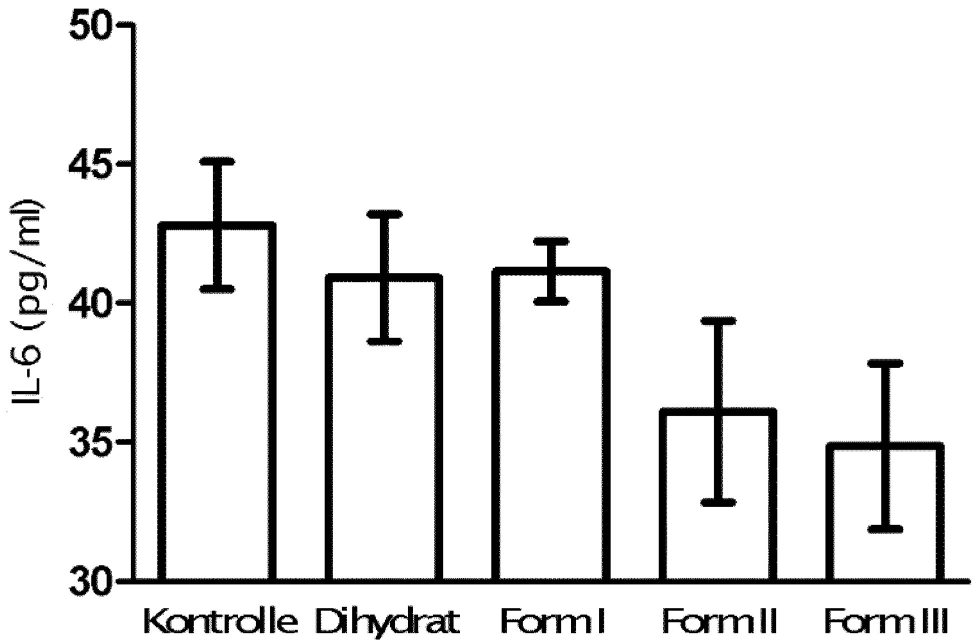

Abb. 8:

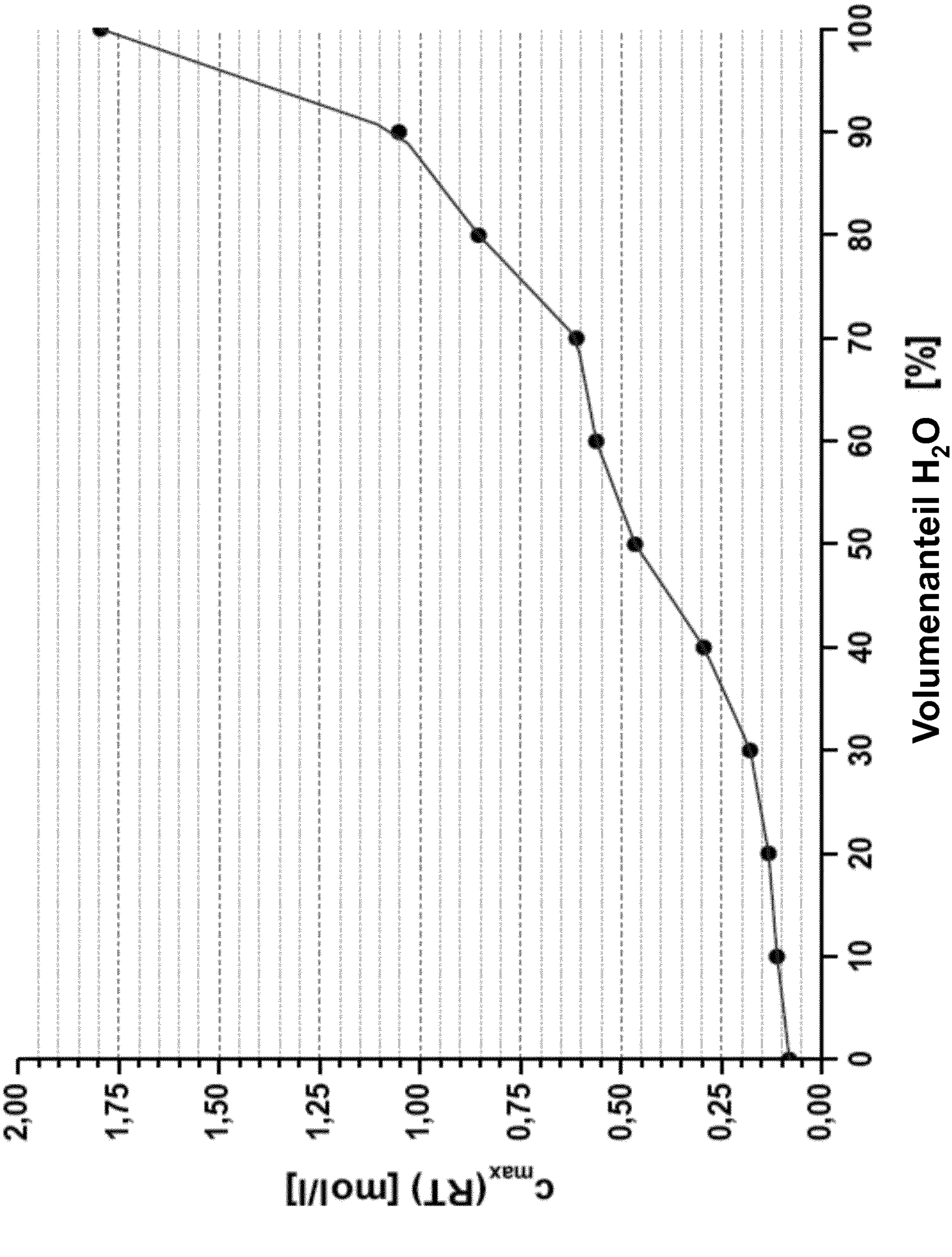
c$_{max}$(RT) [mol/l]
2,00
1,75
1,50
1,25
1,00
0,75
0,50
0,25
0,00
0
10
20
30
40
50
60
70
80
90
100
Volumenanteil H$_2$O [%]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1203587 A1
- RU 2113222 C1
- RU 2211036 C2
- WO 2011107295 A1
- US 6489326 B1
- EP 0617024 B1
- US 5512573 A
- US 5543410 A
- US 7326690 B2


### In der Beschreibung aufgeführte Nicht-Patentliteratur


- **GUZEI et al.** *Journal of Coordination Chemistry,* 2013, vol. 66 (21), 3722-3739
- **RYBAKOV et al.** *Crystallography Reports,* 2014, vol. 59, 383-393
- **HALEBLIAN ; MCCRONE.** *Journal of Pharmaceutical Sciences,* 1969, vol. 58, 911-929
- **GRIESSER.** Polymorphisms in the Pharmaceutical Industry. 2006, 211-234
- **MILLER et al.** Polymorphisms in the Pharmaceutical Industry. 2006, 385-403
- **ULRICH ; JONES.** *Nachrichten aus der Chemie,* 2005, vol. 53, 19-23
- **DESCOTES.** *Expert Opin. Drug Metab. Toxicol.,* 2008, vol. 4 (12), 1537-1549
- **HOFFMANN.** *Intensivmed,* 2005, vol. 42, 371-377
- **LINUS PAULING.** *Journal of the American Chemical Society,* 1929, vol. 51, 1010-1026
- **WILLIAMSON, K. L.** Macroscale and Microscale Organic Experiments. Heath, 1994